# EUROPEAN PATENT APPLICATION

(11) **EP 4 653 451 A1**
(43) Date of publication of application: **26.11.2025**
(21) Application number: 24752926.6
(22) Date of filing: 08.02.2024
(51) Int. Cl.: C07K 7/06, C07K 7/56, A61K 38/08, A61K 38/12, A61P 35/00

(54) **POLYPEPTIDE COMPOUND AND USE THEREOF**

(30) Priority: 10.02.2023 CN 202310098607; 27.04.2023 CN 202310477987
(71) Applicant: Chengdu New Radiomedicine Technology Co., Ltd., Chengdu, Sichuan 610200 (CN)
(72) Inventor: LI, Xianghui, Chengdu, Sichuan 610200 (CN); LIU, Qiangqiang, Chengdu, Sichuan 610200 (CN); SUN, Haifeng, Chengdu, Sichuan 610200 (CN); ZHU, Zhiyuan, Chengdu, Sichuan 610200 (CN); CHEN, Huaxi, Chengdu, Sichuan 610200 (CN); RAN, Chonglin, Chengdu, Sichuan 610200 (CN)
(74) Representative: Isarpatent
(86) International application number: PCT/CN2024/077016
(87) International publication number: WO 2024/165072

(57) **Abstract**

Disclosed are a polypeptide compound and the use thereof. Provided are a polypeptide compound, a pharmaceutically acceptable salt thereof, a solvate thereof, or a solvate of the pharmaceutically acceptable salt thereof, wherein the polypeptide compound is a compound as represented by formula (I); wherein A₁, A₂, A₃, A₄, As, A₆ and A₇ are sequentially connected via a peptide bond (-CO-NH-). The polypeptide compound of the present invention has relatively good inhibitory activity against an FAP protein.

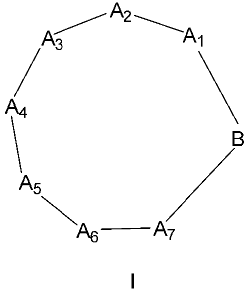

## Description

### TECHNICAL FIELD

The present disclosure relates to a polypeptide compound and the use thereof.

### BACKGROUND ART

Tumor tissue contains a tumor microenvironment formed by tumor cells, stromal cells, immune cells and other types of cells. Cancer associated fibroblasts (CAFs) are one of the main cell types in the tumor microenvironment in solid tumors, usually showing the properties of interstitial cells. CAFs can be derived from various types of cells, such as fibroblasts, mesenchymal stem cells, smooth muscle cells, etc. *(*Kalluri, Nat Rev Cancer, 2016, 16: 582*;* Gascard, et al., Genes Dev, 2016, 30: 1002*).* CAFs play an important role in the production, progression, and metastasis of tumors, and thus is receiving increasing attention in anti-tumor therapy.

Fibroblast activation protein α (FAP) is a type II transmembrane serine protease composed of 760 amino acids, which is highly expressed in tumor-associated fibroblasts. In addition, FAP is also highly expressed in a variety of tumor cells, such as oral squamous carcinoma, esophageal carcinoma, stomach cancer, mesothelioma, fibrosarcoma, etc. *(*Busek, et al., Front Biosci (Landmark Ed), 2018, 23: 1933).

FAP, as a marker protein of tumor-associated fibroblasts, exists in stromal cells of tumors and tumor-associated fibroblasts. In recent years, FAP has been considered as a marker protein of tumor radioactive diagnosis and an important target protein of tumor radiotherapy. *(*Siveke, J Nucl Med, 2018, 59: 1412*).* In 2018, Haberkorn's team reported a class of small-molecule quinoline FAP inhibitors and developed them as FAP-targeted radioactive diagnostic drugs. However, such compounds are enriched in tumor tissues for a short time, and have only entered clinical research as diagnostic drugs at present. *(*Thomas Lindner, et al., J Nucl Med. 2018; 59(9): 1415-1422*).* In 2022, Frank Osterkamp's team reported a class of cyclic peptide FAP inhibitors as ligands for the development of FAP-targeted radioactive diagnostic drugs or therapeutic drugs. In animal experiments, such inhibitors demonstrated higher enrichment, longer retention time and better efficacy in tumor tissues. However, the clinical data of phase 1 showed that the radiation dose of such inhibitors to the tumor tissue of patients at a safe dose is still relatively low, showing a poor therapeutic effect. *(*Dirk Zboralski, et al., Eur J Nucl Med Mol Imaging. 2022; 49(11): 3651-3667*;* Richard P Baum, et al., J Nucl Med. 2022 Mar; 63(3): 415-423*).*

In addition, FAP not only plays an important role in the production and development of tumors, but also plays a vital role in rheumatoid arthritis, wound healing *(*Dienus, et al., Arch Dermatol Res, 2010, 302: 725*),* fibrosis diseases (including liver fibrosis, pulmonary fibrosis, etc.) and atherosclerosis. Therefore, the radioactive drugs of FAP can also be developed for the treatment or diagnosis of rheumatoid arthritis, scar repair drugs *(*Dienus, et al., Arch Dermatol Res, 2010, 302: 725*),* the treatment or diagnosis of various fibrotic diseases, and for imaging diagnostic drugs for atherosclerosis as a radioactive tracer (Meletta, et al., Molecules, 2015, 20: 2081*).*

Although FAP-targeted radioactive drugs for the diagnosis and treatment of tumors have entered clinical research, it is still of significant clinical value to develop radioactive therapeutic drugs with better target selectivity, higher tissue specificity, and higher enrichment and longer retention time in tumor tissues.

### SUMMARY OF THE INVENTION

The technical problem to be solved by the present disclosure is that the existing compound targeting FAP protein has a single structure. To this end, the present disclosure provides a polypeptide compound and the use thereof. The compound of the present disclosure has a novel structure and relatively good inhibitory activity against an FAP protein.

In a first aspect, the present disclosure provides a polypeptide compound, a pharmaceutically acceptable salt thereof, a solvate thereof or a solvate of a pharmaceutically acceptable salt thereof, wherein the polypeptide compound is a compound represented by formula I; wherein A₁, A₂, A₃, A₄, A₅, A₆ and A₇ are sequentially connected via a peptide bond (-CO-NH-);

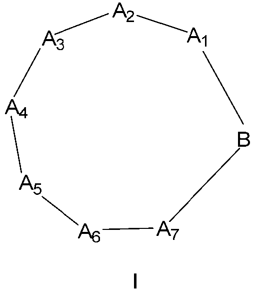

A₇ has a structure represented by formula a-7, 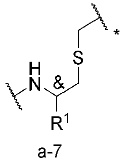
"&" denotes that the carbon atom herein is a chiral carbon, that is, 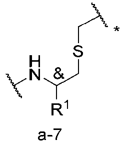 is 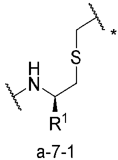 and/or 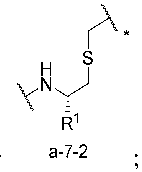 "*" end denotes attachment to B;
R¹ is -(CH₂)ₘM;
m is 1, 2, 3, 4, 5 or 6;
M is -COR¹⁻¹ or -NR¹⁻²R¹⁻³;
R¹⁻¹ is -OH, -NR^{a}R^{b}, -OC₁₋₁₂alkyl, -OC₃₋₁₂cycloalkyl, -OC₆₋₁₀aryl, -O-5-10-membered heteroaryl, a group formed by the loss of a H atom by an amino group on an amino acid, a group formed by the loss of a H atom by an amino group on a peptide formed by 2 or more amino acids, -OC₁₋₁₂alkyl substituted with 1, 2 or 3 R^{e}, -OC₃₋₁₂cycloalkyl substituted with 1, 2 or 3 R^{f}, -OC₆₋₁₀aryl substituted with 1, 2 or 3 R^{e-1}, or -O-5-10-membered heteroaryl substituted with 1, 2 or 3 R^{f-1};
R^{a} and R^{b} are independently H, C₁₋₁₂alkyl, C₃₋₁₂cycloalkyl, C₆₋₁₀aryl, 5-10-membered heteroaryl, C₁₋₁₂alkyl substituted with 1, 2 or 3 R^{b-1}, C₃₋₁₂cycloalkyl substituted with 1, 2 or 3 R^{b-2}, C₆₋₁₀aryl substituted with 1, 2 or 3 R^{b-3}, or 5-10-membered heteroaryl substituted with 1, 2 or 3 R^{b-4};
R^{b-1}, R^{b-2}, R^{b-3} and R^{b-4} are independently deuterium, -OH, -NH₂, -COOH, -CONH₂, -CN, halogen, C₁₋₁₂alkyl, C₃₋₁₂cycloalkyl, C₆₋₁₀aryl, 5-10-membered heteroaryl, C₆₋₁₀aryl substituted with 1, 2 or 3 R^{b-1-1}, 5-10-membered heteroaryl substituted with 1, 2 or 3 R^{b-1-2}, C₃₋₁₂cycloalkyl substituted with 1, 2 or 3 R^{b-1-3} , or C₁₋₁₂alkyl substituted with 1, 2 or 3 R^{b-1-4};
R^{b-1-1}, R^{b-1-2}, R^{b-1-3} and R^{b-1-4} are independently C₁₋₁₂alkyl, halogen, or C₁₋₁₂alkyl substituted with 1, 2 or 3 halogen;
R^{e}, R^{f}, R^{e-1} and R^{f-1} are independently halogen, -OH or -NH₂;
R¹⁻² and R¹⁻³ are independently H, C₁₋₁₂alkyl, C₃₋₁₂cycloalkyl, C₆₋₁₀aryl, 5-10-membered heteroaryl, -COR^{g}, a group formed by the loss of a -OH group by a carboxyl group on an amino acid, a group formed by the loss of a -OH group by a carboxyl group on a peptide formed by 2 or more amino acids, C₁₋₁₂alkyl substituted with 1, 2 or 3 R^{h}, C₃₋₁₂cycloalkyl substituted with 1, 2 or 3 R^{h-1}, C₆₋₁₀aryl substituted with 1, 2 or 3 R^{h-2}, or 5-10-membered heteroaryl substituted with 1, 2 or 3 R^{h-3};
R^{g} is independently C₁₋₁₂alkyl, C₃₋₁₂cycloalkyl, C₆₋₁₀aryl, 5-10-membered heteroaryl, C₁₋₁₂alkyl substituted with 1, 2 or 3 R^{g-1}, C₃₋₁₂cycloalkyl substituted with 1, 2 or 3 R^{g-2}, C₆₋₁₀aryl substituted with 1, 2 or 3 R^{g-3}, or 5-10-membered heteroaryl substituted with 1, 2 or 3 R^{g-4};
R^{g-1}, R^{g-2}, R^{g-3} and R^{g-4} are independently deuterium, -OH, -NH₂, -COOH, -CN, halogen, C₁₋₁₂alkyl, C₃₋₁₂cycloalkyl, C₆₋₁₀aryl, or C₆₋₁₀aryl substituted with 1, 2 or 3 R^{g-1-1};
R^{g-1-1} is independently -OH, -NH₂, -COOH, -CN, halogen, C₁₋₁₂alkyl, or C₁₋₁₂alkyl substituted with 1, 2 or 3 halogen;
R^{h}, R^{h-1}, R^{h-2} and R^{h-3} are independently deuterium, -COOH, -CN, halogen, -OH, or -NH₂;
B is 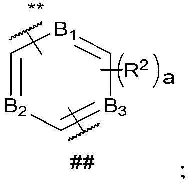
B₁, B₂ and B₃ are independently N or C;
a is 1, 2 or 3;
R² is independently H, C₁₋₁₂alkyl or halogen;
"**" end denotes attachment to A₇; "##" end denotes attachment to A₁;
A₁ has a structure represented by formula a-1, 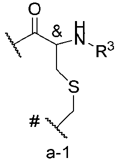
"&" denotes that the carbon atom herein is a chiral carbon, that is, 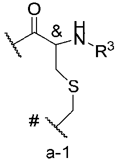 is 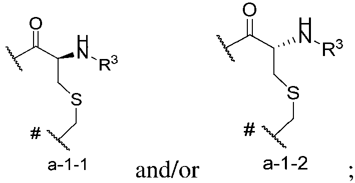 "#" end denotes attachment to B;
wherein R³ is -COC₁₋₁₂alkyl, -COOC₁₋₁₂alkyl, -COC₁₋₁₂alkyl substituted with 1, 2 or 3 R³⁻¹, -COOC₁₋₁₂alkyl substituted with 1, 2 or 3 R³⁻², a group formed by the loss of a -OH group by a carboxyl group on an amino acid, or a group formed by the loss of a -OH group by a carboxyl group on a peptide formed by 2 or more amino acids;
R³⁻¹ and R³⁻² are independently deuterium, -NH₂, -COOH, -CN, halogen, -OH, -NR³⁻¹⁻¹R³⁻¹⁻², or -OC₁₋₁₂alkyl;
R³⁻¹⁻¹ and R³⁻¹⁻² are independently H or C₁₋₁₂alkyl;
A₂ has a structure represented by formula a-2 or a-2-2, the nitrogen end of which is attached to A₁, and the carbonyl end of which is attached to A₃;

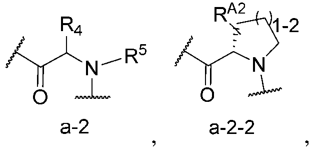

R⁴ and R⁵ are independently H, C₁₋₁₂alkyl or C₁₋₁₂alkyl substituted with 1, 2 or 3 R⁴⁻¹;
R⁴⁻¹ is independently deuterium, -OH, halogen, -NH₂, or -COOH;
R^{A2} is independently H, deuterium, -CH₃, -OH, -NH₂, or F; the number of R^{A2} is 1, 2, 3 or 4;
A₃ has a structure represented by formula a-3, the nitrogen end of which is attached to A₂, and the carbonyl end of which is attached to A₄; 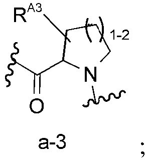
the number of R^{A3} is 1, 2, 3 or 4; optionally A₃ is 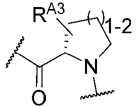 or 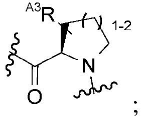
R^{A3} is H, deuterium, -CH₃, -OH, -NH₂ or F;
A₄ has a structure represented by formula a-4, the nitrogen end of which is attached to A₃, and the carbonyl end of which is attached to As; 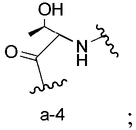
optionally A₄ is 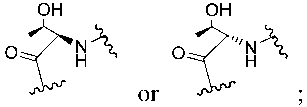
As has a structure represented by formula a-5, the nitrogen end of which is attached to A₄, and the carbonyl end of which is attached to A₆; 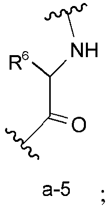
optionally As is 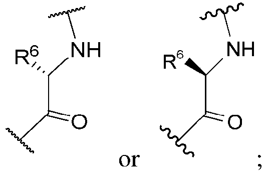
wherein R⁶ is -(CH₂)ₓCOR⁶⁻¹, in which x is 1, 2 or 3, and R⁶⁻¹ is -OH or -NH₂;
A₆ has a structure represented by formula a-6, the nitrogen end of which is attached to As, and the carbonyl end of which is attached to A₇; 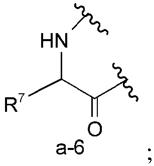
optionally A₆ is 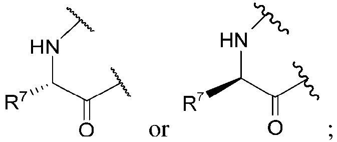
wherein R⁷ is -CH₂C₆₋₁₀aryl, -CH₂-5-10-membered heteroaryl, -CH₂C₃₋₁₂cycloalkyl, - CH₂C₆₋₁₀aryl substituted with 1, 2 or 3 R⁷⁻¹, -CH₂-5-10-membered heteroaryl substituted with 1, 2 or 3 R⁷⁻², or -CH₂C₃₋₁₂cycloalkyl substituted with 1, 2 or 3 R⁷⁻³;
R⁷⁻¹, R⁷⁻² and R⁷⁻³ are independently deuterium, -OH, -NH₂, halogen, -CN, C₁₋₁₂alkyl, or C₁₋₁₂alkyl substituted with 1, 2 or 3 halogen;
in the 5-10-membered heteroaryl, the heteroatom in each 5-10-membered heteroaryl is independently selected from one or more of N, O and S, and the number of the heteroatom is independently 1, 2 or 3.

In one embodiment, in the polypeptide compound, the pharmaceutically acceptable salt thereof, the solvate thereof, or the solvate of the pharmaceutically acceptable salt thereof, some groups can be defined as follows, and others can be defined as described in any embodiment of the present disclosure (hereinafter referred to as "in one embodiment"): 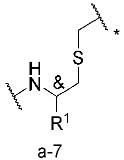 is

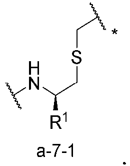

In one embodiment, m is 1 or 2.

In one embodiment, R¹⁻¹ is -OH, -NR^{a}R^{b}, -OC₁₋₁₂alkyl, -OC₃₋₁₂cycloalkyl, -OC₆₋₁₀aryl, a group formed by the loss of a H atom by an amino group on an amino acid, or a group formed by the loss of a H atom by an amino group on a peptide formed by 2 or more amino acids. As an example, R¹⁻¹ is -OH, -NR^{a}R^{b}, -OC₁₋₁₂alkyl (e.g., -OC₁₋₇alkyl, -OC₁₋₆alkyl, -OC₁₋₅alkyl), -OC₃₋₁₂cycloalkyl (e.g., -OC₃₋₅cycloalkyl, -OC₃₋₆cycloalkyl, -OC₃₋₇cycloalkyl), a group formed by the loss of a H atom by an amino group on an amino acid, or a group formed by the loss of a H atom by an amino group on a peptide formed by 2 or more amino acids.

In one embodiment, R^{a} and R^{b} are independently H, C₁₋₁₂alkyl (e.g., C₁₋₇alkyl), C₃₋₁₂cycloalkyl (e.g., C₃₋₇cycloalkyl), C₆₋₁₀aryl, 5-10-membered heteroaryl, or C₁₋₁₂alkyl substituted with 1, 2 or 3 R^{b-1}, C₆₋₁₀aryl substituted with 1, 2 or 3 R^{b-3}.

In one embodiment, R^{b-1} and R^{b-3} are independently deuterium, -OH, -NH₂, -COOH, - CONH₂, -CN, halogen, C₃₋₁₂cycloalkyl (e.g., C₃₋₇cycloalkyl), C₆₋₁₀aryl, 5-10-membered heteroaryl, or C₆₋₁₀aryl substituted with 1, 2 or 3 R^{b-1-1}; for example, R^{e-1} is independently C₃₋₁₂cycloalkyl, C₆₋₁₀aryl, 5-10-membered heteroaryl, or C₆₋₁₀aryl substituted with 1, 2 or 3 R^{b-1-1}.

In one embodiment, R^{b-1-1} is independently C₁₋₁₂alkyl (e.g., C₁₋₆alkyl) substituted with 1, 2 or 3 halogen.

In one embodiment, R¹⁻² and R¹⁻³ are independently H, C₁₋₁₂alkyl (e.g., C₁₋₇alkyl, C₁₋₆alkyl), -COR^{g}, a group formed by the loss of a -OH group by a carboxyl group on an amino acid, or a group formed by the loss of a -OH group by a carboxyl group on a peptide formed by 2 or more amino acids.

In one embodiment, R^{g} is independently C₁₋₁₂alkyl (e.g., C₁₋₇alkyl, C₁₋₆alkyl), C₃₋₁₂cycloalkyl (e.g., C₃₋₇cycloalkyl), C₆₋₁₀aryl, 5-10-membered heteroaryl, C₁₋₁₂alkyl substituted with 1, 2 or 3 R^{g-1}, C₆₋₁₀aryl substituted with 1, 2 or 3 R^{g-3}, or 5-10-membered heteroaryl substituted with 1, 2 or 3 R^{g-4}; for example, R^{g} is independently C₁₋₁₂alkyl, C₃₋₁₂cycloalkyl, C₆₋₁₀aryl, 5-10-membered heteroaryl, C₁₋₁₂alkyl substituted with 1, 2 or 3 R^{g-1}, or C₆₋₁₀aryl substituted with 1, 2 or 3 R^{g-3}.

In one embodiment, R^{g-1}, R^{g-3} and R^{g-4} are independently -OH, -NH₂, halogen, C₁₋₁₂alkyl (e.g., C₁₋₇alkyl, C₁₋₆alkyl, C₁₋₅alkyl, C₁₋₄alkyl), C₆₋₁₀aryl, or C₆₋₁₀aryl substituted with 1, 2 or 3 R^{g-1-1}; for example, R^{g-1} and R^{g-3} are independently halogen, C₆₋₁₀aryl, or C₆₋₁₀aryl substituted with 1, 2 or 3 R^{g-1-1}.

In one embodiment, R^{g-1-1} is independently C₁₋₁₂alkyl (C₁₋₅alkyl, C₁₋₄alkyl, C₁₋₃alkyl) substituted with 1, 2 or 3 halogen.

In one embodiment, R³ is -COC₁₋₁₂alkyl (e.g., -CO(CH₂)₁-₄CH₃), -COC₁₋₁₂alkyl substituted with 1, 2 or 3 R³⁻¹, a group formed by the loss of a -OH group by a carboxyl group on an amino acid, or a group formed by the loss of a -OH group by a carboxyl group on a peptide formed by 2 or more amino acids.

In one embodiment, R³⁻¹ is independently -NH₂ or -OC₁₋₁₂alkyl (e.g., -OC₁₋₅alkyl).

In one embodiment, R⁴ and R⁵ are independently C₁₋₁₂alkyl (e.g., C₁₋₈alkyl, C₁₋₅alkyl, C₁₋₃alkyl). In one embodiment, the nitrogen-containing heterocycle in the structure represented by formula a-2-2 is a five-membered ring.

In one embodiment, x is 2 or 3.

In one embodiment, R⁷ is -CH₂C₆₋₁₀aryl, -CH₂C₆₋₁₀heteroaryl, -CH₂C₃₋₁₂cycloalkyl (e.g., - CH₂C₃₋₇cycloalkyl, -CH₂C₃₋₆cycloalkyl), -CH₂C₆₋₁₀aryl substituted with 1, 2 or 3 R⁷⁻¹, or -CH₂-5-10-membered heteroaryl substituted with 1, 2 or 3 R⁷⁻²; for example, R⁷ is -CH₂C₆₋₁₀aryl, -CH₂C₃₋₁₂cycloalkyl, or -CH₂C₆₋₁₀aryl substituted with 1, 2 or 3 R⁷⁻¹.

In one embodiment, R⁷⁻¹ and R⁷⁻² are independently deuterium, -OH, -NH₂, halogen, C₁₋₃alkyl, or C₁₋₁₂alkyl (e.g., C₁₋₈alkyl, C₁₋₆alkyl, C₁₋₅alkyl) substituted with 1, 2 or 3 halogen, for example, R⁷⁻¹ is independently C₁₋₁₂alkyl substituted with 1, 2 or 3 halogen.

In one embodiment, 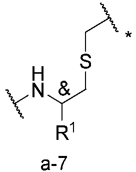 is 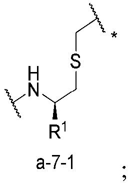
m is 1 or 2;
R¹⁻¹ is -OH, -NR^{a}R^{b}, -OC₁₋₁₂alkyl (e.g., -OC₃₋₉alkyl, -OC₃₋₆alkyl), -OC₃₋₁₂cycloalkyl (e.g., -OC₃₋₇cycloalkyl, -OC₃₋₆cycloalkyl), a group formed by the loss of a H atom by an amino group on an amino acid, or a group formed by the loss of a H atom by an amino group on a peptide formed by 2 or more amino acids;
R^{a} and R^{b} are independently H, C₁₋₁₂alkyl (e.g., C₁₋₆alkyl, C₁₋₄alkyl, C₁₋₃alkyl), C₃₋₁₂cycloalkyl (e.g., C₃₋₇cycloalkyl, C₃₋₆cycloalkyl, C₃₋₅cycloalkyl), C₆₋₁₀aryl, or C₁₋₁₂alkyl substituted with 1, 2 or 3 R^{b-1};
R^{e-1} is independently C₃₋₁₂cycloalkyl (e.g., C₃₋₇cycloalkyl, C₃₋₆cycloalkyl, C₃₋₅cycloalkyl), C₆₋₁₀aryl, 5-10-membered heteroaryl, C₆₋₁₀aryl substituted with 1, 2 or 3 R^{b-1-1};
R^{b-1-1} is independently C₁₋₁₂alkyl (e.g., C₁₋₆alkyl, C₁₋₄alkyl, C₁₋₃alkyl) substituted with 1, 2 or 3 halogen;
R¹⁻² and R¹⁻³ are independently H, C₁₋₁₂alkyl (e.g., C₁₋₆alkyl, C₁₋₄alkyl, C₁₋₃alkyl), -COR^{g}, a group formed by the loss of a -OH group by a carboxyl group on an amino acid, or a group formed by the loss of a -OH group by a carboxyl group on a peptide formed by 2 or more amino acids;
R^{g} is independently C₁₋₁₂alkyl (e.g., C₁₋₈alkyl, C₁₋₆alkyl, C₁₋₄alkyl, C₁₋₃alkyl), C₃₋₁₂cycloalkyl, C₆₋₁₀aryl, 5-10-membered heteroaryl, C₁₋₁₂alkyl substituted with 1, 2 or 3 R^{g-1}, or C₆₋₁₀aryl substituted with 1, 2 or 3 R^{g-3};
R^{g-1} and R^{g-3} are independently halogen, C₆₋₁₀aryl, or C₆₋₁₀aryl substituted with 1, 2 or 3 R^{g-1-1};
R^{g-1-1} is independently C₁₋₁₂alkyl (e.g., C₁₋₈alkyl, C₁₋₆alkyl, C₁₋₄alkyl, C₁₋₃alkyl) substituted with 1, 2 or 3 halogen;
R³ is -COC₁₋₁₂alkyl, -COC₁₋₁₂alkyl (e.g., -COC₁₋₈alkyl, -COC₁₋₆alkyl, -COC₁₋₅alkyl) substituted with 1, 2 or 3 R³⁻¹, a group formed by the loss of a -OH group by a carboxyl group on an amino acid, or a group formed by the loss of a -OH group by a carboxyl group on a peptide formed by 2 or more amino acids;
R³⁻¹ is independently -NH₂ or -OC₁₋₁₂alkyl (e.g., -COC₁₋₅alkyl, -COC₁₋₄alkyl);
R⁴ and R⁵ are independently C₁₋₁₂alkyl;
the nitrogen-containing heterocycle in formula a-2-2 is a five-membered ring, and R^{A2} is independently H, deuterium, -CH₃, -OH, -NH₂, or F; the number of R^{A2} is 1, 2 or 3;
x is 1 or 2;
R⁷ is -CH₂C₆₋₁₀aryl, -CH₂C₃₋₁₂cycloalkyl (e.g., -CH₂C₃₋₁₀cycloalkyl, -CH₂C₃₋₈cycloalkyl, - CH₂C₃₋₇cycloalkyl, -CH₂C₃₋₆cycloalkyl), or -CH₂C₆₋₁₀aryl substituted with 1, 2 or 3 R⁷⁻¹;
R⁷⁻¹ is independently C₁₋₁₂alkyl (e.g., C₁₋₈alkyl, C₁₋₇alkyl, C₁₋₆alkyl, C₁₋₅alkyl) substituted with 1, 2 or 3 halogen.

In any embodiment of the present application, in each substituent of A₁ - A₇ and B, the C₁₋₁₂alkyl is each independently selected from C₁₋₁₂alkyl, C₁₋₁₁alkyl, C₁₋₁₀alkyl, C₁₋₉alkyl, C₁₋₈alkyl, C₁₋₇alkyl, C₁₋₆alkyl, C₁₋₅alkyl, C₁₋₄alkyl, C₁₋₃alkyl, or C₁₋₂alkyl; the C₃₋₁₂cycloalkyl is each independently selected from C₃₋₁₂cycloalkyl, C₃₋₁₁cycloalkyl, C₃₋₁₀cycloalkyl, C₃₋₉cycloalkyl, C₃₋₈cycloalkyl, C₃₋₇cycloalkyl, C₃₋₆cycloalkyl, C₃₋₅cycloalkyl, or C₃₋₄cycloalkyl; the C₆₋₁₀aryl is each independently selected from C₆₋₁₀aryl, C₆₋₉aryl, C₆₋₈aryl, or C₆₋₇aryl; and the 5-10-membered heteroaryl is each independently selected from 5-10-membered heteroaryl, 5-9-membered heteroaryl, 5-8-membered heteroaryl, 5-7-membered heteroaryl, or 5-6-membered heteroaryl.

In one embodiment, in R¹⁻¹, the C₁₋₁₂alkyl in the -OC₁₋₁₂alkyl is C₁₋₆alkyl, for example n-propyl, isopropyl, n-butyl, isobutyl, or tert-butyl; further for example tert-butyl.

In one embodiment, in R¹⁻¹, the C₃₋₁₂cycloalkyl in the -OC₃₋₁₂cycloalkyl is C₃₋₆cycloalkyl, for example cyclopentyl.

In one embodiment, in R¹⁻¹, the C₆₋₁₀aryl in the -OC₆₋₁₀aryl is phenyl or naphthyl.

In one embodiment, in R¹⁻¹, the group formed by the loss of a H atom by an amino group on an amino acid is 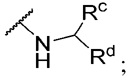
R^{c} is -(CH₂)ₒCOOH or -(CH₂)ₚCONH₂;
o and p are independently 0, 1, 2, 3 or 4; for example, o and p are independently 0 or 1;
R^{d} is hydrogen, deuterium, C₁₋₁₂alkyl (e.g., C₁₋₆alkyl, C₁₋₄alkyl, C₁₋₃alkyl), C₃₋₁₂cycloalkyl (e.g., C₃₋₇cycloalkyl, C₃₋₆cycloalkyl, C₃₋₅cycloalkyl), C₆₋₁₀aryl, 5-10-membered heteroaryl, C₁₋₁₂alkyl substituted with 1, 2 or 3 R^{d-1}, C₃₋₁₂cycloalkyl substituted with 1, 2 or 3 R^{d-2}, C₆₋₁₀aryl substituted with 1, 2 or 3 R^{d-3}, or 5-10-membered heteroaryl substituted with 1, 2 or 3 R^{d-4}; for example, R^{d} is C₆₋₁₀aryl, further for example naphthyl;
R^{d-1}, R^{d-2}, R^{d-3} and R^{d-4} are independently C₁₋₁₂alkyl (e.g., C₁₋₆alkyl, C₁₋₄alkyl, C₁₋₃alkyl), C₃₋₁₂cycloalkyl (e.g., C₃₋₇cycloalkyl, C₃₋₆cycloalkyl, C₃₋₅cycloalkyl), or C₆₋₁₀aryl.

In one embodiment, in R¹⁻¹, the peptide formed by 2 or more amino acids is a peptide formed by 2 to 20 amino acids, for example, a peptide formed by 2 to 18, 2 to 16, 2 to 14, 2 to 12, 2 to 10, 2 to 8, 2 to 6, 2 to 5, 2 to 4, 2 to 3 amino acids.

In one embodiment, in R^{a} and R^{b}, the C₁₋₁₂alkyl or the C₁₋₁₂alkyl in the C₁₋₁₂alkyl substituted with 1, 2 or 3 R^{b-1} is independently C₁₋₆alkyl, for example methyl, ethyl, n-propyl, isopropyl, n-butyl, isobutyl, t-butyl or n-pentyl; further for example methyl or n-pentyl.

In one embodiment, in R^{a} and R^{b}, the C₃₋₁₂cycloalkyl is independently C₃₋₆cycloalkyl, for example cyclopropyl, cyclobutyl, cyclopentyl, cyclohexyl or 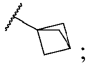 further for example cyclopentyl or

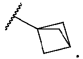

In one embodiment, in R^{a} and R^{b}, the C₆₋₁₀aryl is independently phenyl, biphenyl or naphthyl; for example phenyl.

In one embodiment, in R^{a} and R^{b}, the number of the heteroatom in the 5-10-membered heteroaryl is 1 or 2; for example furyl, thienyl, imidazolyl, pyridyl, or quinolinyl.

In one embodiment, in R^{b-1}, the C₃₋₁₂cycloalkyl is independently C₃₋₆cycloalkyl, for example cyclopropyl, cyclobutyl, cyclopentyl, or cyclohexyl; further for example cyclopentyl.

In one embodiment, in R^{b-1}, the C₆₋₁₀aryl or the C₆₋₁₀aryl in the C₆₋₁₀aryl substituted with 1, 2 or 3 R^{b-1-1} is independently phenyl or naphthyl; for example phenyl.

In one embodiment, in R^{b-1}, the 5-10-membered heteroaryl is independently 5-6 membered heteroaryl.

In one embodiment, in R^{b-1}, the number of the heteroatom in the 5-10-membered heteroaryl is 1 or 2.

In one embodiment, in R^{b-1-1}, the halogen or the halogen in the C₁₋₁₂alkyl substituted with 1, 2 or 3 halogen is independently F, Cl, Br or I, for example F.

In one embodiment, in R^{b-1-1}, the C₁₋₁₂alkyl in the C₁₋₁₂alkyl substituted with 1, 2 or 3 halogen is independently C₁₋₆alkyl, for example methyl, ethyl, n-propyl, isopropyl, n-butyl, isobutyl, tert-butyl, or n-pentyl; further for example methyl.

In one embodiment, in R¹⁻² and R¹⁻³, the C₁₋₁₂alkyl is independently C₁₋₆alkyl, for example methyl, ethyl, n-propyl, isopropyl, n-butyl, isobutyl, tert-butyl, n-pentyl, or n-hexyl; further for example methyl, isopropyl, n-pentyl or n-hexyl.

In one embodiment, in R¹⁻² and R¹⁻³, the C₃₋₁₂cycloalkyl or the C₃₋₁₂cycloalkyl in the C₃₋₁₂cycloalkyl substituted with 1, 2 or 3 R^{h-1} is independently C₃₋₆cycloalkyl, for example cyclopropyl, cyclobutyl, cyclopentyl, or cyclohexyl.

**In** one embodiment, in R¹⁻² and R¹⁻³, the C₆₋₁₀aryl or the C₆₋₁₀aryl in the C₆₋₁₀aryl substituted with 1, 2 or 3 R^{h-2} is independently phenyl or naphthyl.

**In** one embodiment, in R¹⁻² and R¹⁻³, the number of the heteroatom in the 5-10-membered heteroaryl or the 5-10-membered heteroaryl in the 5-10-membered heteroaryl substituted with 1, 2 or 3 R^{h-3} is 1 or 2.

**In** one embodiment, in R¹⁻² and R¹⁻³, the peptide formed by 2 or more amino acids is a peptide formed by 2 to 20 amino acids, for example a peptide formed by 2 to 10 amino acids.

**In** one embodiment, in R^{g}, the C₁₋₁₂alkyl or the C₁₋₁₂alkyl in the C₁₋₁₂alkyl substituted with 1, 2 or 3 R^{g-1} is independently C₁₋₆alkyl, for example methyl, ethyl, n-propyl, isopropyl, n-butyl, isobutyl, tert-butyl, n-pentyl, or n-hexyl; further for example methyl, ethyl or n-pentyl.

**In** one embodiment, in R^{g}, the C₃₋₁₂cycloalkyl is independently C₃₋₆cycloalkyl, for example cyclopropyl, cyclobutyl, cyclopentyl, cyclohexyl or 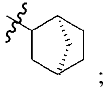 for example cyclopentyl or cyclohexyl.

In one embodiment, in R^{g}, the C₆₋₁₀aryl or the C₆₋₁₀aryl in the C₆₋₁₀aryl substituted with 1, 2 or 3 R^{g-3} is independently phenyl or naphthyl; for example phenyl.

In one embodiment, in R^{g}, the 5-10-membered heteroaryl or the 5-10-membered heteroaryl in the 5-10-membered heteroaryl substituted with 1, 2 or 3 R^{g-4} is 5-6-membered heteroaryl.

In one embodiment, in R^{g-1}, R^{g-3} and R^{g-4}, the halogen is independently F, Cl, Br or I; for example Cl.

In one embodiment, in R^{g-1}, R^{g-3} and R^{g-4}, the C₆₋₁₀aryl or the C₆₋₁₀aryl in the C₆₋₁₀aryl substituted with 1, 2 or 3 R^{g-1-1} is independently phenyl or naphthyl; for example phenyl.

In one embodiment, in R^{g-1-1}, the C₁₋₁₂alkyl in the C₁₋₁₂alkyl substituted with 1, 2 or 3 halogen is independently C₁₋₆alkyl, for example methyl, ethyl, n-propyl, isopropyl, n-butyl, isobutyl, tert-butyl, or n-pentyl; further for example methyl.

In one embodiment, in R^{g-1-1}, the halogen in the C₁₋₁₂alkyl substituted with 1, 2 or 3 halogen is independently F, Cl, Br or I, for example F.

In one embodiment, in R², the C₁₋₁₂alkyl is independently C₁₋₆alkyl, for example methyl, ethyl, n-propyl, isopropyl, n-butyl, isobutyl, tert-butyl, n-pentyl, or n-hexyl; further for example methyl.

In one embodiment, in R², the halogen is independently F, Cl, Br or I; for example F.

In one embodiment, in R³, the -COC₁₋₁₂alkyl or the C₁₋₁₂alkyl in the -COC₁₋₁₂alkyl substituted with 1, 2 or 3 R³⁻¹ is independently C₁₋₆alkyl, for example methyl, ethyl, n-propyl, isopropyl, n-butyl, isobutyl, tert-butyl, n-pentyl, or n-hexyl; further for example methyl, ethyl or n-pentyl.

In one embodiment, in R³, the peptide formed by 2 or more amino acids is a peptide formed by 2 to 20 amino acids, for example a peptide formed by 2 to 10 amino acids.

In one embodiment, in R³⁻¹, the C₁₋₁₂alkyl in the -OC₁₋₁₂alkyl is independently C₁₋₆alkyl, for example methyl, ethyl, n-propyl, isopropyl, n-butyl, isobutyl, tert-butyl, n-pentyl, or n-hexyl; further for example methyl.

In one embodiment, in R⁴ and R⁵, the C₁₋₁₂alkyl is independently C₁₋₆alkyl, for example methyl, ethyl, n-propyl, isopropyl, n-butyl, isobutyl, tert-butyl, n-pentyl, or n-hexyl; further for example methyl.

In one embodiment, in R⁷, the C₃₋₁₂cycloalkyl in the -CH₂C₃₋₁₂cycloalkyl is independently C₃₋₆cycloalkyl, for example cyclopropyl, cyclobutyl, cyclopentyl, or cyclohexyl; for example cyclohexyl.

In one embodiment, in R⁷, the -CH₂C₆₋₁₀aryl or the C₆₋₁₀aryl in the C₆₋₁₀aryl substituted with 1, 2 or 3 R⁷⁻¹ is independently phenyl or naphthyl.

In one embodiment, in R⁷, the 5-10-membered heteroaryl in the -CH₂-5-10-membered heteroaryl substituted with 1, 2 or 3 R⁷⁻² is 5-6-membered heteroaryl.

In one embodiment, in R⁷, the number of the heteroatom in the 5-10-membered heteroaryl in the -CH₂-5-10-membered heteroaryl substituted with 1, 2 or 3 R⁷⁻² is 1 or 2.

In one embodiment, in R⁷⁻¹, the halogen in the C₁₋₁₂alkyl substituted with 1, 2 or 3 halogen is independently F, Cl, Br or I, for example F.

In one embodiment, in R⁷⁻¹, the C₁₋₁₂alkyl in the C₁₋₁₂alkyl substituted with 1, 2 or 3 halogen is independently C₁₋₆alkyl, for example methyl, ethyl, n-propyl, isopropyl, n-butyl, isobutyl, tert-butyl, or n-pentyl; further for example methyl.

In one embodiment, B is

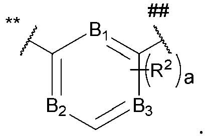

In one embodiment, R¹ is -CH₂COOH, -CH₂CH₂COOH, 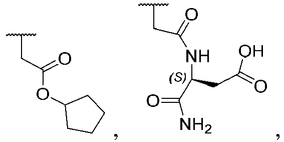 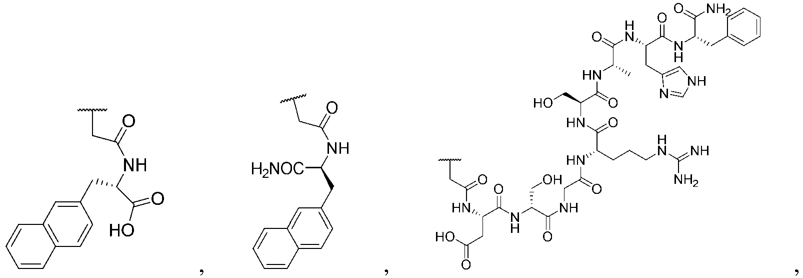 -CH₂CONH₂, 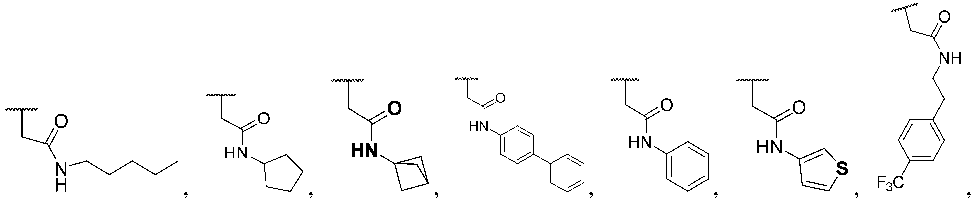 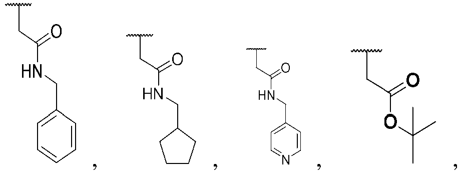 -CH₂NH₂, -CH₂NHCOCH₃, -CH₂CH₂NH₂, - CH₂NH(CH₂)₄CH₃, -CH₂NH(CH₂)sCH₃,

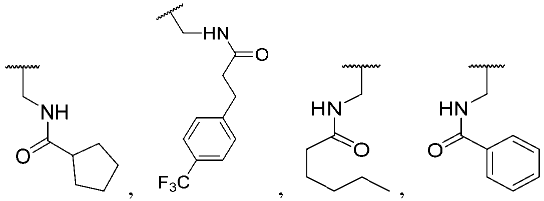

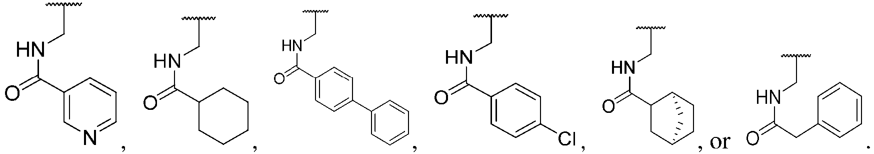

In one embodiment, R³ is 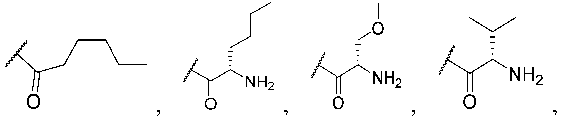 or 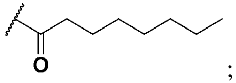 for example

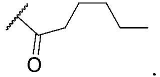

In one embodiment, B is 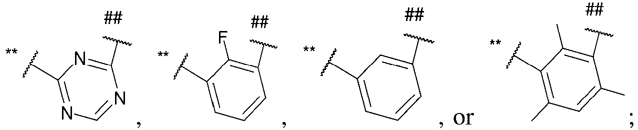 for example

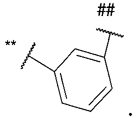

In one embodiment, 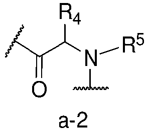 is 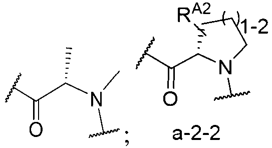 is

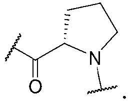

In one embodiment, 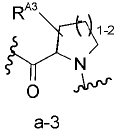 is 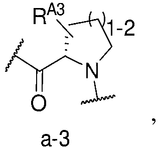 optionally 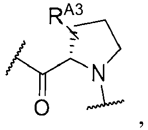 for example

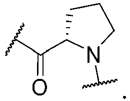

In one embodiment, 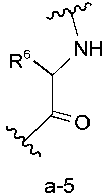 is 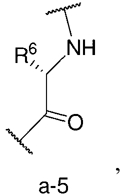 optionally 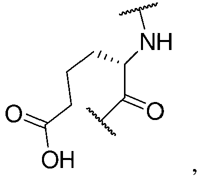 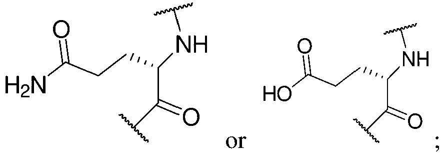 for example

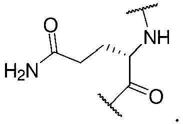

In one embodiment, 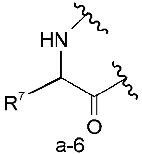 is 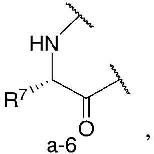 optionally 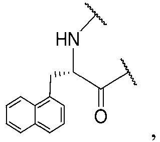 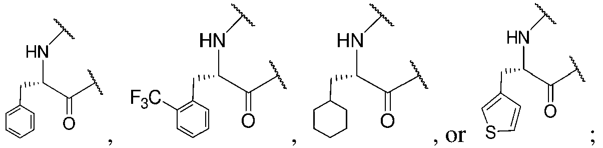 for example

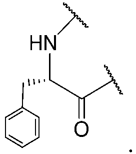

In one embodiment, the polypeptide compound is selected from any of the following compounds: 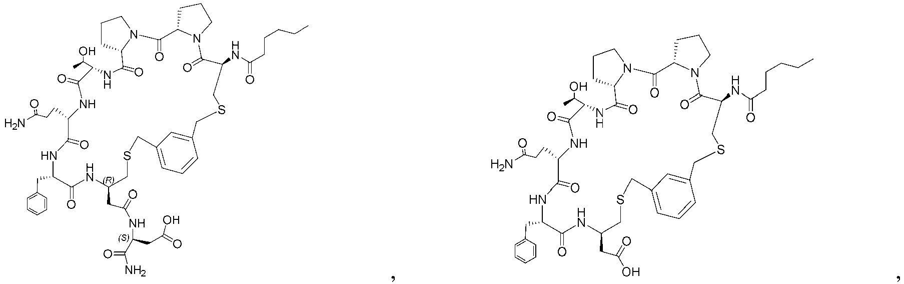 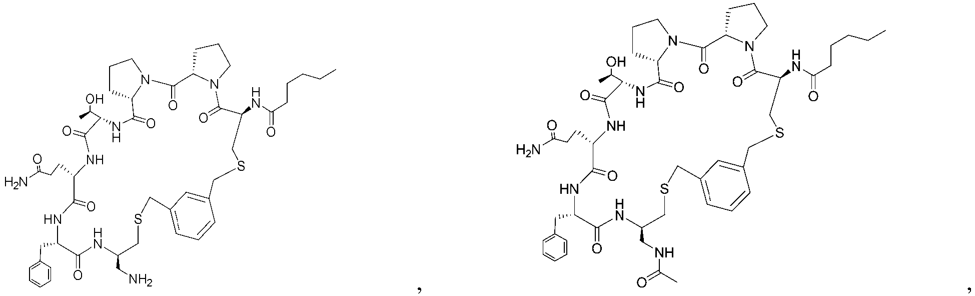 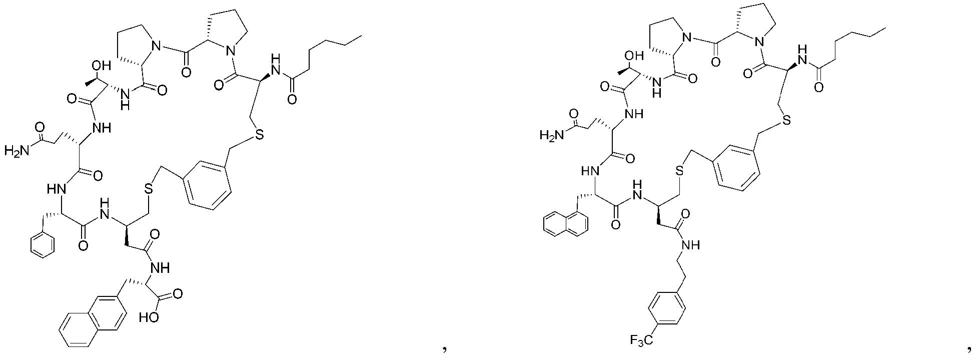 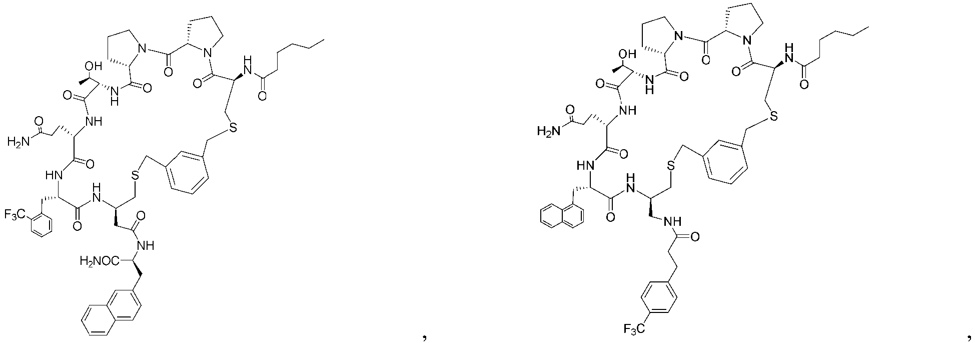 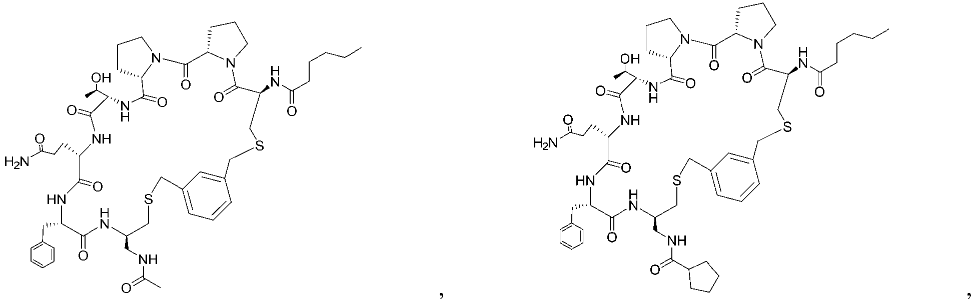 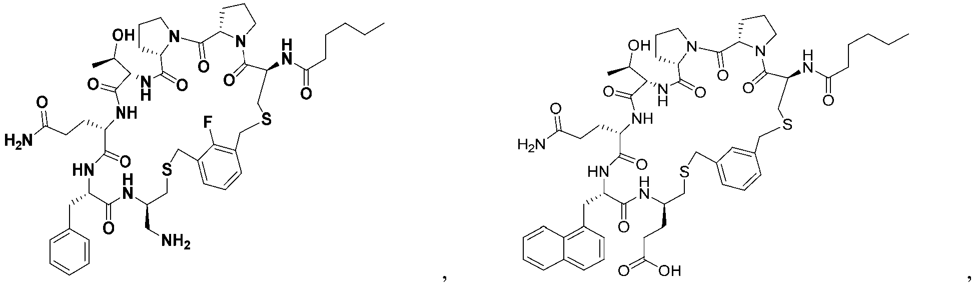 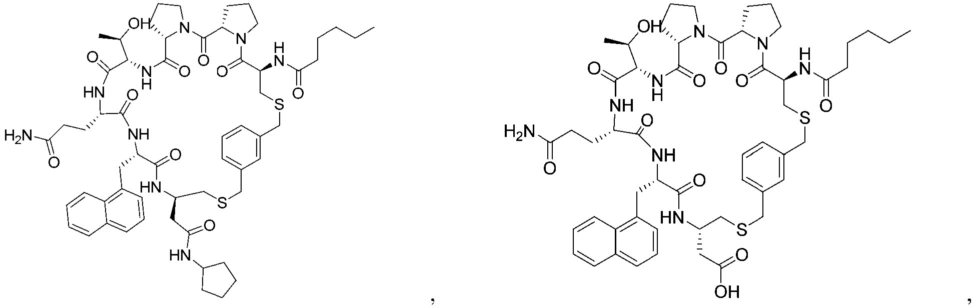 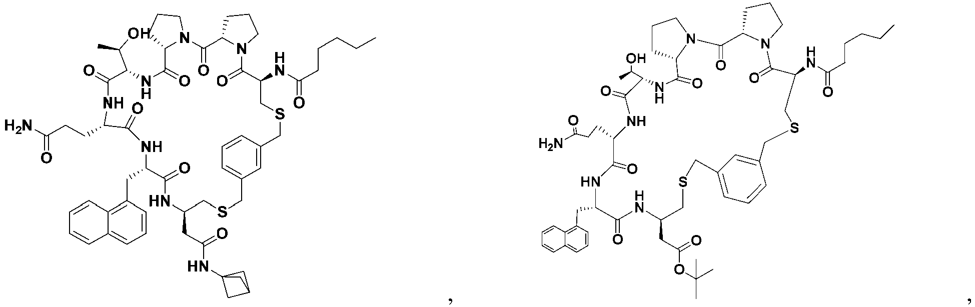 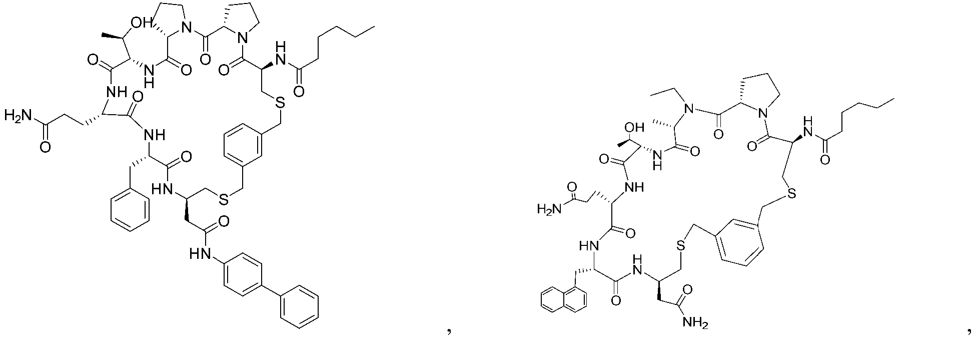 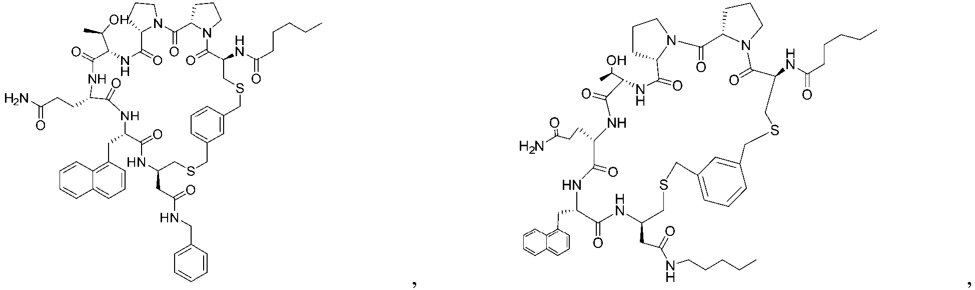 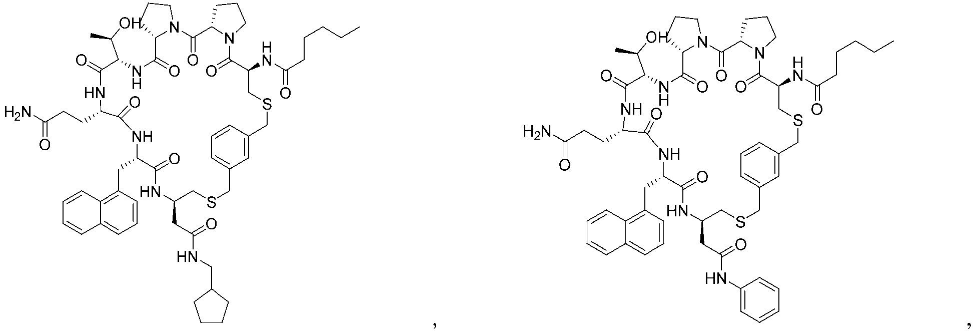 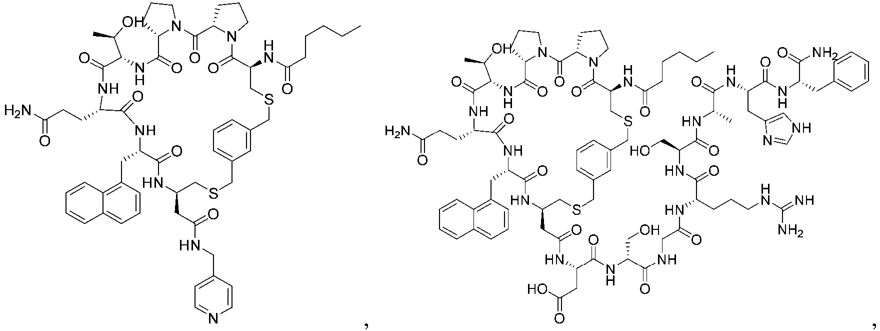 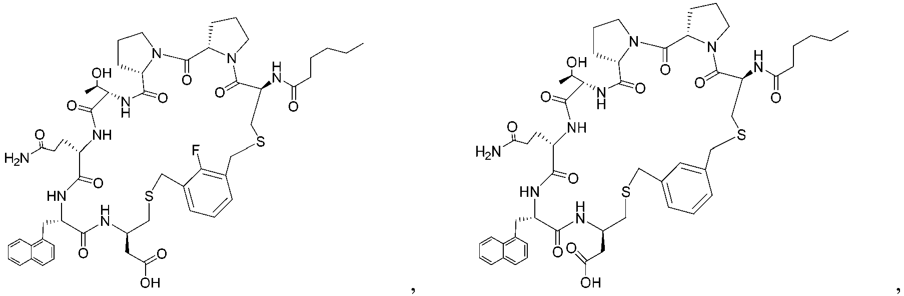 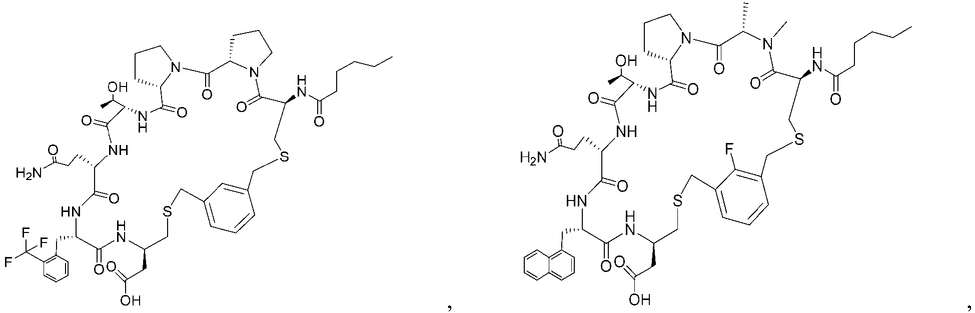 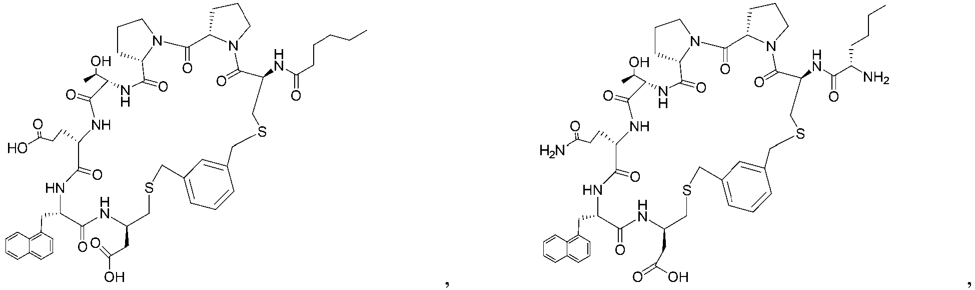 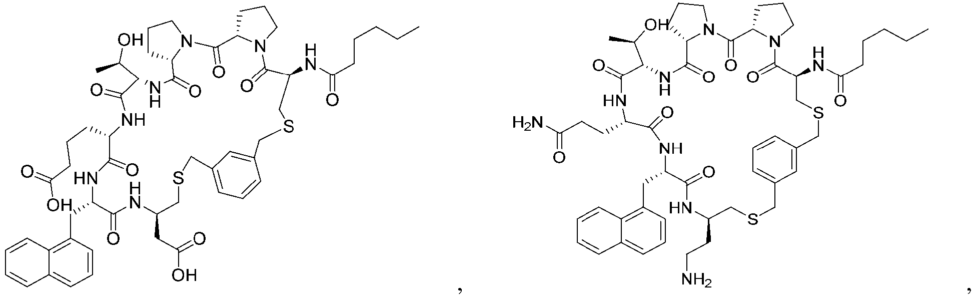 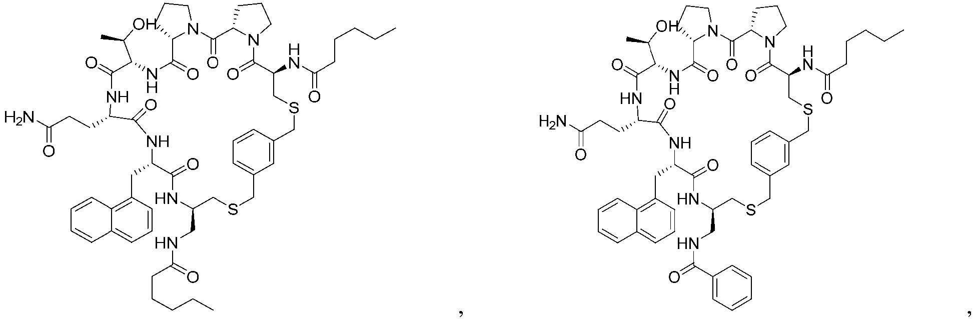 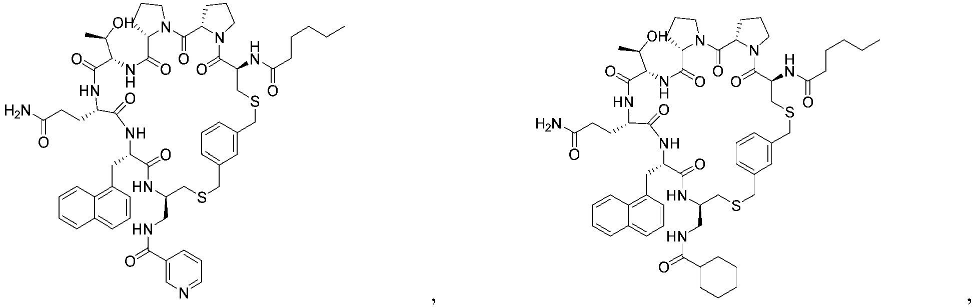 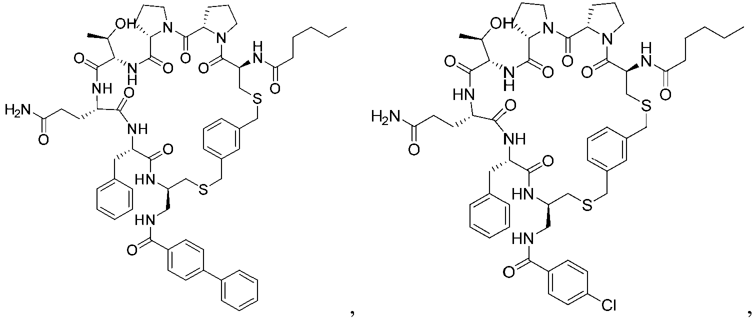 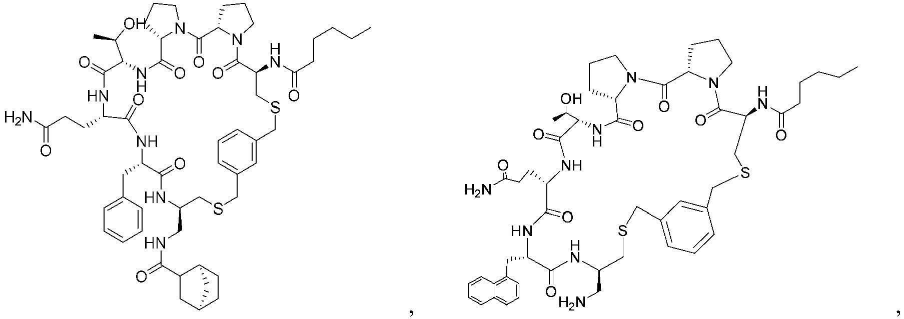 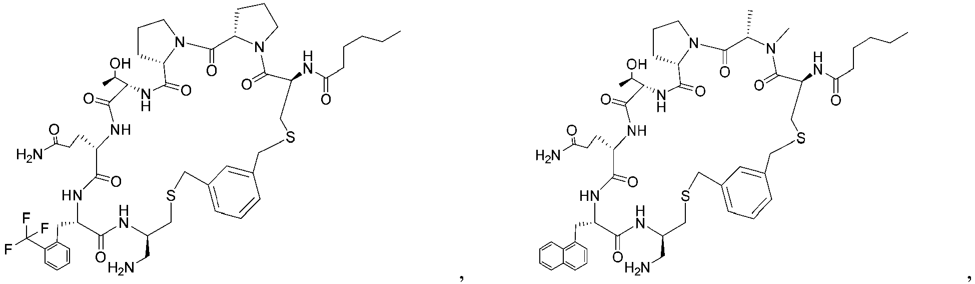 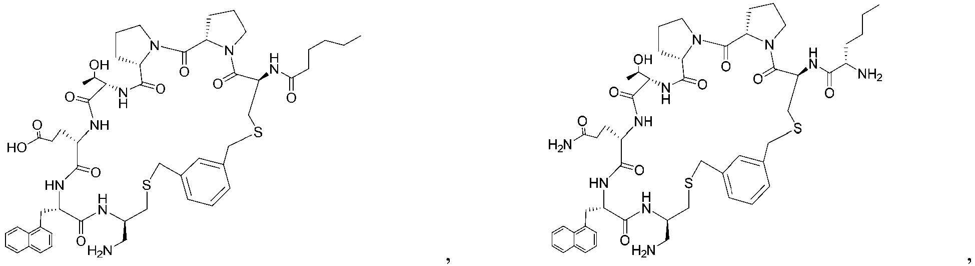 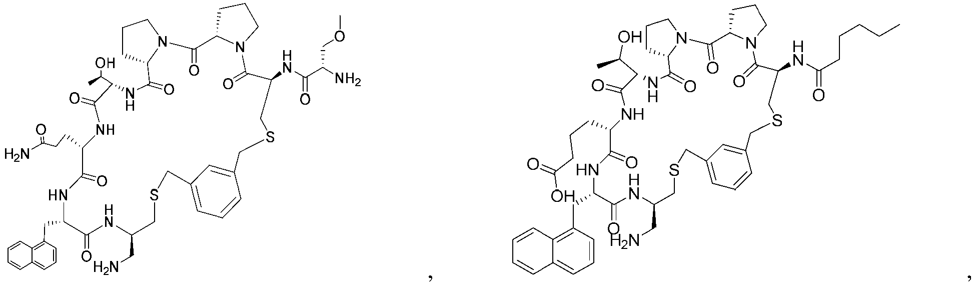 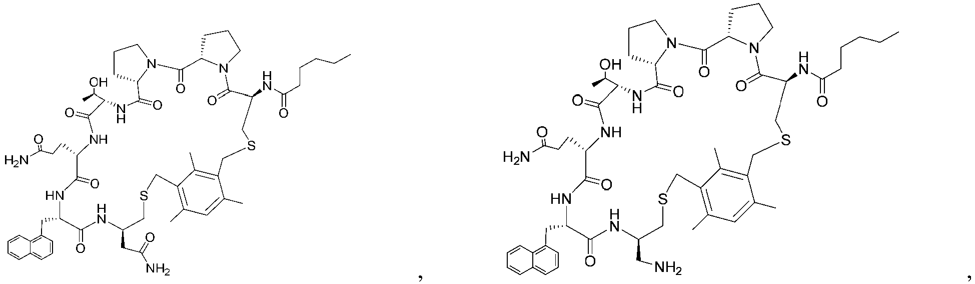 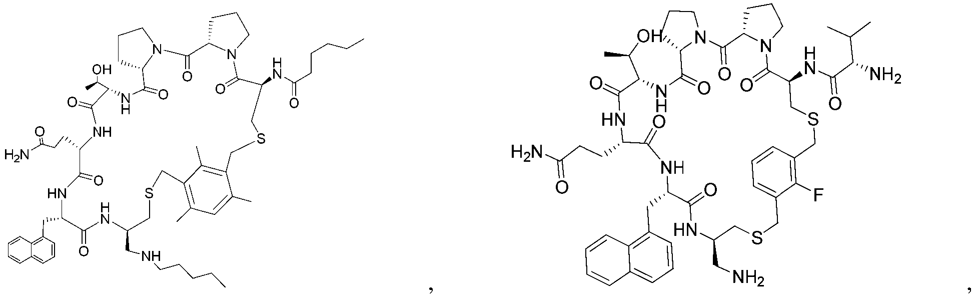 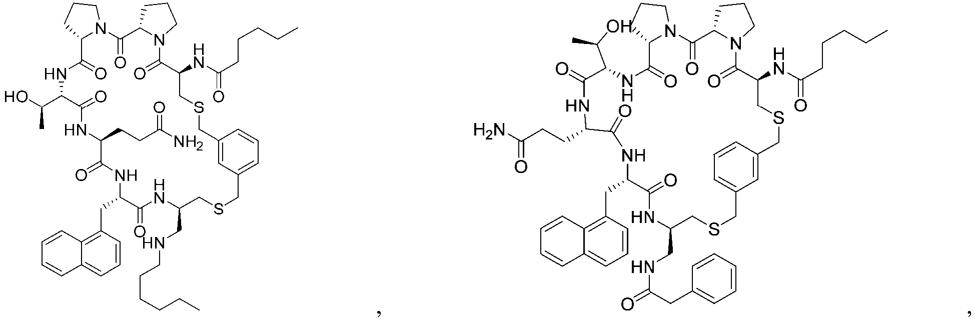 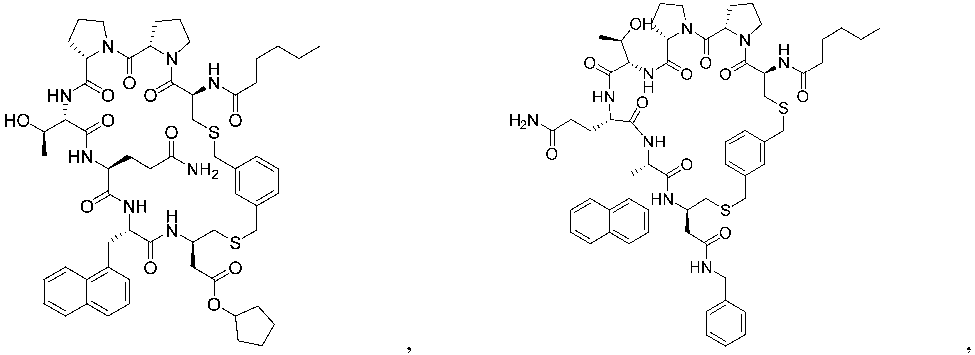 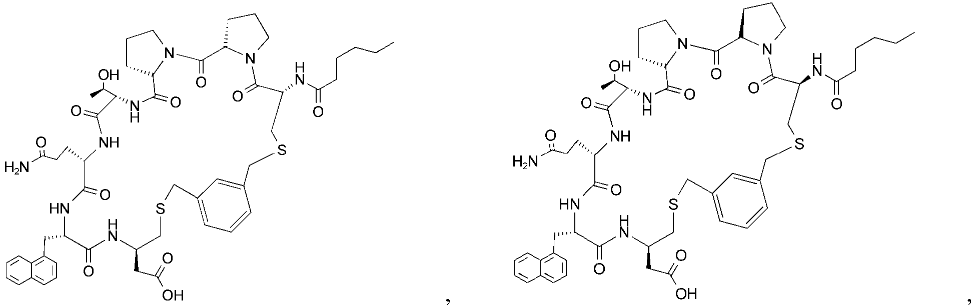 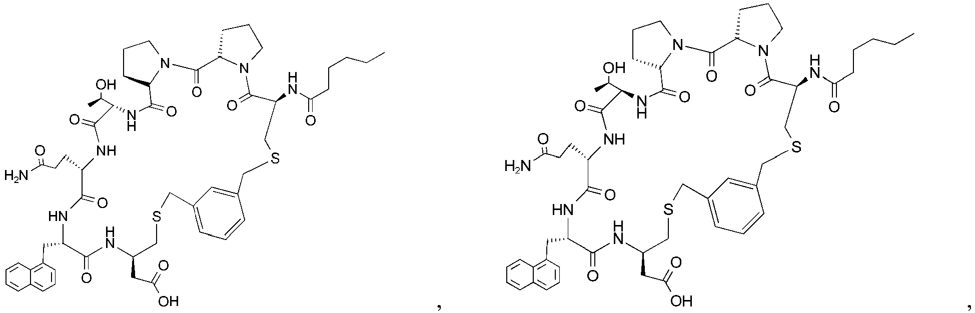 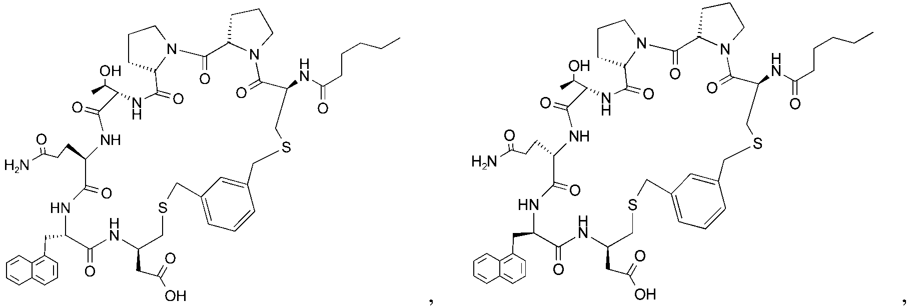 and

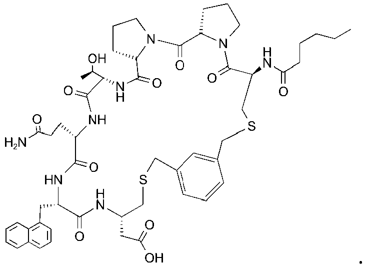

In a second aspect, the present disclosure further provides a polypeptide compound, a pharmaceutically acceptable salt thereof, a solvate thereof or a solvate of a pharmaceutically acceptable salt thereof, wherein the polypeptide compound is a compound represented by formula I-B; wherein A₁, A₂, A₃, A₄, A₅, A₆ and A₇ are sequentially connected via a peptide bond (-CO-NH-); 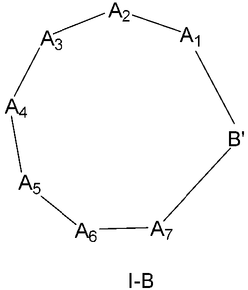
R¹ is -(CH₂)_{m'}M;
m' is 0, 1, 2, 3, 4, 5, or 6;
B' is 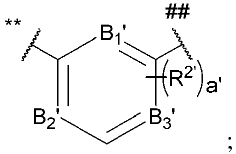
a' is 1, 2 or 3;
B₁' is CH, and B₂' and B₃' are independently N or C; R²' is independently H, C₁₋₁₂alkyl or halogen, and at least one R²' is halogen;
or B₁', B₂' and B₃' are independently N or CH; R²' is independently C₁₋₁₂alkyl or halogen; and
other groups are defined as for the groups in the compound represented by formula I.

In one embodiment, in B, the C₁₋₁₂alkyl is selected from C₁₋₆alkyl, C₁₋₅alkyl, C₁₋₄alkyl, C₁₋₃alkyl, or C₁₋₂alkyl.

In one embodiment, B' is

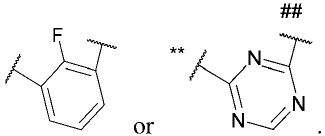

In one embodiment, R¹ is -COOH.

In one embodiment, the compound represented by formula I-B is selected from any of the following compounds:

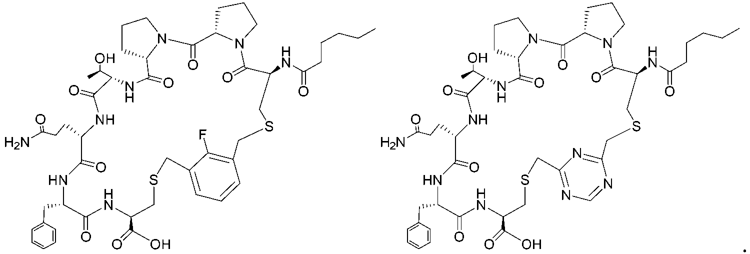

In a third aspect, the present disclosure further provides a polypeptide compound, a pharmaceutically acceptable salt thereof, a solvate thereof or a solvate of a pharmaceutically acceptable salt thereof, wherein the polypeptide compound is a compound represented by formula I-C; wherein A₁, A₂, A₃, A₄, A₅, A₆' and A₇ are sequentially connected via a peptide bond (-CO-NH-); 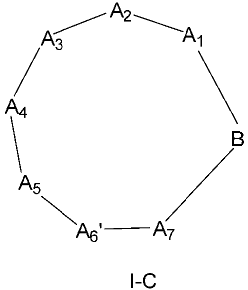
R¹ is -(CH₂)_{m'}M;
m' is 0, 1, 2, 3, 4, 5, or 6;
A₆' has a structure represented by formula a-6',

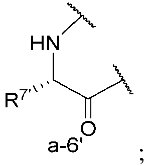

wherein R^{7'} is -CH₂C₃₋₁₂cycloalkyl (e.g., -CH₂C₃₋₇cycloalkyl, -CH₂C₃₋₆cycloalkyl), C₆₋₁₀aryl substituted with 1, 2 or 3 R⁷⁻¹, or -CH₂C₃₋₁₂cycloalkyl substituted with 1, 2 or 3 R⁷⁻²;
R⁷⁻¹ and R⁷⁻² are independently C₁₋₁₂alkyl (e.g., C₁₋₆alkyl, C₁₋₄alkyl, C₁₋₃alkyl, C₁₋₂alkyl), or C₁₋₁₂alkyl substituted with 1, 2 or 3 halogen; and
other groups are defined as for the groups in the compound represented by formula I.

In one embodiment, R^{7'} is -CH₂C₃₋₁₂cycloalkyl, or C₆₋₁₀aryl substituted with 1, 2 or 3 R⁷⁻¹, for example -CH₂cyclohexyl or

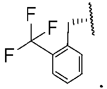

In one embodiment, R¹ is -COOH.

In one embodiment, the compound represented by formula I-C is the following compound:

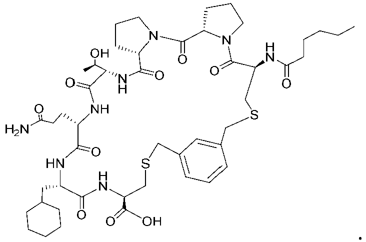

In a fourth aspect, the present disclosure further provides a polypeptide compound, a pharmaceutically acceptable salt thereof, a solvate thereof or a solvate of a pharmaceutically acceptable salt thereof, wherein the polypeptide compound is represented by formula II,

D-L; II

D is the compound represented by formula I of any one of preceding embodiments or a group formed by the loss of a H atom or OH group at the position of B, R¹ or R³ of the compound represented by formula I of any one of preceding embodiments, a group formed by the loss of a H atom or OH group at the position of B', R¹ or R³ of the compound of any one of preceding embodiments, or a group formed by the loss of a H atom or OH group at the position of B, R¹ or R³ of the compound represented by formula I-C of any one of the preceding embodiments; and
L is a non-cleavable linker.

In one embodiment, L is: -A, -W, -W-A, -A-W, -W-A-W, or -A-W-A;
A is a group formed by the loss of a OH group by a carboxyl group on an amino acid, a group formed by the loss of a H atom by an amino group on an amino acid, a group formed by the loss of a -OH group by a carboxyl group on a peptide formed by more than 2 amino acids (for example 2 to 15, 2 to 12, 2 to 10, 2 to 8, 2 to 6, 2 to 5, 2 to 4, 2 to 3 amino acids), or a group formed by the loss of a H atom by an amino group on a peptide formed by 2 or more amino acids,
W is -(Y)_{g-}(CH₂)_{c}-NHR^{t};
Y is independently selected from any one or a combination of the following groups: - (CH₂)_{b}-X-, -(CH₂)_{c}-C₆₋₁₀arylene-(CH₂)_{d}-, -(CH₂)_{c}-5-12-membered heterocycloalkyl-(CH₂)_{d}-, - (CH₂)_{c}-C₃₋₁₂cycloalkylene-(CH₂)_{d}-, and -(CH₂)_{c}-CO-(CH₂)_{d}-; the heteroatom of the 5 to 12-membered heterocycloalkyl is selected from one or more of N, O and S; the number of the heteroatom is 1, 2 or 3;
X is S, O or NH;
R^{t} is H or C₁₋₁₂alkyl (e.g., C₁₋₃alkyl, C₁₋₅alkyl, C₁₋₆alkyl), or R^{t} together with -NH forms a 5-10-membered nitrogen-containing heterocycloalkyl;
g, b, c and d are independently 0, 1, 2, 3, 4, 5, 6, 7, 8, 9, and 10.

In one embodiment, D is selected from any of the following segments:

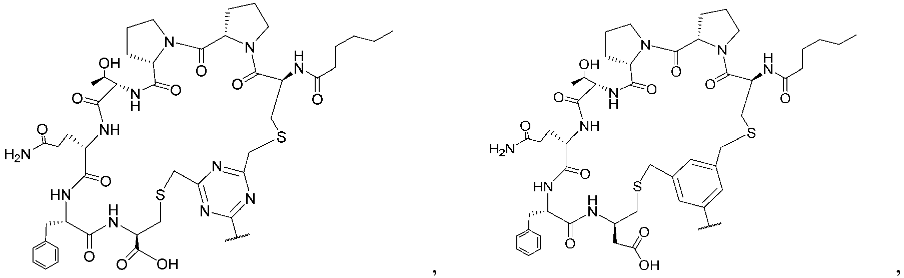

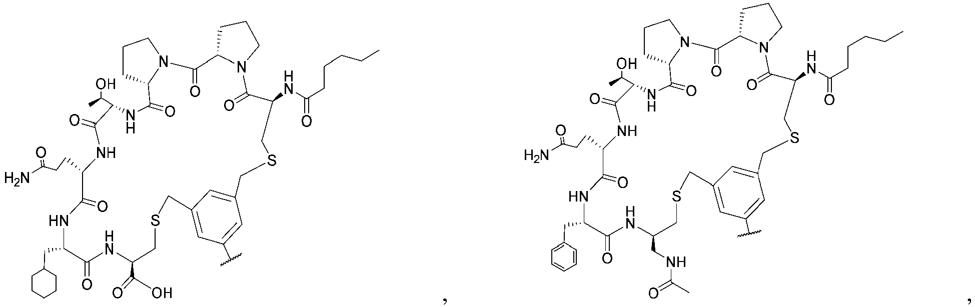

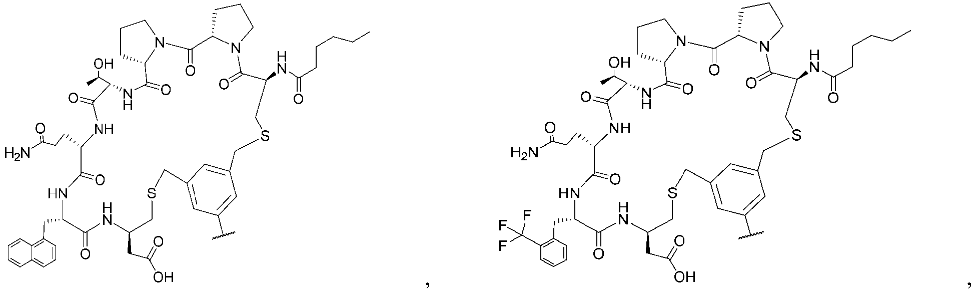

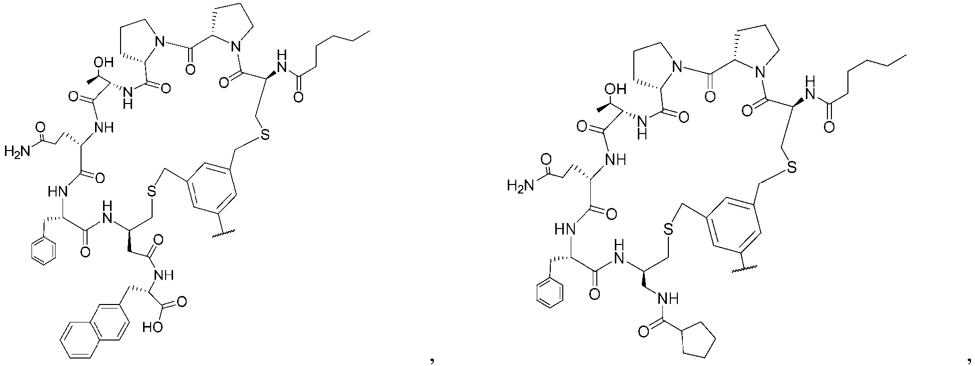

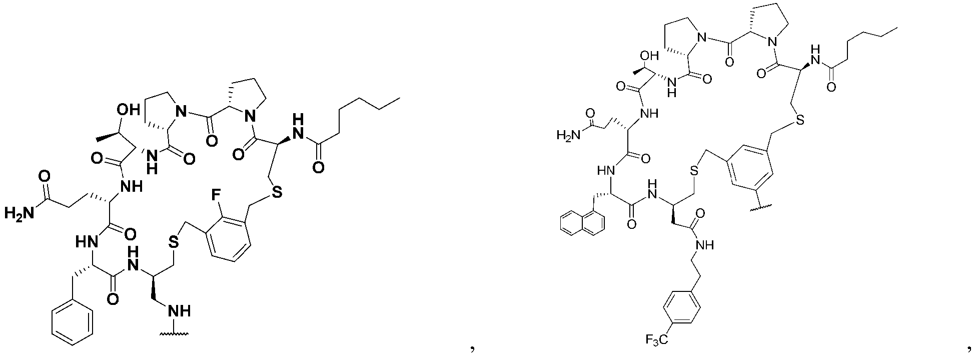

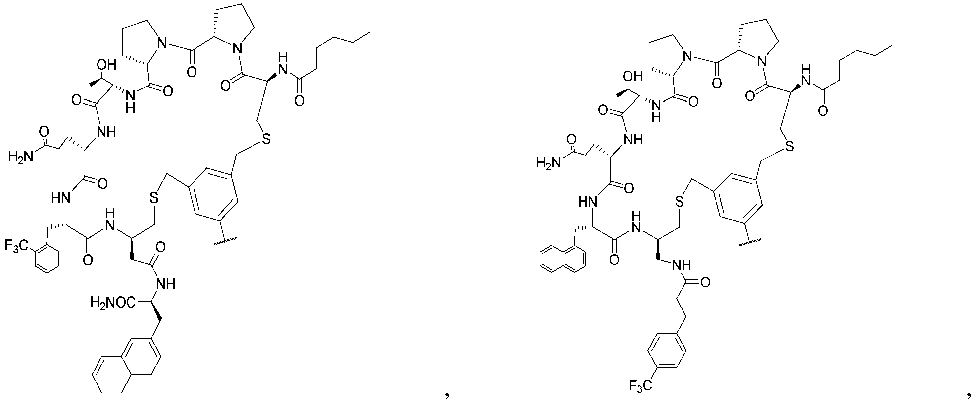

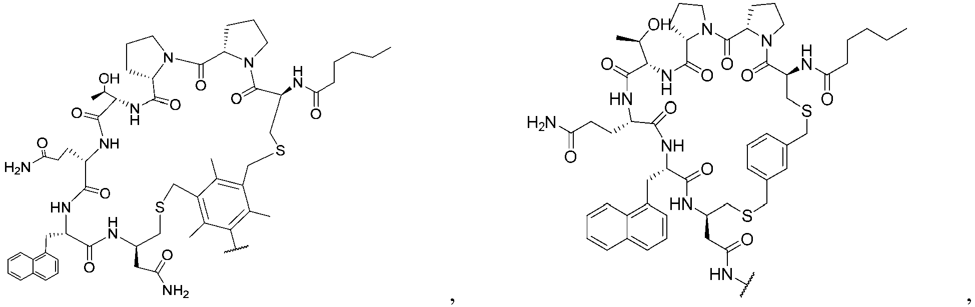

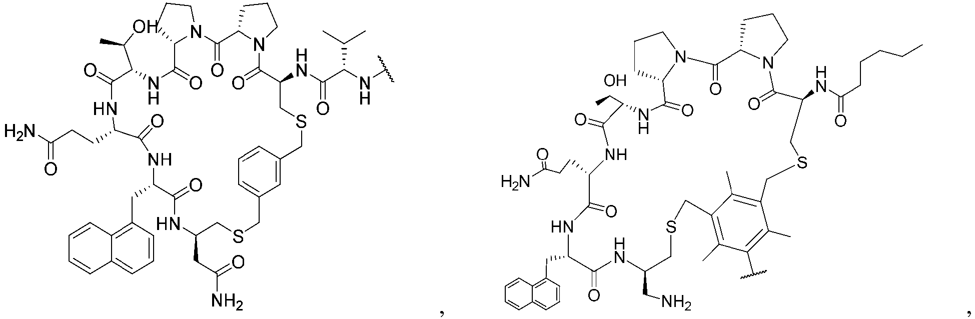

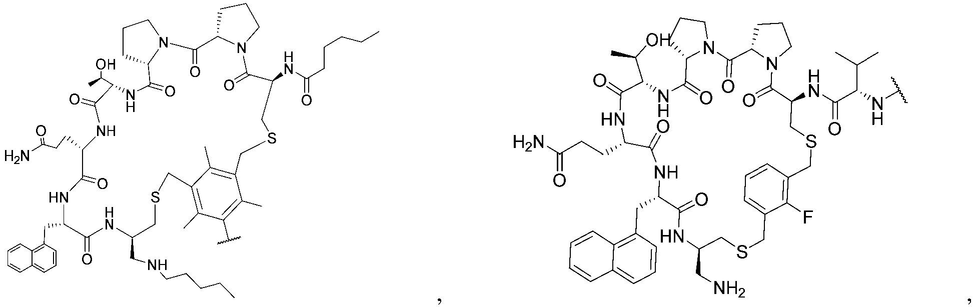

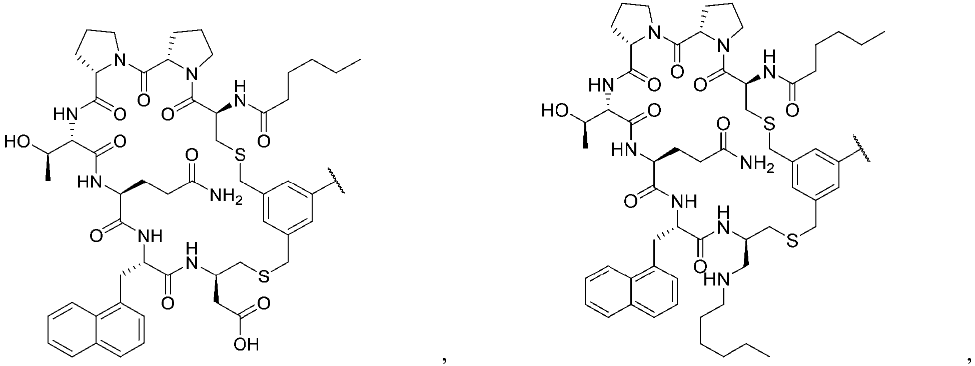

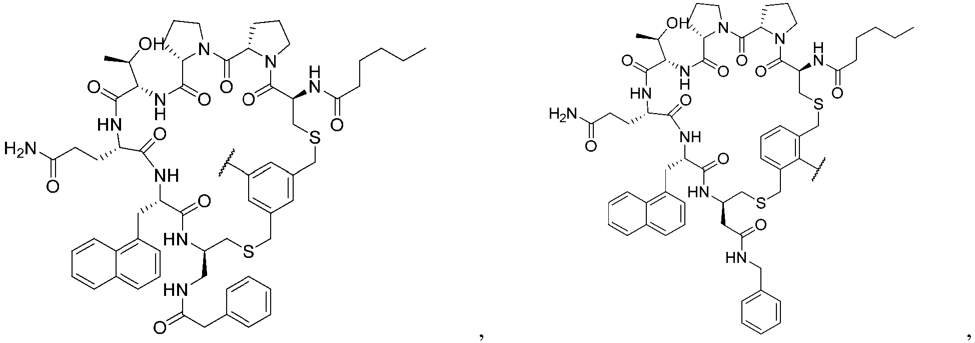

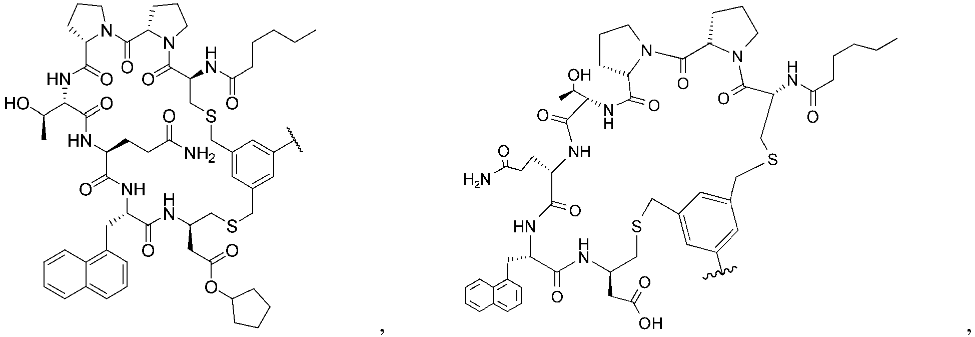

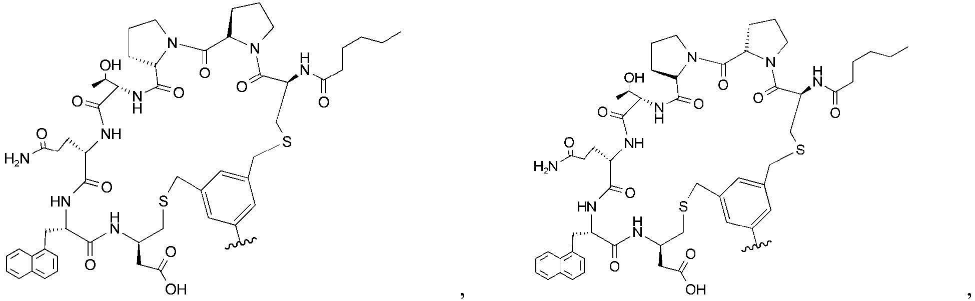

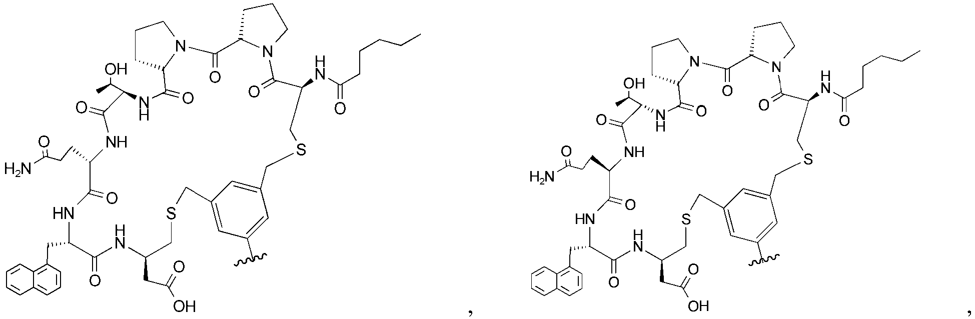

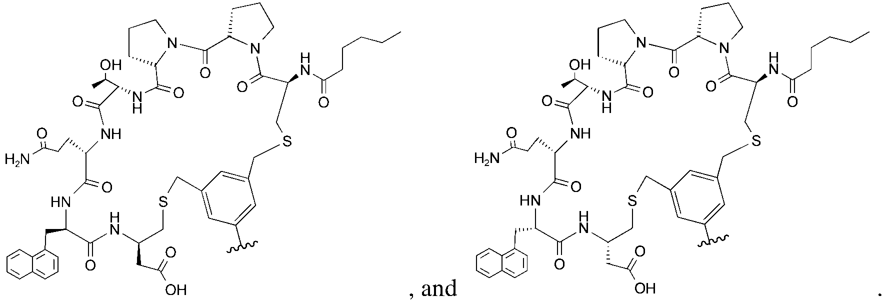

In one embodiment, L is selected from any of the following segments:

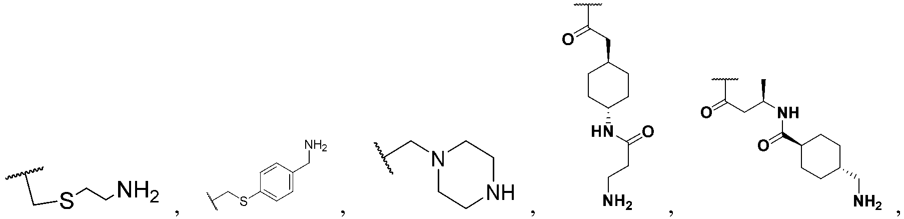

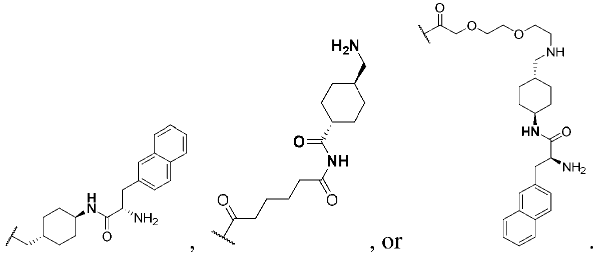

In one embodiment, the polypeptide compound represented by formula II is selected from any of the following compounds:

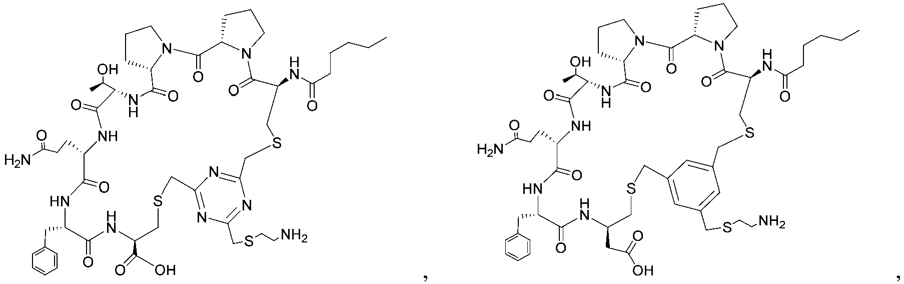

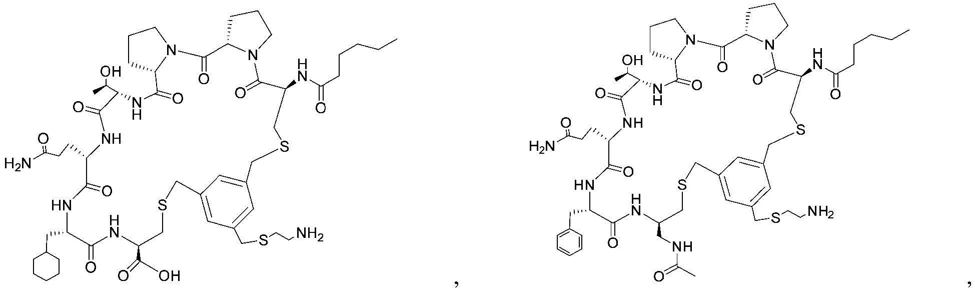

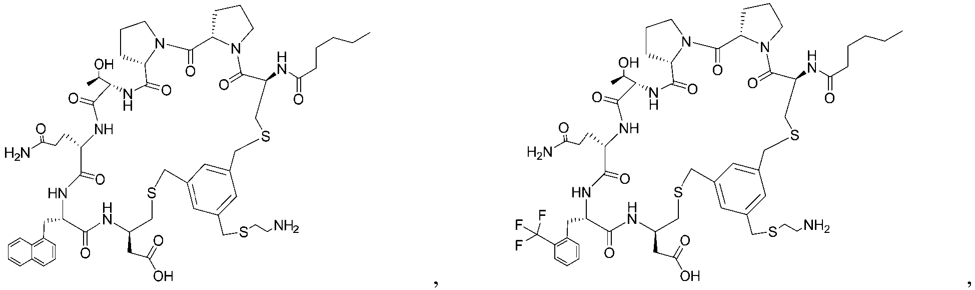

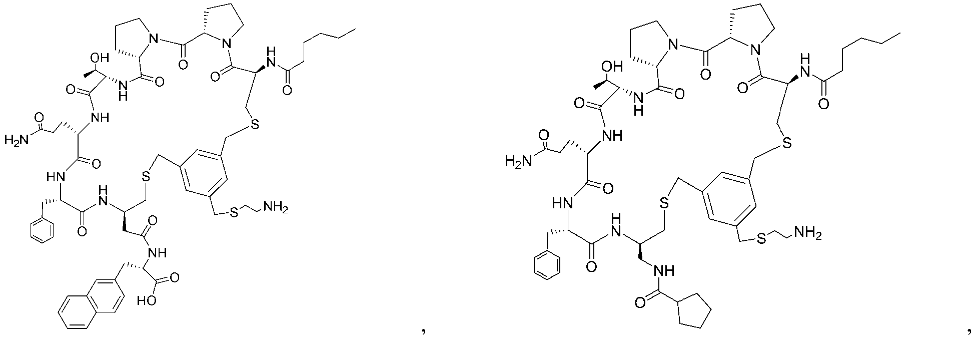

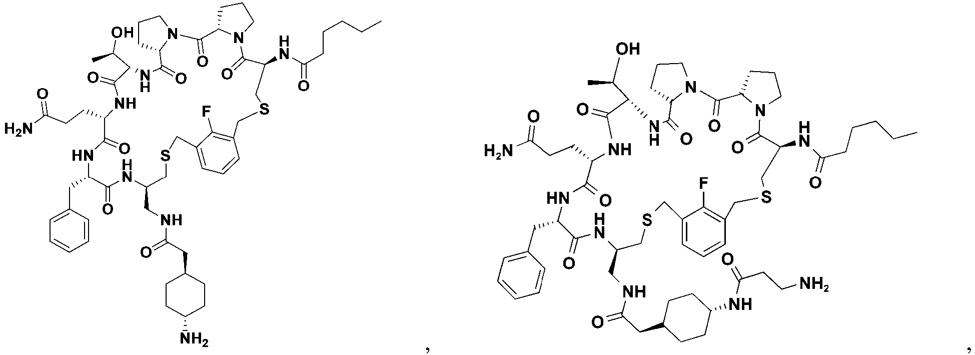

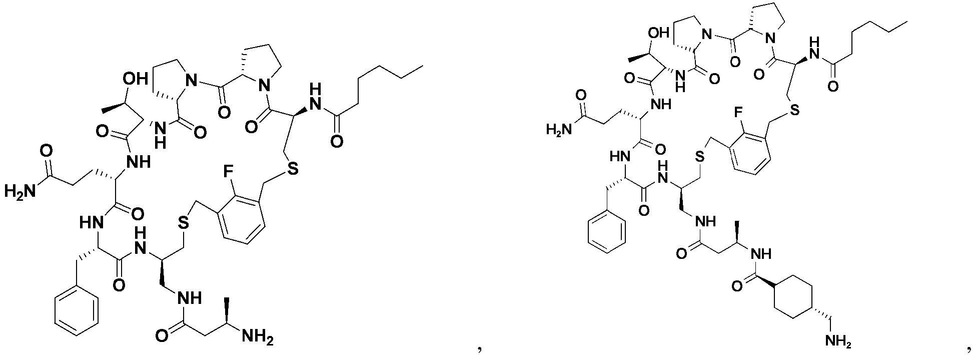

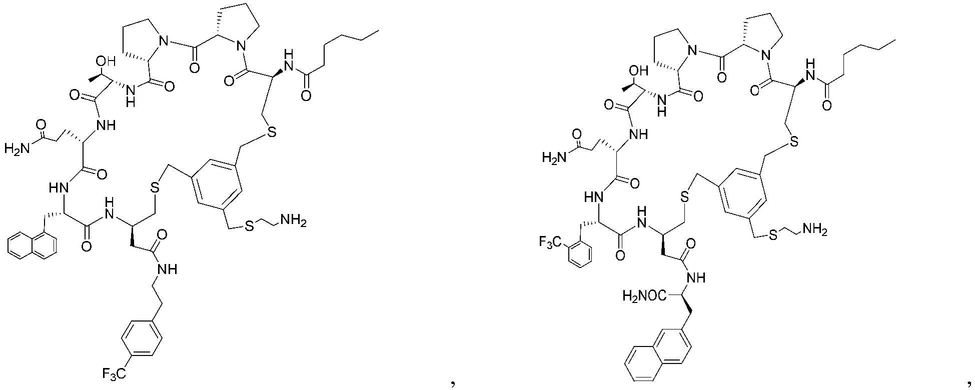

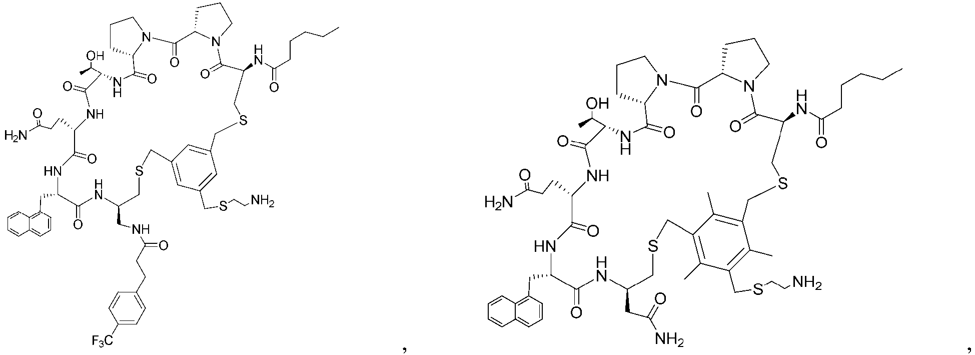

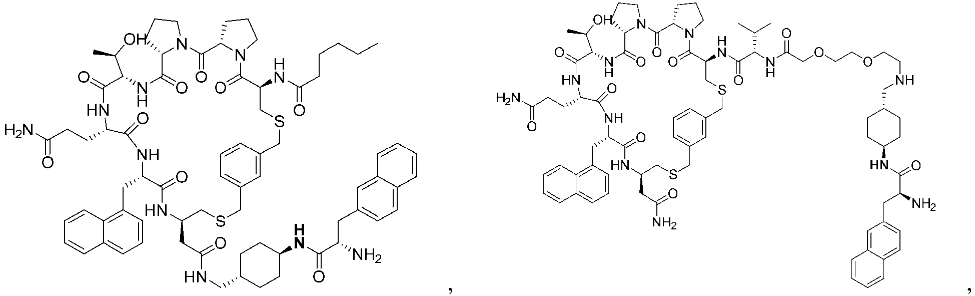

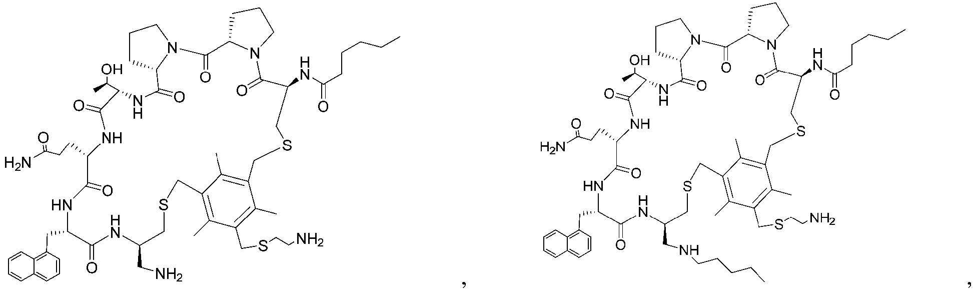

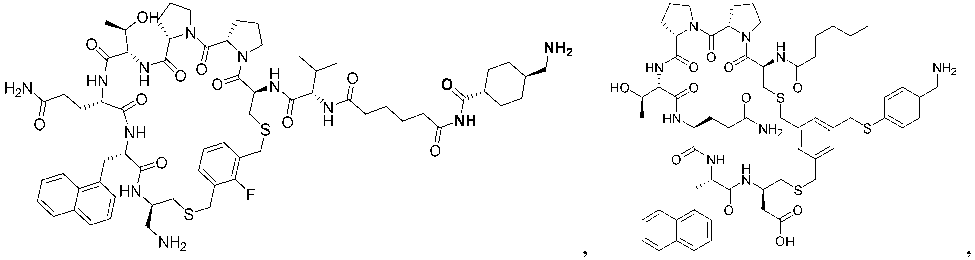

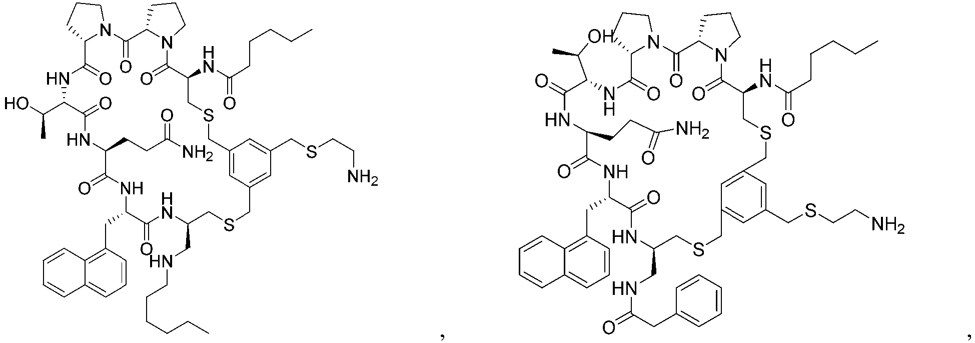

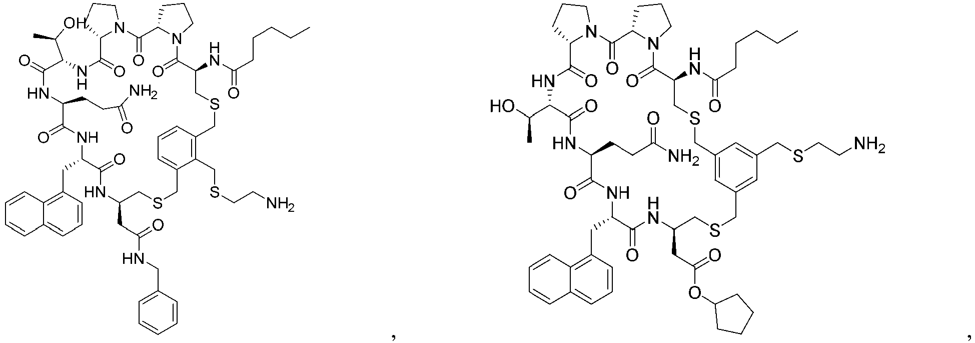

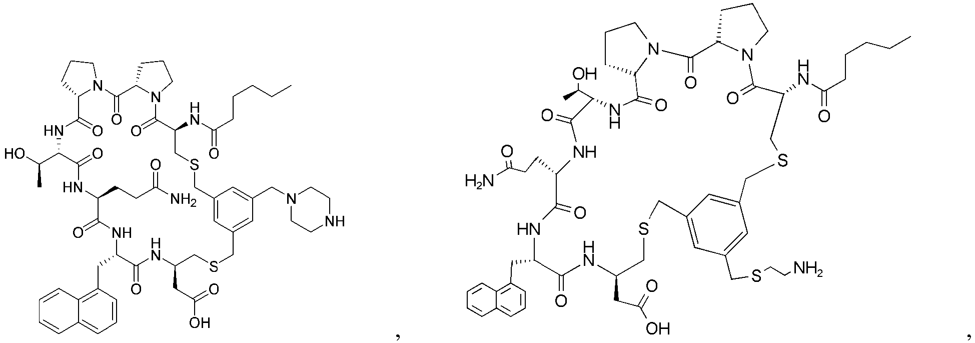

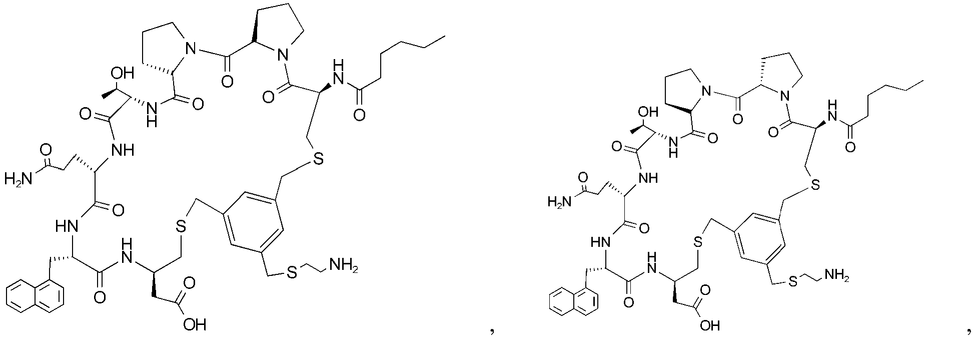

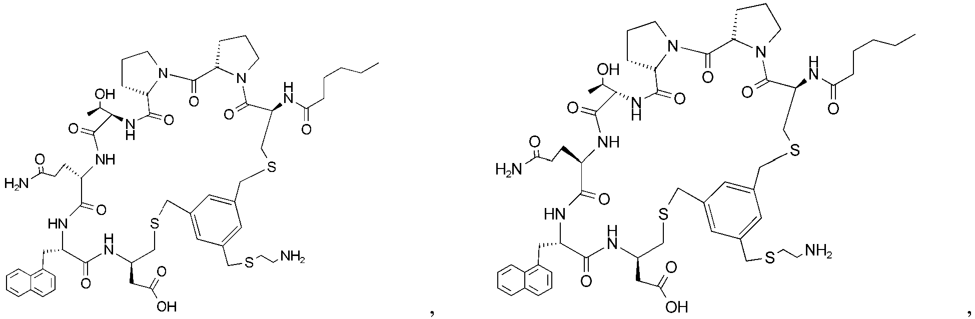

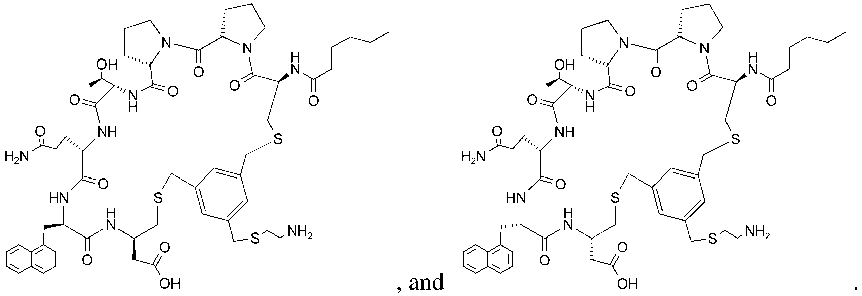

In a fifth aspect, the present disclosure further provides a polypeptide compound, a pharmaceutically acceptable salt thereof, a solvate thereof or a solvate of a pharmaceutically acceptable salt thereof, wherein the polypeptide compound is represented by formula III:

D-L'-R III

D is as defined in any one of the preceding embodiments;
L' is a group formed by the loss of a H atom by an amino group on L as defined in any one of the preceding embodiments; and
R is a group with the function of chelating metal ions.

In one embodiment, R is a group formed by the loss of H or hydroxyl by a chelating agent shown below, as an example, 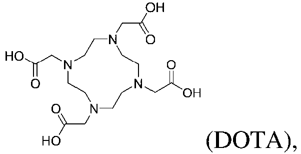 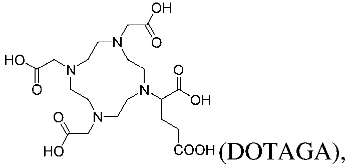 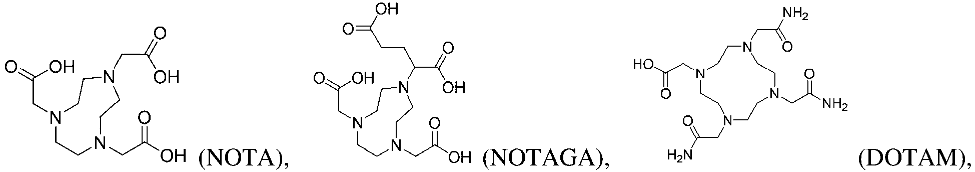 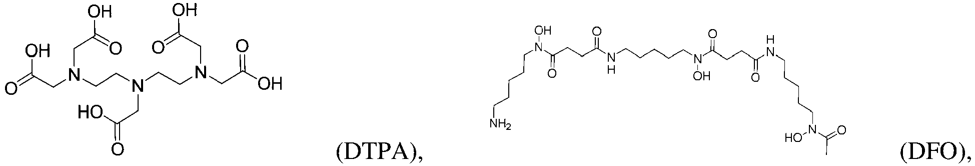 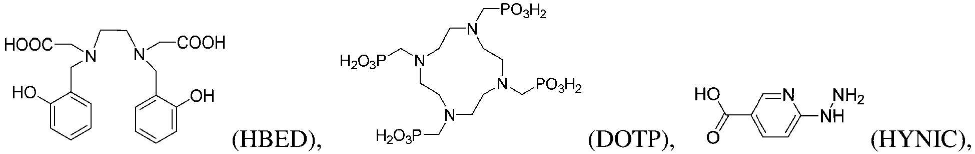 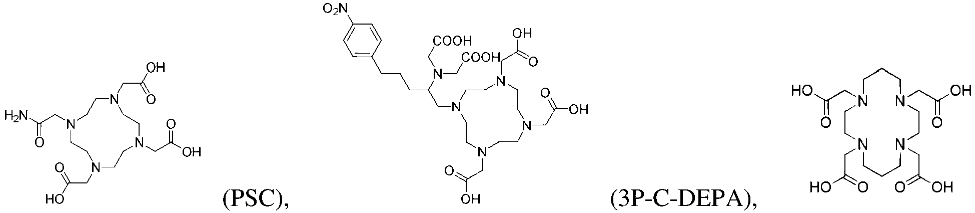 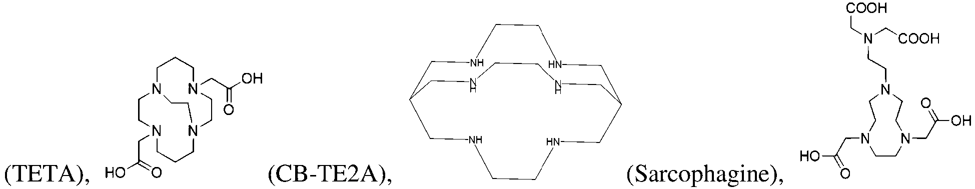 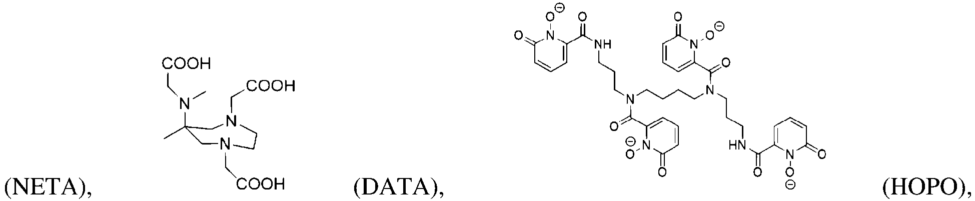 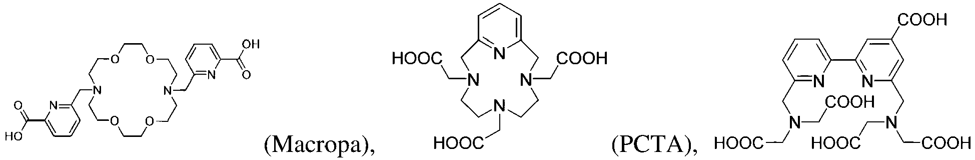 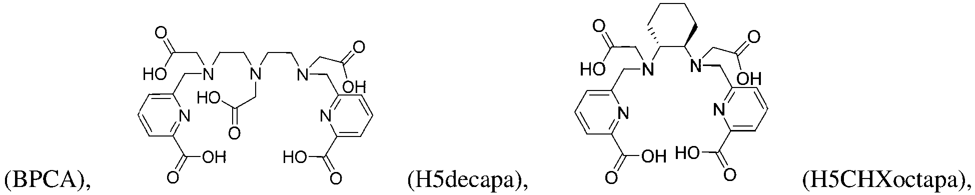 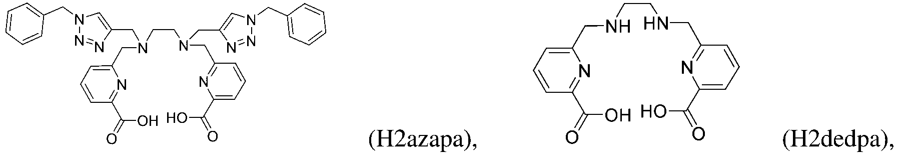 and

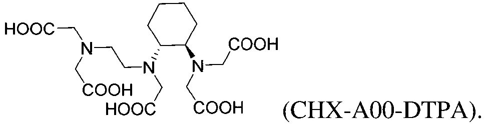

In one embodiment, R is 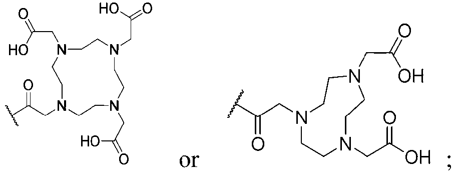 preferably

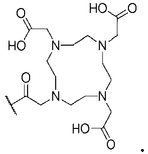

In one embodiment, the polypeptide compound represented by formula III is selected from any of the following compounds:

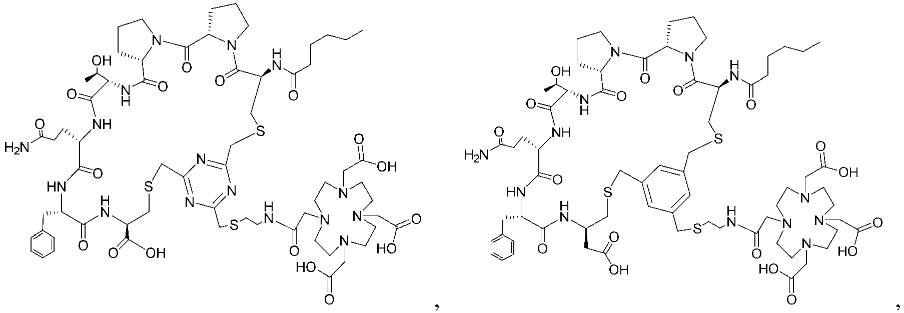

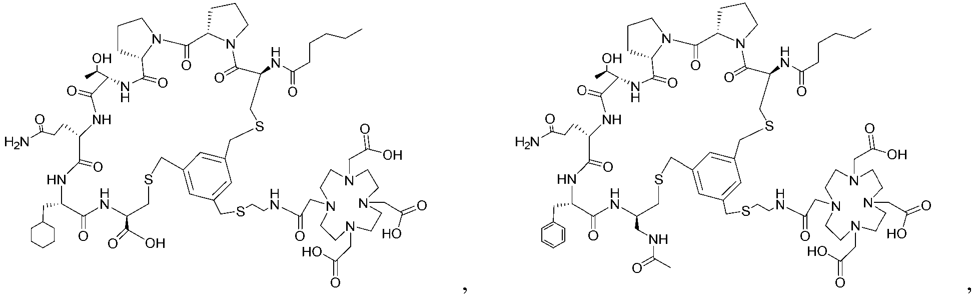

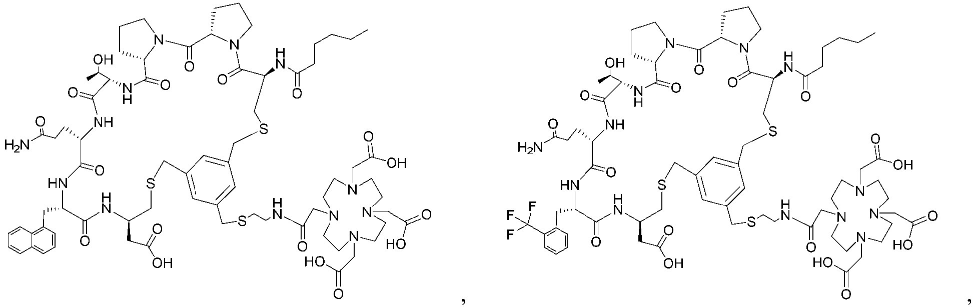

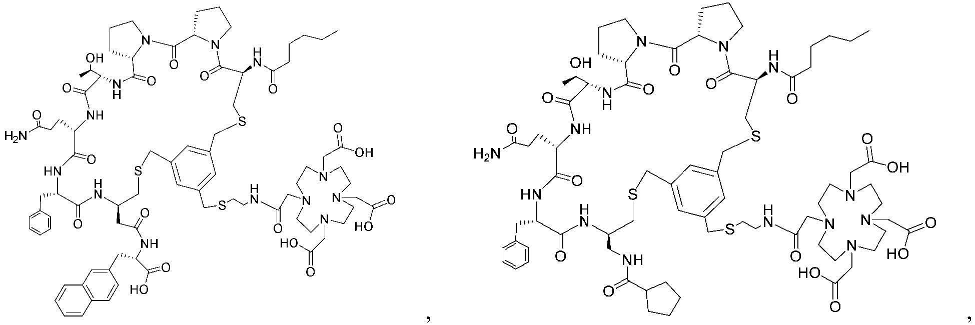

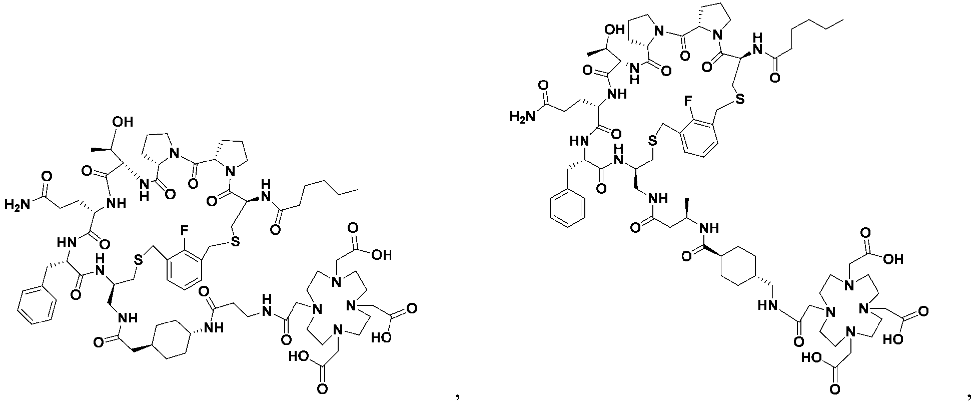

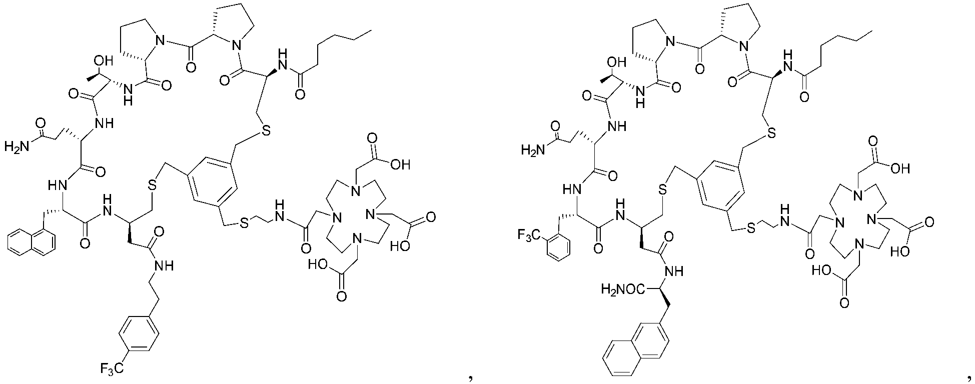

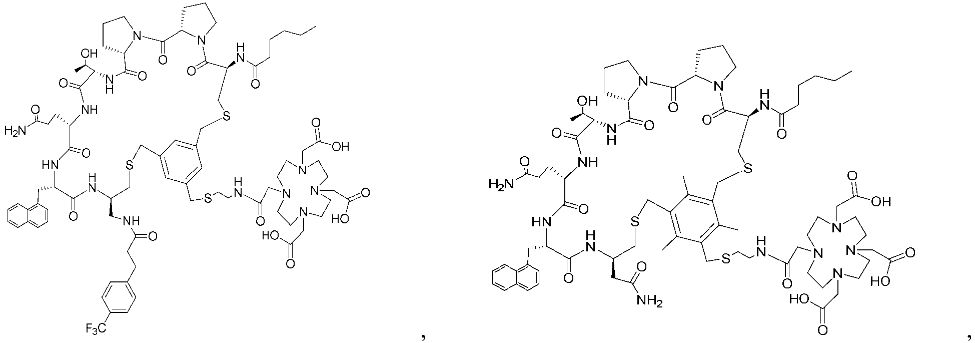

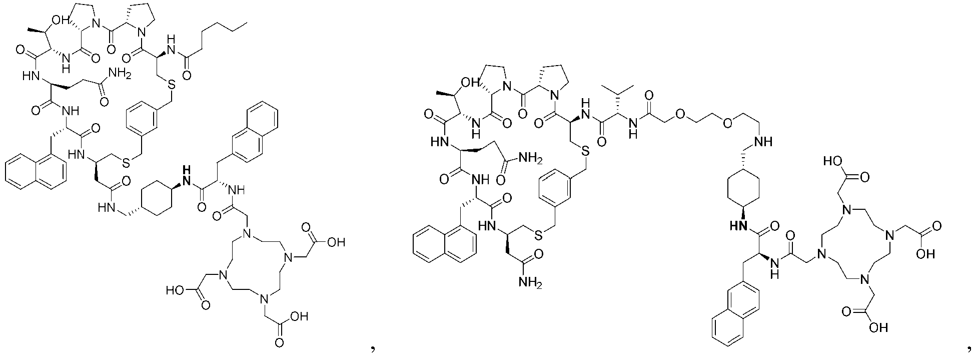

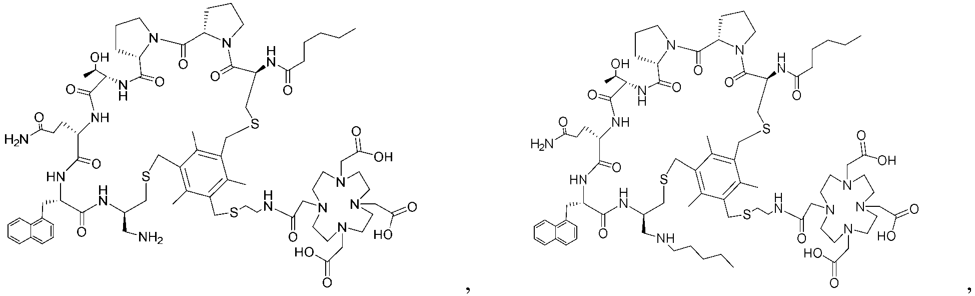

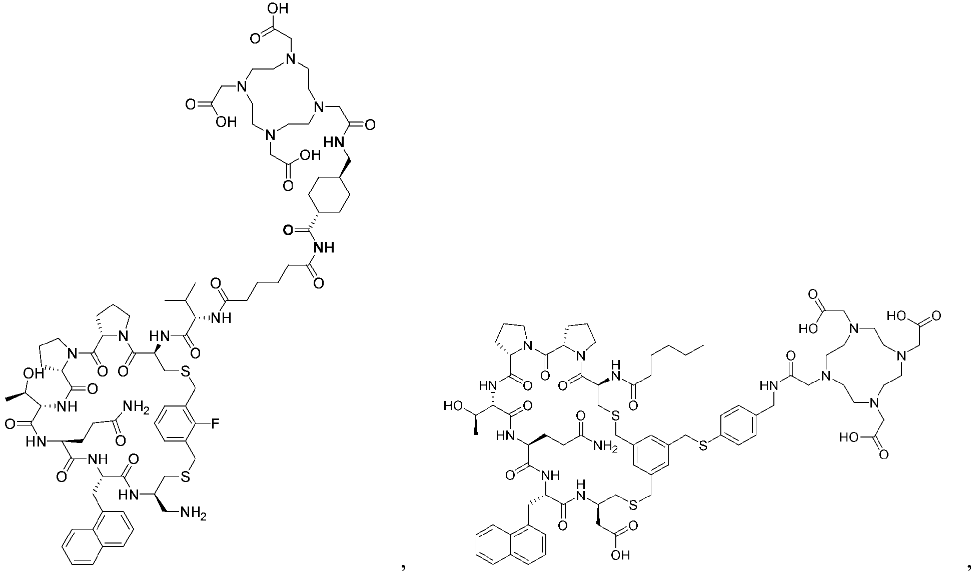

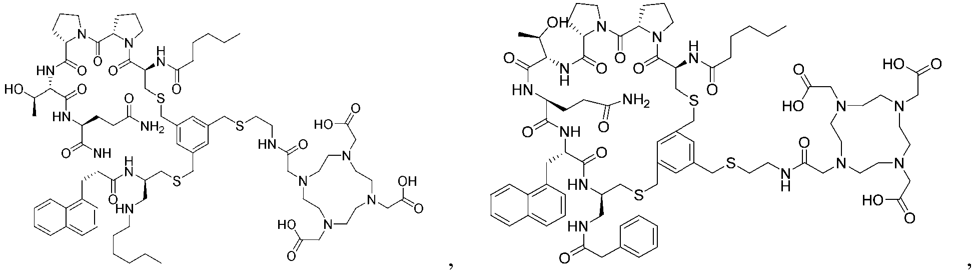

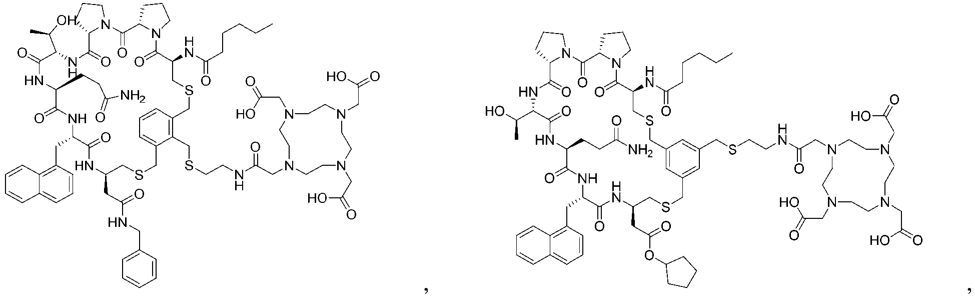

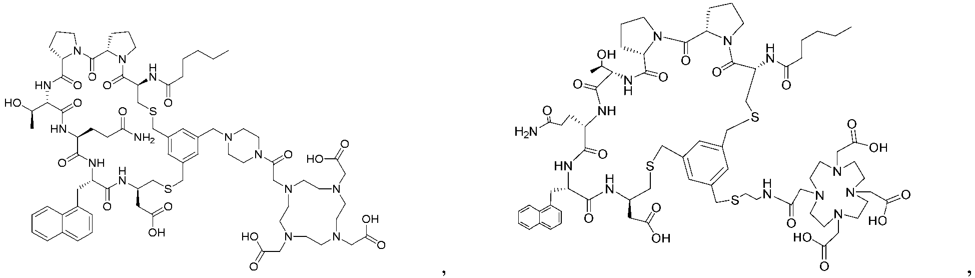

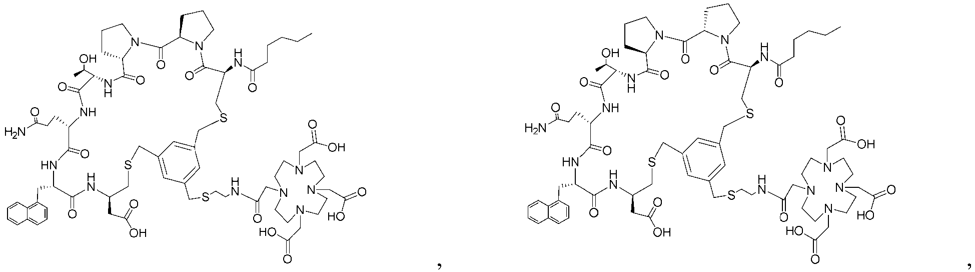

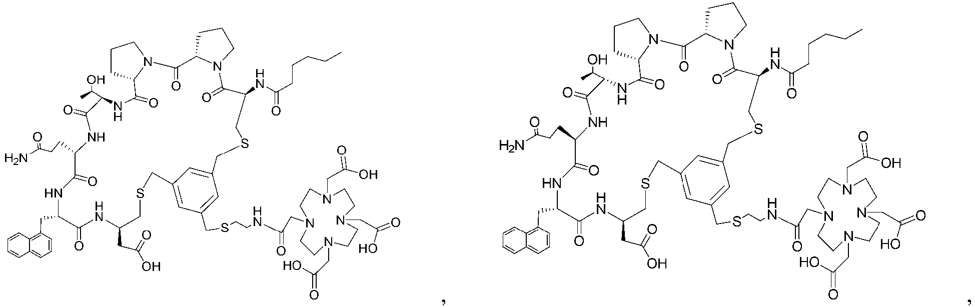

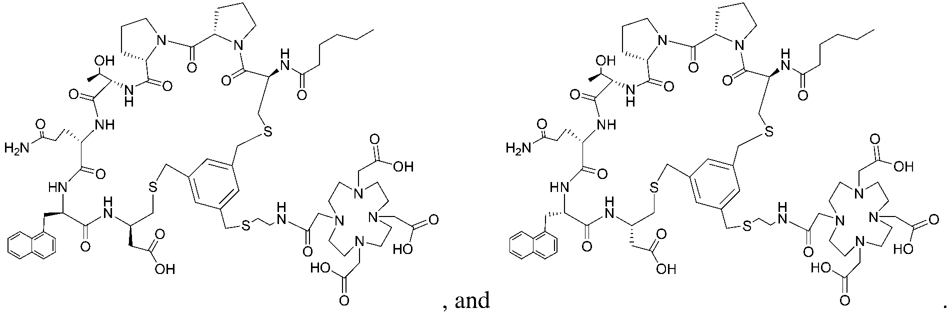

In a sixth aspect, the present disclosure further provides a polypeptide compound, a pharmaceutically acceptable salt thereof, a solvate thereof or a solvate of a pharmaceutically acceptable salt thereof, wherein the polypeptide compound is represented by formula V:

[D-L'-R]·M V

D is as defined in any one of the preceding embodiments;
L' is as defined in any one of the preceding embodiments;
R is as defined in any one of the preceding embodiments;
M is a diagnostically active radionuclide or a therapeutically active radionuclide; the diagnostically active radionuclide is selected from ¹⁸F, ^{99m}Tc, ⁸⁹Zr, ¹¹¹In, ⁶⁷Ga, ⁶⁸Ga, ⁴³Sc, ⁴⁴Sc, ⁶⁴Cu, ⁸⁶Y, ¹⁵²Tb, ¹⁵⁵Tb, ²⁰³Pb, ¹²³I, ¹²⁴I, ¹²⁵I, optionally ⁶⁴Cu, ⁶⁸Ga, ¹¹¹In, ¹⁸F, ^{99m}Tc, ²⁰³Pb, and ⁸⁹Zr; and the therapeutically active radionuclide is selected from ⁹⁰Y, ¹⁷⁷Lu, ²²⁵Ac, ²¹²Pb, ¹⁸⁸Re, ²²⁷Th, ¹³¹I, ²¹¹At, ¹⁶¹Tb, ¹⁴⁹Tb, ¹⁵³Sm, ⁶⁷Cu, ⁴⁷Sc, optionally ⁹⁰Y, ¹⁷⁷Lu, ²²⁵Ac, ²¹²Pb, and ¹⁶¹Tb.

In one embodiment, the diagnostically active radionuclide is ⁶⁸Ga, and the therapeutically active radionuclide is ¹⁷⁷Lu.

In one embodiment, the polypeptide compound represented by formula V is selected from any of the following compounds: 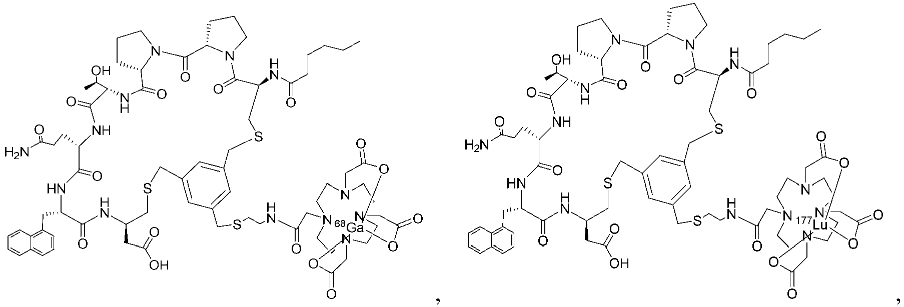 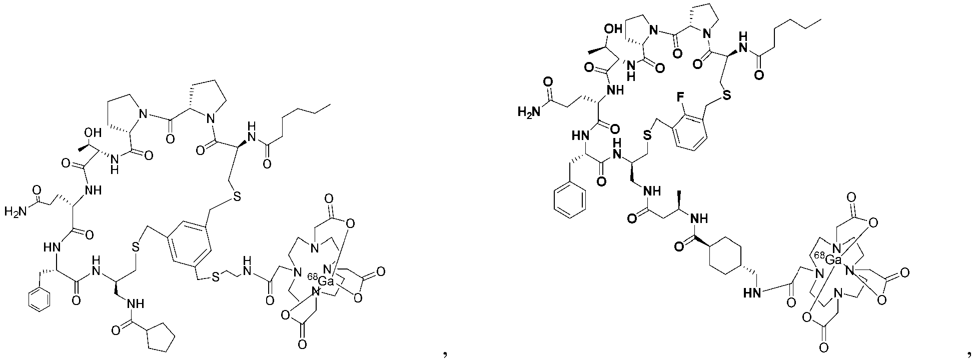 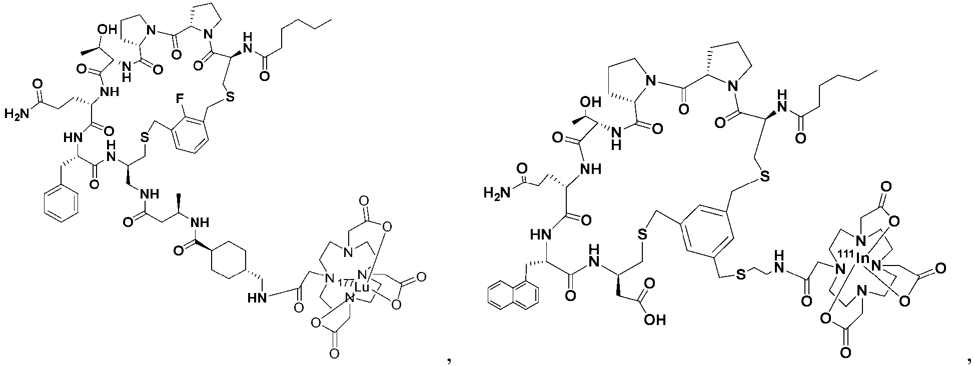 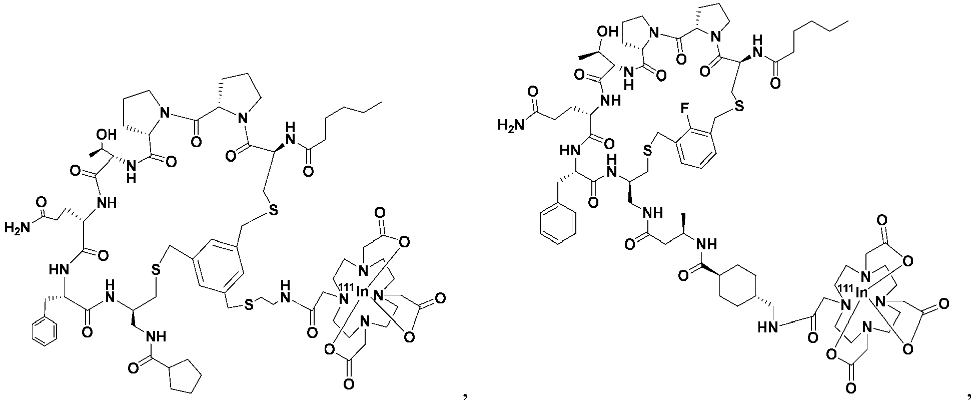 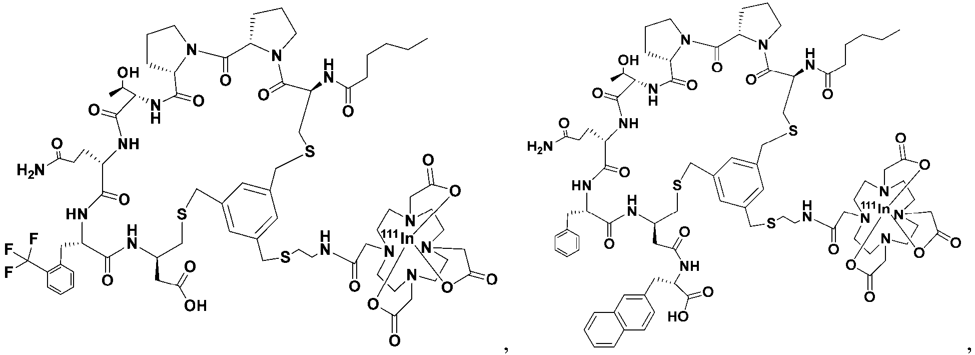 and

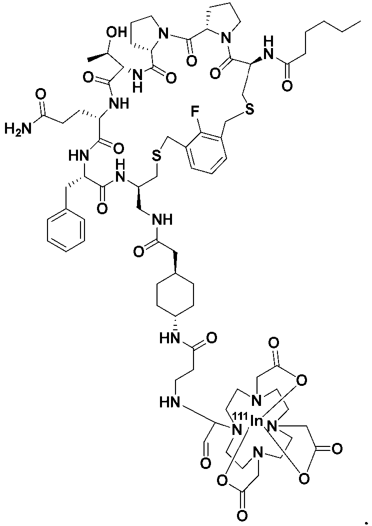

In a seventh aspect, the present disclosure further provides a polypeptide compound, a pharmaceutically acceptable salt thereof, a solvate thereof or a solvate of a pharmaceutically acceptable salt thereof, wherein the polypeptide compound is represented by formula VI:

[D-L'-R]·Q VI

D is as defined in any one of the fourth aspects;
L' is as defined in any one of the fourth aspects;
R is as defined in any one of the fourth aspects;
Q is a non-radionuclide; for example the non-radionuclide is selected from In, Ga, Y and Lu; for example In.

In one embodiment, the polypeptide compound is selected from any of the following compounds:

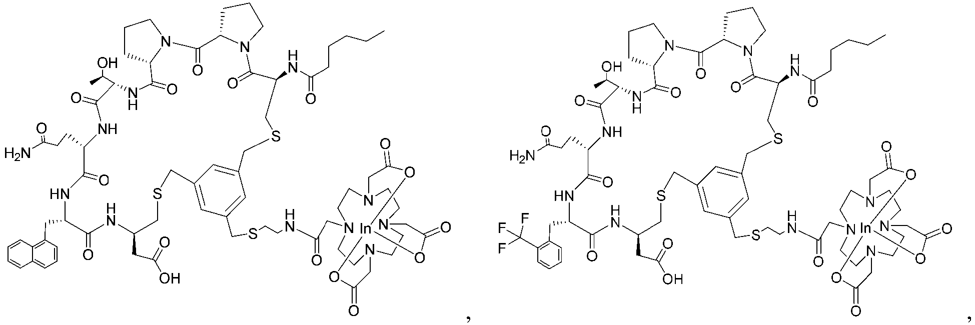

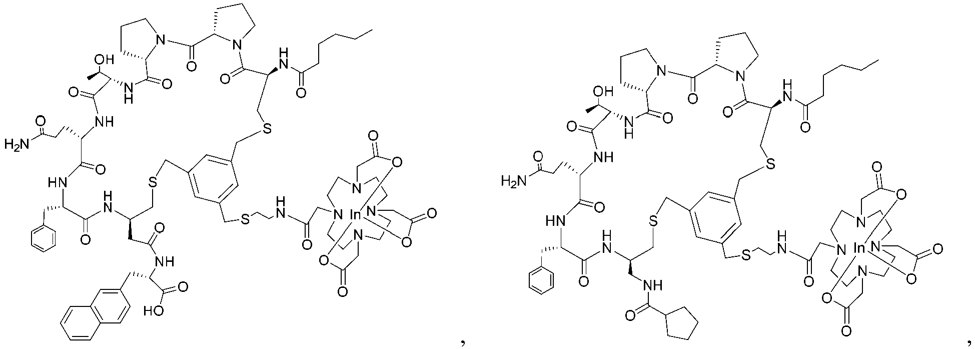

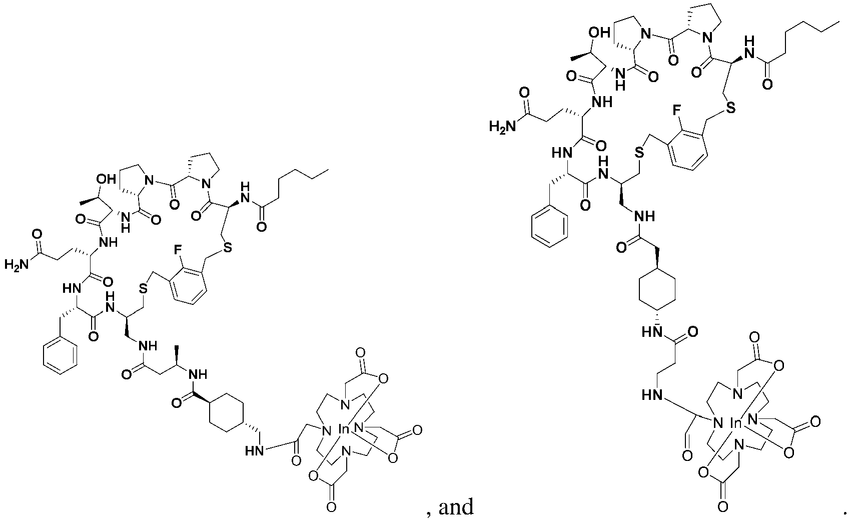

In an eighth aspect, the present disclosure further provides a pharmaceutical composition comprising a substance X, and a pharmaceutically acceptable adjuvant; wherein the substance X is a polypeptide compound of any one of the preceding embodiments, a pharmaceutically acceptable salt thereof, a solvate thereof or a solvate of a pharmaceutically acceptable salt thereof.

In a ninth aspect, the present disclosure further provides a kit comprising a substance X, and instructions for use; wherein the substance X is a polypeptide compound of any one of the preceding embodiments, a pharmaceutically acceptable salt thereof, a solvate thereof or a solvate of a pharmaceutically acceptable salt thereof.

In a tenth aspect, the present disclosure further provides the use of a substance X for the manufacture of a medicament, wherein the substance X is a polypeptide compound of any one of the preceding embodiments, a pharmaceutically acceptable salt thereof, a solvate thereof or a solvate of a pharmaceutically acceptable salt thereof; and the medicament is used for the diagnosis or treatment of a FAP protein-associated disease, which is preferably a tumor.

In one embodiment, the tumor is selected from bronchogenic carcinoma, hidradenocarcinoma, breast cancer, ovarian cancer, prostate cancer, melanoma, oral squamous carcinoma, brain tumor, esophageal cancer, gastric cancer, liver cancer, pancreatic cancer, colorectal cancer, lung cancer, small cell lung cancer, non-small cell lung cancer, renal cancer, skin cancer, glioblastoma, neurilemmoma, meningioma, neuroblastoma, mesothelioma, sarcoma, liposarcoma, chondroioma, osteoma, osteosarcoma, semaginoblastoma, testicular tumor, uterine cancer, head and neck cancer, multiple myeloma, malignant lymphoma, polycythemia vera, leukemia, thyroid cancer, ureter tumor, bladder tumor, gall bladder cancer, cholangiocarcinoma, choriocarcinoma and pediatric tumor.

In an eleventh aspect, the present disclosure further provides the use of a substance X for the manufacture of a FAP inhibitor, wherein the substance X is a polypeptide compound of any one of the preceding embodiments, a pharmaceutically acceptable salt thereof, a solvate thereof or a solvate of a pharmaceutically acceptable salt thereof.

The term "pharmaceutically acceptable salt" refers to a salt obtained by the reaction of a compound with a pharmaceutically acceptable (relatively non-toxic, safe, suitable for use by a patient) acid or base. When the compound contains a relatively acidic functional group, a base addition salt can be obtained by contacting the free form of the compound with a sufficient amount of a pharmaceutically acceptable base in a suitable inert solvent. When the compound contains a relatively basic functional group, an acid addition salt can be obtained by contacting the free form of the compound with a sufficient amount of a pharmaceutically acceptable acid in a suitable inert solvent.

The term "solvate" refers to a substance formed by crystallization of a compound with a solvent (including, but not limited to, water, methanol, ethanol, etc.). The solvates are classified into stoichiometric and non-stoichiometric solvates.

The term "solvate of a pharmaceutically acceptable salt" refers to a substance formed by combining a compound with a pharmaceutically acceptable (relatively non-toxic, safe and suitable for use by a patient) acid or base, and solvent (including, but not limited to, water, methanol, ethanol, etc.), wherein the pharmaceutically acceptable salt has the same meaning as the above term "pharmaceutically acceptable salt" above, and the solvent is stoichiometric or non-stoichiometric. The solvate of a pharmaceutically acceptable salt includes, but is not limited to, hydrochloride monohydrate.

The term "halogen" refers to fluorine, chlorine, bromine or iodine.

The term "alkyl" refers to a straight or branched alkyl consisting only of carbon atoms and hydrogen atoms and having a specified number of carbon atoms (for example, C₁-C₆), and the alkyl also includes straight or branched alkyl substituted with cycloalkyl. Alkyl includes, but is not limited to, methyl, ethyl, n-propyl, isopropyl, n-butyl, tert-butyl, isobutyl, sec-butyl, n-pentyl, n-hexyl, n-heptyl, n-octyl, etc.

The term "cycloalkyl" refers to a saturated cyclic group having a specified number of carbon atoms (for example, C₃-C₆) and a cyclic skeleton consisting only of carbon atoms, and the cycloalkyl also includes bridged cycloalkanes. Cycloalkyl includes, but is not limited to, cyclopropyl, cyclobutyl, cyclopentyl, cyclohexyl, etc.

The term "aryl" refers to a cyclic group consisting only of carbon atoms and having a specified number of carbon atoms (for example, C₆-C₁₀), which is a monocyclic or fused ring, and at least one ring is aromatic (in accordance with the Hückel's rule). Aryl is attached to other segments of the molecule by an aromatic ring or a non-aromatic ring. Aryl includes, but is not limited to, phenyl or naphthyl, etc.

The term "heteroaryl" refers to a cyclic group having a specified number of ring atoms (for example, 5-10 members), a specified number of heteroatoms (for example, 1, 2 or 3 heteroatoms) and a specified type of heteroatoms (one or more of N, O and S), which is monocyclic or polycyclic, and at least one ring is aromatic (in accordance with the Hückel's rule). Heteroaryl is attached to other segments of the molecule by an aromatic ring or a non-aromatic ring.

The term "heterocycloalkyl" refers to a saturated cyclic group having a specified number of ring atoms (for example, 5-12 members), a specified number of heteroatoms (for example, 1, 2 or 3 heteroatoms) and a specified type of heteroatoms (one or more of N, O and S).

The term "amino acid" refers to a compound with at least one amino group and at least one carboxyl group. Amino acids are broadly defined and can be natural or non-natural amino acids.

The term "pharmaceutically acceptable adjuvant" refers to the excipients and additives used in the manufacture of medicament and in the formulation of prescriptions, and refers to all substances contained in pharmaceutical preparations in addition to the active ingredients. See People's Republic of China Pharmacopoeia (2020 edition) or Handbook of Pharmaceutical Excipients (Raymond C Rowe, 2009) for details.

The term "therapeutically effective amount" refers to the dose of a compound and radiation administered to a patient that is sufficient to effectively treat a disease. The therapeutically effective amount will vary depending on the compound, the type of disease, the severity of the disease, the age of the patient, etc. However, it also can be adjusted by a person skilled in the art as appropriate.

The term "patient" refers to any animal that has been or will be treated, preferably a mammal, most preferably a human. Mammals include, but are not limited to, cattles, horses, sheep, pigs, cats, dogs, mice, rats, rabbits, guinea pigs, monkeys, humans, etc.

The term "treatment" refers to any of the following situations: (1) alleviating one or more biological manifestations of the disease; (2) interfering with one or more points in the disease-causing biological cascade; and (3) slowing the development of one or more biological manifestations of the disease.

The above-mentioned preferred conditions can be arbitrarily combined, without departing from the general knowledge in the art, to obtain the preferred examples of the present disclosure.

The reagents and raw materials used in the present disclosure are commercially available.

The positive and progressive effect of the present disclosure consists in that the polypeptide compound of the present disclosure has relatively good inhibitory activity against an FAP protein.

### BRIEF DESCRIPTION OF THE DRAWINGS

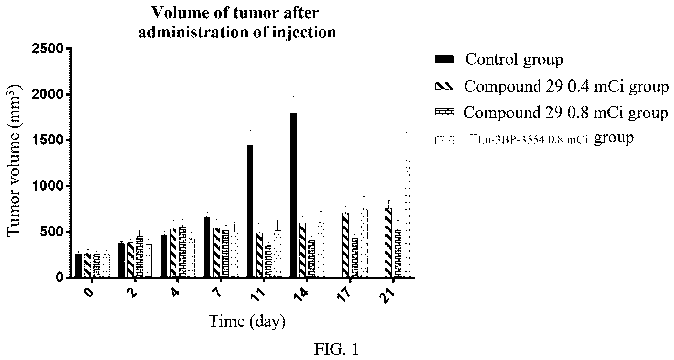

Figure 1 is a bar graph showing the changes in volume of tumor over time in tumor-bearing mice after administration of injection.

### DETAILED DESCRIPTION OF EMBODIMENTS

The present disclosure is further illustrated by way of examples below, but is not intended to limit the present invention within the scope of the described examples. The experimental methods in the following examples without specifying specific conditions were carried out according to conventional methods and conditions, or selected according to the product instructions.

### Example 1

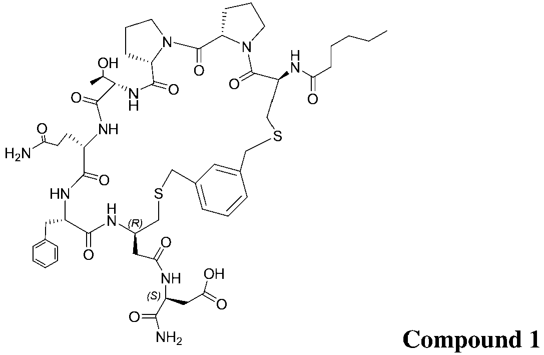

**Step 1:** 0.287 g of Fmoc-Linker MBHA Resin with a degree of substitution of 0.35 mmol/g was weighed and placed in a reactor, soaked with 10 mL of N,N-dimethylformamide (DMF) for 2 h and then drained; and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The resulting mixture was drained and successively washed with DMF, dichloromethane (DCM) and methanol (MeOH) to give 0.3 g of a crude resin. The crude resin was cut by 6 mL of solution E (trifluoroacetic acid (TFA) : 1,2-ethanedithiol (EDT) : H₂O : phenol : triisopropylsilane (TIS) = 90 : 2.5 : 2.5 : 2.5 : 2.5), filtered with suction, centrifuged, washed with methyl tert-butyl ether and purified by HPLC (separated by preparative chromatography, column: Huapu C18 10 µm 100A 20*250 mm, mobile phase: A: 0.1% TFA in water; B: 0.1% TFA in acetonitrile (ACN), linear gradient: 26-56% over 60 min, wavelength: 220 nm) to give an intermediate Hex-Cys-Pro-Pro-Thr-Gln-Phe-beta-HomoCys-Asp-NH₂.

**Step 2:** 26 mg (1.0 eq.) of the final product obtained in step 1 was dissolved in 20 mL of 50 mM aqueous ammonium bicarbonate solution and acetonitrile (with a volume ratio of 1 : 1), and a solution of 6.7 mg DBMB (1.0 eq.) in 0.5 mL of acetonitrile was added, the mixture was reacted at room temperature for 1 h, then 1 mL of acetic acid was added, and the mixture was concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 33-63% over 60 min, wavelength: 220 nm) to give a final product Hex-Cys-Pro-Pro-Thr-Gln-Phe-beta-HomoCys-Asp-NH₂ (Cys bridge DBMB) trifluoroacetate.

HPLC purity was 98.2%, MS (m/z): 1123.8 [M+H]⁺.

### Example 2

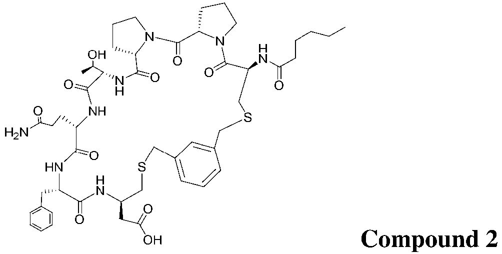

**Step 1:** 0.185 g of Fmoc-Beta-HomoCys(Trt)-CTC Resin with a degree of substitution of 0.54 mmol/g was weighed and placed in a reactor, soaked with N,N-dimethylformamide (DMF) for 2 h and then drained, and deprotection was performed with 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with DMF. According to the polypeptide sequence, the respective amino acids were added successively, and all amino acids were linked via solid phase synthesis. The crude resin was transferred to a cutting tube, cut with solution E (TFA : EDT : H₂O : phenol : TIS = 90 : 2.5 : 2.5 : 2.5 : 2.5) at 25°C and treated with methyl tert-butyl ether, and the crude product was dried and purified by HPLC (separated by preparative chromatography, column: Huapu C18 10 µm 100A 20*250 mm, mobile phase: A: 0.1% TFA in water; B: 0.1% TFA in ACN, linear gradient: 37-67% over 60 min, wavelength: 220 nm) to give an intermediate Hex-Cys-Pro-Pro-Thr-Gln-Phe-beta-HomoCys-OH.

**Step 2:** 10 mg (1.0 eq.) of the final product obtained in step 1 was dissolved in 10 mL of 50 mM aqueous ammonium bicarbonate solution and acetonitrile (with a volume ratio of 1 : 1), and a solution of 2.9 mg DBMB (1.0 eq.) in 0.5 mL of acetonitrile was added, the mixture was reacted at room temperature for 1 h, then 1 mL of acetic acid was added, and the mixture was concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 37-67% over 60 min, wavelength: 220 nm) to give a final product Hex-Cys-Pro-Pro-Thr-Gln-Phe-beta-HomoCys-OH (Cys bridge DBMB) trifluoroacetate.

HPLC purity was 99.3%, MS (m/z): 1009.7 [M+H]⁺.

### Example 3

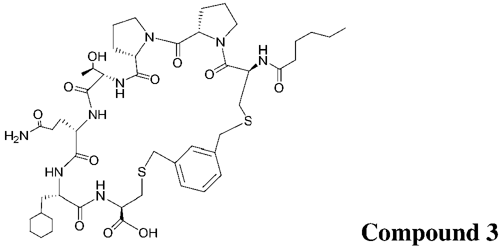

**Step 1:** 0.13 g of Fmoc-Cys(Trt)-CTC Resin with a degree of substitution of 0.767 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, treated with solution E (TFA : EDT : H₂O : phenol : TIS = 90 : 2.5 : 2.5 : 2.5 : 2.5) and methyl tert-butyl ether, and centrifuged, and the crude product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 32-62% over 60 min, wavelength: 220 nm) to give an intermediate Hex-Cys-Pro-Pro-Thr-Gln-Cha-Cys-OH.

**Step 2:** 57 mg (1.0 eq.) of the final product obtained in step 1 was dissolved in 60 mL of 50 mM aqueous ammonium bicarbonate solution and acetonitrile (with a volume ratio of 1 : 1), and a solution of 19 mg DBMB (1.0 eq.) in 0.5 mL of acetonitrile was added, the mixture was reacted at room temperature for 1 h, then 1 mL of acetic acid was added, and the mixture was concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 40-70% over 60 min, wavelength: 220 nm) to give a final product Hex-Cys-Pro-Pro-Thr-Gln-Cha-Cys-OH (Cys bridge DBMB) trifluoroacetate.

HPLC purity was 99.1%, MS (m/z): 1001.7 [M+H]⁺.

### Example 4

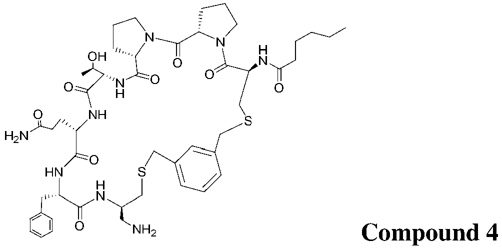

**Step 1:** 0.64 g of Fmoc-Phe-CTC Resin with a degree of substitution of 0.94 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, and the obtained crude resin was treated with 10 mL of 1% TFA/DCM. The organic phase was respectively washed with water and saturated brine, and concentrated in vacuum to give the fully protected intermediate Hex-Cys(Trt)-Pro-Pro-Thr(tBu)-Gln(Trt)-Phe-OH. 239 mg of the above intermediate and 32 mg of HOOBt (1.1 eq.) were dissolved in 10 mL of DMF and cooled to 0°C, then 38 mg of EDC hydrochloride (1.1 eq.) and 101 mg of H-Cys(Trt)-CH₂NHBoc (1.0 eq.) were successively added, and the mixture was reacted at room temperature for 4 h. DMF was removed via concentration, 10 mL of 5% aqueous phosphoric acid solution and 20 mL of ethyl acetate were added, and the organic phase was washed with water and saturated brine and concentrated in vacuum to give the intermediate Hex-Cys(Trt)-Pro-Pro-Thr(tBu)-Gln(Trt)-Phe-Cys(Trt)-CH₂NH₂. The intermediate was treated with mL of solution E (TFA : EDT : H₂O : phenol : TIS = 90 : 2.5 : 2.5 : 2.5 : 2.5) and methyl tert-butyl ether, and the crude product was dried and purified by HPLC (separated by preparative chromatography, column: Huapu C18 10 µm 100A 20*250 mm, mobile phase: A: 0.1% TFA in water; B: 0.1% TFA in ACN, linear gradient: 25-55% over 60 min, wavelength: 220 nm) to give an intermediate Hex-Cys-Pro-Pro-Thr-Gln-Phe-Cys-CH₂NH₂.

**Step 2:** 38 mg (1.0 eq.) of the final product obtained in step 1 was dissolved in 40 mL of 50 mM aqueous ammonium bicarbonate solution and acetonitrile (with a volume ratio of 1 : 1), and a solution of 15 mg DBMB (1.0 eq.) in 0.5 mL of acetonitrile was added, the mixture was reacted at room temperature for 1 h, then 1 mL of acetic acid was added, and the mixture was concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 32-62% over 60 min, wavelength: 220 nm) to give a final product Hex-Cys-Pro-Pro-Thr-Gln-Phe-Cys-CH₂NH₂ (Cys bridge DBMB) trifluoroacetate.

HPLC purity was 95.2%, MS (m/z): 980.9 [M+H]⁺.

### Example 5

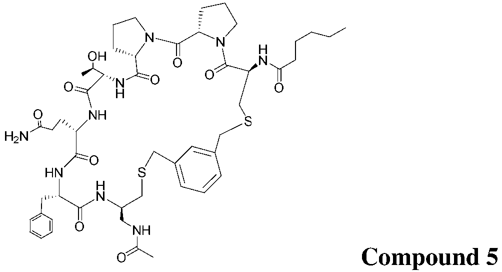

**Step 1:** 0.64 g of Fmoc-Phe-CTC Resin with a degree of substitution of 0.94 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, and the obtained crude resin was treated with 10 mL of 1% TFA/DCM. The organic phase was respectively washed with water and saturated brine, and concentrated in vacuum to give the fully protected intermediate Hex-Cys(Trt)-Pro-Pro-Thr(tBu)-Gln(Trt)-Phe-OH. 277 mg of the intermediate from the previous step and 37 mg of HOOBt (1.1 eq.) were dissolved in 10 mL of DMF and cooled to 0°C, then 44 mg of EDC hydrochloride (1.1 eq.) and H-Cys(Trt)-CH₂NHAc (1.0 eq.) were successively added, and the mixture was reacted at room temperature. Upon completion of the reaction, DMF was removed via concentration, 10 mL of 5% aqueous phosphoric acid solution and 20 mL of ethyl acetate were added, and the mixture was stirred and then separated. The organic phase was successively washed with H₂O and saturated brine and concentrated in vacuum to give the intermediate Hex-Cys(Trt)-Pro-Pro-Thr(tBu)-Gln(Trt)-Phe-Cys(Trt)-CH₂NHAc. The intermediate was successively treated with solution E (TFA : EDT : H₂O : phenol : TIS = 90 : 2.5 : 2.5 : 2.5 : 2.5) and methyl tert-butyl ether, and the crude product was dried in vacuum and purified by HPLC (separated by preparative chromatography, column: Huapu C18 10 µm 100A 20*250 mm, mobile phase: A: 0.1% TFA in water; B: 0.1% TFA in ACN, linear gradient: 30-60% over 60 min, wavelength: 220 nm) to give an intermediate Hex-Cys-Pro-Pro-Thr-Gln-Phe-Cys-CH₂NHAc.

**Step 2:** 50 mg (1.0 eq.) of the final product obtained in step 1 was dissolved in 50 mL of 50 mM aqueous ammonium bicarbonate solution and acetonitrile (with a volume ratio of 1 : 1), and a solution of 20 mg DBMB (1.0 eq.) in 0.5 mL of acetonitrile was added, the mixture was reacted at room temperature for 1 h, then 1 mL of acetic acid was added, and the mixture was concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 35-65% over 60 min, wavelength: 220 nm) to give a final product Hex-Cys-Pro-Pro-Thr-Gln-Phe-Cys-CH₂NHAc (Cys bridge DBMB) trifluoroacetate.

HPLC purity was 96.9%, MS (m/z): 1022.9 [M+H]⁺.

### Example 6

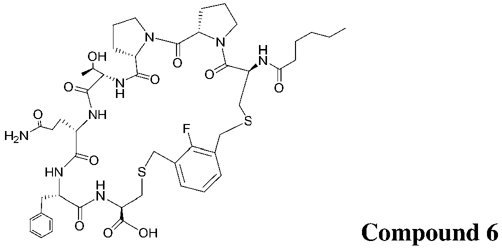

**Step 1:** 0.31 g of Fmoc-Cys(Trt)-CTC Resin with a degree of substitution of 0.656 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, treated with solution E (TFA : EDT : H₂O : phenol : TIS = 90 : 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 28-58% over 60 min, wavelength: 220 nm) to give an intermediate Hex-Cys-Pro-Pro-Thr-Gln-Phe-Cys-OH.

**Step 2:** 56 mg (1.0 eq.) of the final product obtained in step 1 was dissolved in 60 mL of 50 mM aqueous ammonium bicarbonate solution and acetonitrile (with a volume ratio of 1 : 1), and a solution of 16 mg 2,6-bis(bromomethyl)fluorobenzene (1.0 eq.) in 0.5 mL of acetonitrile was added, the mixture was reacted at room temperature for 1 h, then 1 mL of acetic acid was added, and the mixture was concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 35-65% over 60 min, wavelength: 220 nm) to give a final product Hex-Cys-Pro-Pro-Thr-Gln-Phe-Cys-OH (Cys bridge 2,6-bis(bromomethyl)fluorobenzene) trifluoroacetate.

HPLC purity was 99.6%, MS (m/z): 1013.9 [M+H]⁺.

### Example 7

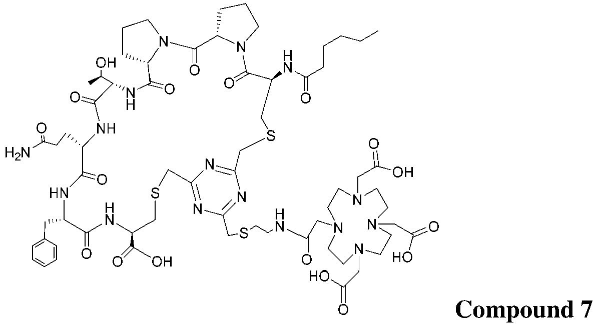

**Step 1:** 0.31 g of Fmoc-Cys(Trt)-CTC Resin with a degree of substitution of 0.656 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, treated with solution E (TFA : EDT : H₂O : phenol : TIS = 90 : 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 28-58% over 60 min, wavelength: 220 nm) to give an intermediate Hex-Cys-Pro-Pro-Thr-Gln-Phe-Cys-OH.

**Step 2:** 112 mg (1.0 eq.) of the final product obtained in step 1 was dissolved in 100 mL of 50 mM aqueous ammonium bicarbonate solution and acetonitrile (with a volume ratio of 1 : 1), and a solution of 68 mg 2,4,6-tribromomethyl-1,3,5-triazine (1.5 eq.) in 0.5 mL of acetonitrile was added, the mixture was reacted at room temperature for 1 h, then 97 mg of cysteamine (10 eq.) was added and stirred for another 2 h, then 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 24-54% over 60 min, wavelength: 220 nm) to give an intermediate 2 Hex-Cys-Pro-Pro-Thr-Gln-Phe-Cys-OH (Cys bridge 2,4,6-tribromomethyl-1,3,5-triazine (H-AET)). 45 mg of the intermediate 2 (1.0 eq.) and 31 mg of DOTA-NHS (1.5 eq.) were dissolved in 5 mL of DMF, then 0.045 mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 24-54% over 60 min, wavelength: 220 nm) to give a final product Hex-Cys-Pro-Pro-Thr-Gln-Phe-Cys-OH (Cys bridge 2,4,6-tribromomethyl-1,3,5-triazine (DOTA-AET)) trifluoroacetate.

HPLC purity was 98.5%, MS (m/z): 1473.7 [M+H]⁺.

### Example 8

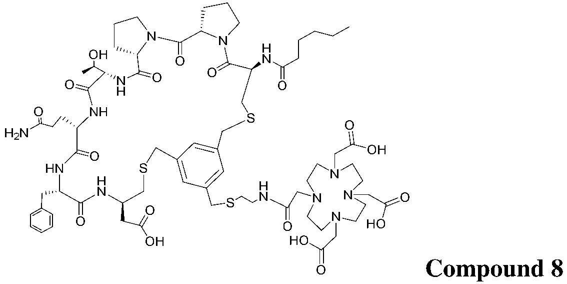

**Step 1:** 0.38 g of Fmoc-Beta-HomoCys(Trt)-CTC Resin with a degree of substitution of 0.52 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, treated with solution E (TFA : EDT : H₂O : phenol : TIS = 90 : 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 37-67% over 60 min, wavelength: 220 nm) to give an intermediate Hex-Cys-Pro-Pro-Thr-Gln-Phe-beta-HomoCys-OH.

**Step 2:** 75 mg (1.0 eq.) of the intermediate was dissolved in 100 mL of 50 mM aqueous ammonium bicarbonate solution and acetonitrile (with a volume ratio of 1 : 1), and a solution of 44 mg TBMB (1.5 eq.) in 0.5 mL of acetonitrile was added, the mixture was reacted at room temperature for 1 h, then 64 mg of cysteamine (10 eq.) was added and stirred for another 2 h, 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 27-57% over 60 min, wavelength: 220 nm) to give an intermediate 2 Hex-Cys-Pro-Pro-Thr-Gln-Phe-beta-HomoCys (Cys bridge TBMB (H-AET)). 57 mg of the intermediate 2 (1.0 eq.) and 39 mg of DOTA-NHS (1.5 eq.) were dissolved in 5 mL of DMF, then 0.054 mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 26-56% over 60 min, wavelength: 220 nm) to give a final product Hex-Cys-Pro-Pro-Thr-Gln-Phe-beta-HomoCys (Cys bridge TBMB (DOTA-AET)) trifluoroacetate.

HPLC purity was 99.1%, MS (m/z): 743.3 [M+2H]²⁺.

### Example 9

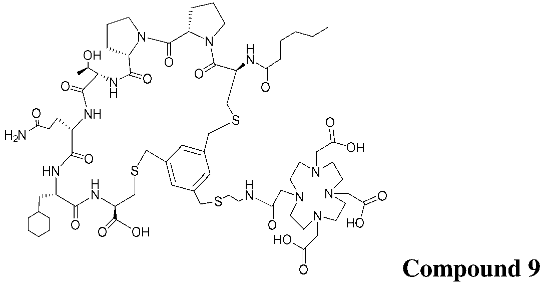

**Step 1:** 0.323 g of Fmoc-Cys(Trt)-CTC Resin with a degree of substitution of 0.619 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, treated with solution E (TFA : EDT : H₂O : phenol : TIS = 90 : 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 32-62% over 60 min, wavelength: 220 nm) to give an intermediate Hex-Cys-Pro-Pro-Thr-Gln-Cha-Cys-OH.

**Step 2:** 77 mg (1.0 eq.) of the final product obtained in step 1 was dissolved in 100 mL of 50 mM aqueous ammonium bicarbonate solution and acetonitrile (with a volume ratio of 1 : 1), and a solution of 46 mg TBMB (1.5 eq.) in 0.5 mL of acetonitrile was added, the mixture was reacted at room temperature for 1 h, then 66 mg of cysteamine (10 eq.) was added and stirred for another 2 h, 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 30-60% over 60 min, wavelength: 220 nm) to give an intermediate 2 Hex-Cys-Pro-Pro-Thr-Gln-Cha-Cys (Cys bridge TBMB (H-AET)). 40 mg of the intermediate 2 (1.0 eq.) and 28 mg of DOTA-NHS (1.5 eq.) were dissolved in 5 mL of DMF, then 0.038mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 29-59% over 60 min, wavelength: 220 nm) to give a final product Hex-Cys-Pro-Pro-Thr-Gln-Cha-Cys (Cys bridge TBMB (DOTA-AET)) trifluoroacetate.

HPLC purity was 98.4%, MS (m/z): 1477.2 [M+H]⁺.

### Example 10

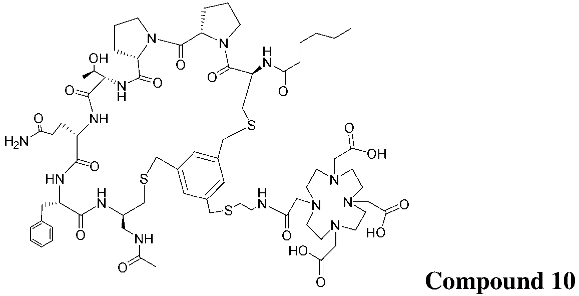

**Step 1:** 0.64 g of Fmoc-Phe-CTC Resin with a degree of substitution of 0.94 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, and the obtained crude resin was treated with 10 mL of 1% TFA/DCM. The organic phase was respectively washed with water and saturated brine, and concentrated in vacuum to give the fully protected intermediate Hex-Cys(Trt)-Pro-Pro-Thr(tBu)-Gln(Trt)-Phe-OH. 277 mg of the above intermediate and 37 mg of HOOBt (1.1 eq.) were dissolved in 10 mL of DMF and cooled to 0°C, then 44 mg of EDC hydrochloride (1.1 eq.) and H-Cys(Trt)-CH₂NHAc (1.0 eq.) were successively added, and the mixture was reacted at room temperature. Upon completion of the reaction, DMF was removed via concentration in vacuum, and 10 mL of 5% aqueous phosphoric acid solution and 20 mL of ethyl acetate were added. The organic phase was respectively washed with H₂O and saturated brine, concentrated in vacuum and drained to give the intermediate Hex-Cys(Trt)-Pro-Pro-Thr(tBu)-Gln(Trt)-Phe-Cys(Trt)-CH₂NHAc. 354 mg of the intermediate was respectively treated with mL of solution E (TFA : EDT : H₂O : phenol : TIS = 90 : 2.5 : 2.5 : 2.5 : 2.5) and methyl tert-butyl ether, and the crude product was washed with 40 mL of methyl tert-butyl ether, dried and purified by HPLC (separated by preparative chromatography, column: Huapu C18 10 µm 100A 20*250 mm, mobile phase: A: 0.1% TFA in water; B: 0.1% TFA in ACN, linear gradient: 30-60% over 60 min, wavelength: 220 nm) to give an intermediate Hex-Cys-Pro-Pro-Thr-Gln-Phe-Cys-CH₂NHAc.

**Step 2:** 102 mg (1.0 eq.) of the intermediate was dissolved in 100 mL of 50 mM aqueous ammonium bicarbonate solution and acetonitrile (with a volume ratio of 1 : 1), and a solution of 59 mg TBMB (1.5 eq.) in 0.5 mL of acetonitrile was added, the mixture was reacted at room temperature for 1 h, then 86 mg of cysteamine (10 eq.) was added and stirred for another 2 h, 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 27-57% over 60 min, wavelength: 220 nm) to give an intermediate 2 Hex-Cys-Pro-Pro-Thr-Gln-Phe-Cys-CH₂NHAc (Cys bridge TBMB (H-AET)). 62 mg of the intermediate 2 (1.0 eq.) and 42 mg of DOTA-NHS (1.5 eq.) were dissolved in 5 mL of DMF, then 0.058 mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 26-56% over 60 min, wavelength: 220 nm) to give a final product Hex-Cys-Pro-Pro-Thr-Gln-Phe-Cys-CH₂NHAc (Cys bridge TBMB (DOTA-AET)) trifluoroacetate.

HPLC purity was 99.5%, MS (m/z): 1498.1 [M+H]⁺.

### Example 11

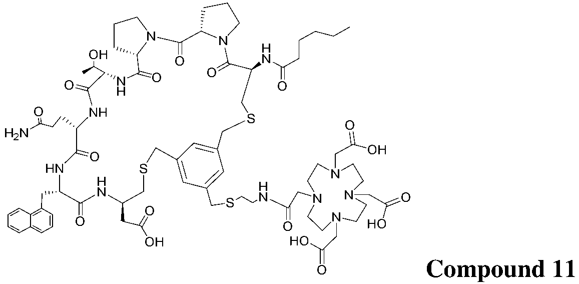

**Step 1:** 0.38 g of Fmoc-Beta-HomoCys(Trt)-CTC Resin with a degree of substitution of 0.52 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, treated with solution E (TFA : EDT : H₂O : phenol : TIS = 90 : 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 33-63% over 60 min, wavelength: 220 nm) to give an intermediate Hex-Cys-Pro-Pro-Thr-Gln-1Nal-beta-HomoCys-OH.

**Step 2:** 72 mg (1.0 eq.) of the final product obtained in step 1 was dissolved in 100 mL of 50 mM aqueous ammonium bicarbonate solution and acetonitrile (with a volume ratio of 1 : 1), and a solution of 40 mg TBMB (1.5 eq.) in 0.5 mL of acetonitrile was added, the mixture was reacted at room temperature for 1 h, then 58 mg of cysteamine (10 eq.) was added and stirred for another 2 h, 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 31-61% over 60 min, wavelength: 220 nm) to give an intermediate 2 Hex-Cys-Pro-Pro-Thr-Gln-1Nal-beta-HomoCys (Cys bridge TBMB (H-AET)). 39 mg of the intermediate 2 (1.0 eq.) and 26 mg of DOTA-NHS (1.5 eq.) were dissolved in 5 mL of DMF, then 0.035 mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 29-59% over 60 min, wavelength: 220 nm) to give a final product Hex-Cys-Pro-Pro-Thr-Gln-1Nal-beta-HomoCys (Cys bridge TBMB (DOTA-AET)) trifluoroacetate.

HPLC purity was 99.0%, MS (m/z): 1534.6 [M+H]⁺.

### Example 12

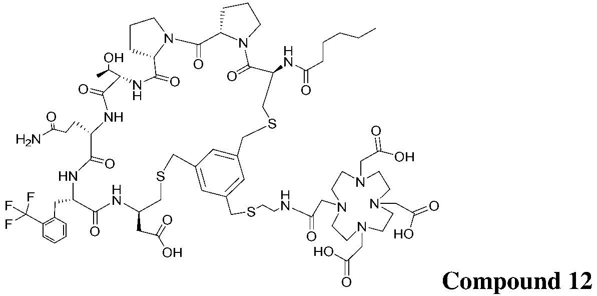

**Step 1:** 0.38 g of Fmoc-Beta-HomoCys(Trt)-CTC Resin with a degree of substitution of 0.52 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, treated with solution E (TFA : EDT : H₂O : phenol : TIS = 90 : 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 32-62% over 60 min, wavelength: 220 nm) to give an intermediate Hex-Cys-Pro-Pro-Thr-Gln-Phe(2-CF3)-beta-HomoCys-OH.

**Step 2:** 116 mg (1.0 eq.) of the product obtained in step 1 was dissolved in 100 mL of 50 mM aqueous ammonium bicarbonate solution and acetonitrile (with a volume ratio of 1 : 1), and a solution of 64 mg TBMB (1.5 eq.) in 0.5 mL of acetonitrile was added, the mixture was reacted at room temperature for 1 h, then 92 mg of cysteamine (10 eq.) was added and stirred for another 2 h, 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 30-70% over 60 min, wavelength: 220 nm) to give an intermediate 2 Hex-Cys-Pro-Pro-Thr-Gln-Phe(2-CF3)-beta-HomoCys (Cys bridge TBMB (H-AET)). 63 mg of the intermediate 2 (1.0 eq.) and 41 mg of DOTA-NHS (1.5 eq.) were dissolved in 5 mL of DMF, then 0.056 mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 31-61% over 60 min, wavelength: 220 nm) to give a final product Hex-Cys-Pro-Pro-Thr-Gln- Phe(2-CF3)-beta-HomoCys (Cys bridge TBMB (DOTA-AET)) trifluoroacetate.

HPLC purity was 99.2%, MS (m/z): 1553.1 [M+H]⁺.

### Example 13

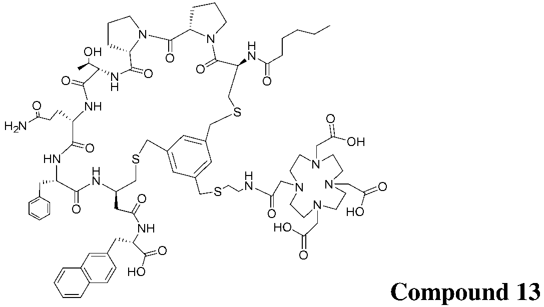

**Step 1:** 0.43 g of Fmoc-2Nal-Wang Resin with a degree of substitution of 0.464 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, treated with solution E (TFA : EDT : H₂O : phenol : TIS = 90 : 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 38-68% over 60 min, wavelength: 220 nm) to give an intermediate Hex-Cys-Pro-Pro-Thr-Gln-Phe-beta-HomoCys-2Nal-OH.

**Step 2:** 83 mg (1.0 eq.) of the final product obtained in step 1 was dissolved in 100 mL of 50 mM aqueous ammonium bicarbonate solution and acetonitrile (with a volume ratio of 1 : 1), and a solution of 40 mg TBMB (1.5 eq.) in 0.5 mL of acetonitrile was added, the mixture was reacted at room temperature for 1 h, then 58 mg of cysteamine (10 eq.) was added and stirred for another 2 h, 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 35-65% over 60 min, wavelength: 220 nm) to give an intermediate 2 Hex-Cys-Pro-Pro-Thr-Gln-Phe-beta-HomoCys-2Nal (Cys bridge TBMB (H-AET)). 41 mg of the intermediate 2 (1.0 eq.) and 24 mg of DOTA-NHS (1.5 eq.) were dissolved in 5 mL of DMF, then 0.0533 mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 33-63% over 60 min, wavelength: 220 nm) to give a final product Hex-Cys-Pro-Pro-Thr-Gln-Phe-beta-HomoCys-2Nal (Cys bridge TBMB (DOTA-AET)) trifluoroacetate.

HPLC purity was 97.5%, MS (m/z): 1682 [M+H]⁺.

### Example 14

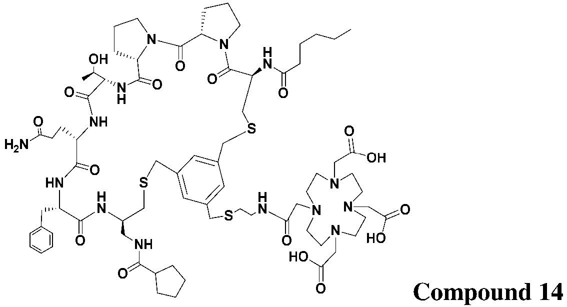

**Step 1:** 0.64 g of Fmoc-Phe-CTC Resin with a degree of substitution of 0.94 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, and the obtained crude resin was treated with 10 mL of 1% TFA/DCM. The organic phase was respectively washed with water and saturated brine, and concentrated in vacuum to give the fully protected intermediate Hex-Cys(Trt)-Pro-Pro-Thr(tBu)-Gln(Trt)-Phe-OH. 267 mg of the intermediate and 65 mg of HOOBt (2.0 eq.) were dissolved in 10 mL of DMF and cooled to 0°C, then 77 mg of EDC hydrochloride (2.0 eq.) and 112 mg of H-Cys(Trt)-CH₂NH-cyclopentanecarboxylic acid (1.0 eq.) were successively added, and the mixture was reacted at room temperature. Upon completion of the reaction, DMF was removed via concentration in vacuum, and 10 mL of 5% aqueous phosphoric acid solution and 20 mL of ethyl acetate were added. The organic phase was successively washed with water and saturated brine and concentrated in vacuum to give the intermediate Hex-Cys(Trt)-Pro-Pro-Thr(tBu)-Gln(Trt)-Phe-Cys(Trt)-CH₂NH-cyclopentanecarboxylic acid. 352 mg of the intermediate was treated with solution E (TFA : EDT : H₂O : phenol : TIS = 90 : 2.5 : 2.5 : 2.5 : 2.5) and methyl tert-butyl ether, and centrifuged, and the obtained solid was washed with methyl tert-butyl ether, dried and purified by HPLC (separated by preparative chromatography, column: Huapu C18 10 µm 100A 20*250 mm, mobile phase: A: 0.1% TFA in water; B: 0.1% TFA in ACN, linear gradient: 34-64% over 60 min, wavelength: 220 nm) to give an intermediate Hex-Cys-Pro-Pro-Thr-Gln-Phe-Cys-CH₂NH-cyclopentacarboxylic acid.

**Step 2:** 76 mg (1.0 eq.) of the final product obtained in step 1 was dissolved in 100 mL of 50 mM aqueous ammonium bicarbonate solution and acetonitrile (with a volume ratio of 1 : 1), and a solution of 42 mg TBMB (1.5 eq.) in 0.5 mL of acetonitrile was added, the mixture was reacted at room temperature for 1 h, then 60 mg of cysteamine (10 eq.) was added and stirred for another 2 h, 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 32-62% over 60 min, wavelength: 220 nm) to give an intermediate 2 Hex-Cys-Pro-Pro-Thr-Gln-Phe-Cys-CH₂NH-cyclopentacarboxylic acid (Cys bridge TBMB (H-AET)). 60 mg of the intermediate 2 (1.0 eq.) and 39 mg of DOTA-NHS (1.5 eq.) were dissolved in 5 mL of DMF, then 0.054 mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 30-60% over 60 min, wavelength: 220 nm) to give a final product Hex-Cys-Pro-Pro-Thr-Gln-Phe-Cys-CH₂NH-cyclopentacarboxylic acid (Cys bridge TBMB (DOTA-AET)) trifluoroacetate.

HPLC purity was 98.1%, MS (m/z): 1552.1 [M+H]⁺.

### Example 15

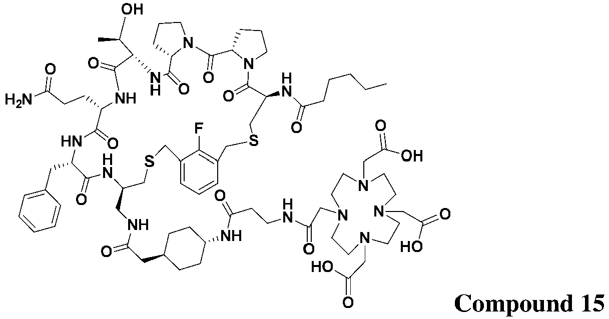

**Step 1:** 0.64 g of Fmoc-Phe-CTC Resin with a degree of substitution of 0.94 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, and the obtained crude resin was treated with 10 mL of 1% TFA/DCM. The organic phase was respectively washed with water and saturated brine, and concentrated in vacuum to give the fully protected intermediate Hex-Cys(Trt)-Pro-Pro-Thr(tBu)-Gln(Trt)-Phe-OH. 55 mg of the intermediate and 14 mg of HOOBt (2.0 eq.) were dissolved in 10 mL of DMF and cooled to 0°C, then 16 mg of EDC hydrochloride (2.0 eq.) and 46 mg of H-Cys(Trt)-CH₂NH-(trans-4-aminocyclohexane acetic acid-betaAla-DOTA(OtBu)3) (1.0 eq.) were successively added, and the mixture was reacted at room temperature. Upon completion of the reaction, DMF was removed via concentration in vacuum, 10 mL of 5% aqueous phosphoric acid solution and 20 mL of ethyl acetate were added, and the mixture was stirred and separated. The organic phase was successively washed with water and saturated brine and concentrated in vacuum to give the intermediate Hex-Cys(Trt)-Pro-Pro-Thr(tBu)-Gln(Trt)-Phe-Cys(Trt)-CH₂NH-(trans-4-aminocyclohexane acetic acid-betaAla-DOTA(OtBu)3). The intermediate was successively washed with DMF, DCM and MeOH, treated with solution E (TFA : EDT : H₂O : phenol : TIS = 90 : 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 26-56% over 60 min, wavelength: 220 nm) to give an intermediate Hex-Cys-Pro-Pro-Thr-Gln-Phe-Cys-CH₂NH-(trans-4-aminocyclohexane acetic acid-DOTA).

**Step 2:** 16 mg (1.0 eq.) of the final product obtained in step 1 was dissolved in 10 mL of 50 mM aqueous ammonium bicarbonate solution and acetonitrile (with a volume ratio of 1 : 1), and a solution of 4.6 mg 2,6-bis(bromomethyl)fluorobenzene (1.0 eq.) in 0.5 mL of acetonitrile was added, the mixture was reacted at room temperature for 1 h, then 1 mL of acetic acid was added, and the mixture was concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 32-62% over 60 min, wavelength: 220 nm) to give a final product Hex-Cys-Pro-Pro-Thr-Gln-Phe-Cys-CH₂NH-(trans-4-aminocyclohexane acetic acid-betaAla-DOTA) (Cys bridge 2,6-bis(bromomethyl)fluorobenzene) trifluoroacetate.

HPLC purity was 96.6%, MS (m/z): 1595.1 [M+H]⁺.

### Example 16

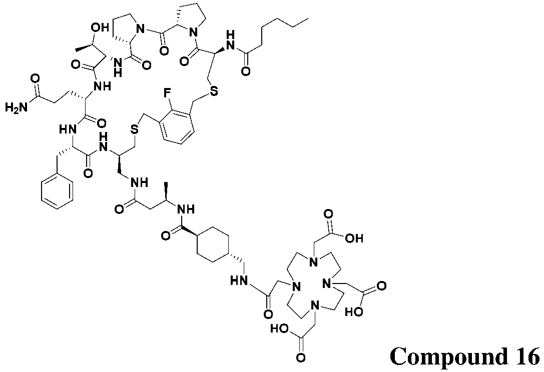

**Step 1:** 0.64 g of Fmoc-Phe-CTC Resin with a degree of substitution of 0.94 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, and the obtained crude resin was treated with 10 mL of 1% TFA/DCM. The organic phase was respectively washed with water and saturated brine, and concentrated in vacuum to give the fully protected intermediate Hex-Cys(Trt)-Pro-Pro-Thr(tBu)-Gln(Trt)-Phe-OH. 77 mg of the intermediate and 19 mg of HOOBt (2.0 eq.) were dissolved in 10 mL of DMF and cooled to 0°C, then 22 mg of EDC hydrochloride (2.0 eq.) and 65 mg of H-Cys-CH₂NH-(D-beta-HomoAla-tranexamic acid-DOTA(OtBu)3) (1.0 eq.) were successively added, and the mixture was reacted at room temperature. Upon completion of the reaction, DMF was removed via concentration in vacuum, 10 mL of 5% aqueous phosphoric acid solution and 20 mL of ethyl acetate were added, and the mixture was stirred and separated. The organic phase was successively washed with water and saturated brine, concentrated in vacuum and drained to give the intermediate Hex-Cys(Trt)-Pro-Pro-Thr(tBu)-Gln(Trt)-Phe-Cys(Trt)-CH₂NH-(D-beta-HomoAla-tranexamic acid-DOTA(OtBu)3). 140 mg of the intermediate was successively washed with DMF, DCM and MeOH, treated with solution E (TFA : EDT : H₂O : phenol : TIS = 90 : 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 26-56% over 60 min, wavelength: 220 nm) to give an intermediate Hex-Cys-Pro-Pro-Thr-Gln-Phe-Cys-CH₂NH-(D-beta-HomoAla-tranexamic acid-1-carboxylicacid-DOTA).

**Step 2:** 22 mg (1.0 eq.) of the final product obtained in step 1 was dissolved in 10 mL of 50 mM aqueous ammonium bicarbonate solution and acetonitrile (with a volume ratio of 1 : 1), and a solution of 6.2 mg 2,6-bis(bromomethyl)fluorobenzene (1.0 eq.) in 0.5 mL of acetonitrile was added, the mixture was reacted at room temperature for 1 h, then 1 mL of acetic acid was added, and the mixture was concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 30-60% over 60 min, wavelength: 220 nm) to give a final product Hex-Cys-Pro-Pro-Thr-Gln-Phe-Cys-CH₂NH-(D-beta-HomoAla-tranexamic acid-DOTA) (Cys bridge 2,6-bis(bromomethyl)fluorobenzene) trifluoroacetate.

HPLC purity was 99.4%, MS (m/z): 1608.6 [M+H]⁺.

### Example 17

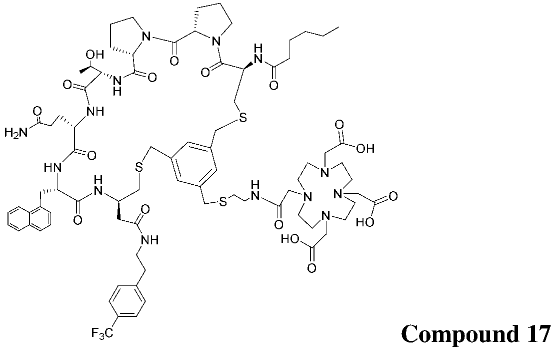

**Step 1:** 0.35 g of Fmoc-beta-Homo Cys(Trt)-CTC Resin with a degree of substitution of 0.59 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, and the obtained crude resin was treated with 10 mL of 1% TFA/DCM. The organic phase was respectively washed with water and saturated brine, and concentrated in vacuum to give the fully protected intermediate Hex-Cys(Trt)-Pro-Pro-Thr(tBu)-Gln(Trt)-1Nal-beta-HomoCys(Trt)-OH. 281 mg of the intermediate and 58 mg of HOOBt (2.2 eq.) were dissolved in 10 mL of DMF and cooled to 0°C, then 68 mg of EDC hydrochloride (2.2 eq.) and 61 mg of 2-(4-trifluoromethylphenyl)ethylamine (2.0 eq.) were successively added, and the mixture was reacted at room temperature. Upon completion of the reaction, DMF was removed via concentration in vacuum, 10 mL of 5% aqueous phosphoric acid solution and 20 mL of ethyl acetate were added, and the mixture was stirred and separated. The organic phase was successively washed with water and saturated brine and concentrated in vacuum to give the intermediate Hex-Cys(Trt)-Pro-Pro-Thr(tBu)-Gln(Trt)-1Nal-beta-HomoCys(Trt)-2-(4-trifluoromethylphenyl)ethylamine. 309 mg of the intermediate was successively washed with DMF, DCM and MeOH, treated with solution E (TFA : EDT : H₂O : phenol : TIS = 90: 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 45-75% over 60 min, wavelength: 220 nm) to give an intermediate Hex-Cys-Pro-Pro-Thr-Gln-1Nal-beta-HomoCys-2-(4-trifluoromethylphenyl)ethylamine.

**Step 2:** 91 mg (1.0 eq.) of the final product obtained in step 1 was dissolved in 100 mL of 50 mM aqueous ammonium bicarbonate solution and acetonitrile (with a volume ratio of 1 : 1), and a solution of 43 mg TBMB (1.5 eq.) in 0.5 mL of acetonitrile was added, the mixture was reacted at room temperature for 1 h, then 62 mg of cysteamine (10 eq.) was added and stirred for another 2 h, 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 40-70% over 60 min, wavelength: 220 nm) to give an intermediate 2 Hex-Cys-Pro-Pro-Thr-Gln-1Nal-beta-HomoCys-2-(4-trifluoromethylphenyl)ethylamine (Cys bridge TBMB (H-AET)). 60 mg of the intermediate 2 (1.0 eq.) and 34 mg of DOTA-NHS (1.5 eq.) were dissolved in 5 mL of DMF, then 0.048 mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 38-68% over 60 min, wavelength: 220 nm) to give a final product Hex-Cys-Pro-Pro-Thr-Gln-1Nal-beta-HomoCys- 2-(4-trifluoromethylphenyl) ethylamine (Cys bridge TBMB (DOTA-AET)) trifluoroacetate.

HPLC purity was 96.7%, MS (m/z): 853.5 [M+2H]²⁺.

### Example 18

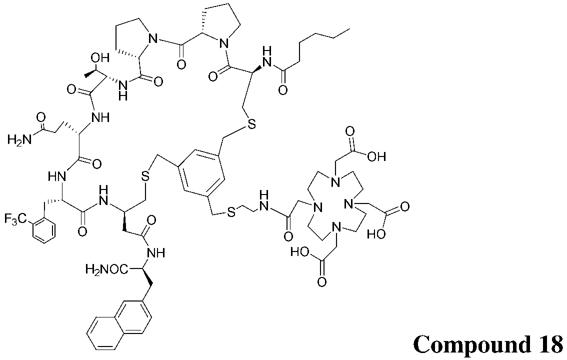

**Step 1:** 0.5 g of Fmoc-Linker MBHA Resin with a degree of substitution of 0.405 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, treated with solution E (TFA : EDT : H₂O : phenol : TIS = 90: 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 40-70% over 60 min, wavelength: 220 nm) to give an intermediate Hex-Cys-Pro-Pro-Thr-Gln-Phe(2-CF3)-beta-HomoCys-2Nal-NH₂.

**Step 2:** 103 mg (1.0 eq.) of the final product obtained in step 1 was dissolved in 100 mL of 50 mM aqueous ammonium bicarbonate solution and acetonitrile (with a volume ratio of 1 : 1), and a solution of 47 mg TBMB (1.5 eq.) in 0.5 mL of acetonitrile was added, the mixture was reacted at room temperature for 1 h, then 68 mg of cysteamine (10 eq.) was added and stirred for another 2 h, 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 37-67% over 60 min, wavelength: 220 nm) to give an intermediate 2 Hex-Cys-Pro-Pro-Thr-Gln-Phe(2-CF3)-beta-HomoCys-2Nal-NH₂ (Cys bridge TBMB (H-AET)). 59 mg of the intermediate 2 (1.0 eq.) and 33 mg of DOTA-NHS (1.5 eq.) were dissolved in 5 mL of DMF, then 0.046 mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 34-64% over 60 min, wavelength: 220 nm) to give a final product Hex-Cys-Pro-Pro-Thr-Gln-Phe(2-CF3)-beta-HomoCys-2Nal-NH₂ trifluoroacetate.

HPLC purity was 97.6%, MS (m/z): 875.1 [M+2H]²⁺.

### Example 19

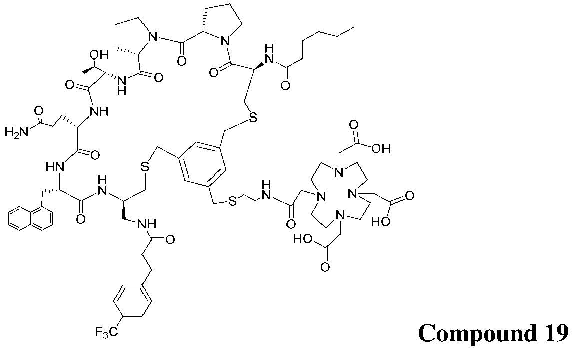

**Step 1:** 0.3 g of Fmoc-1Nal-CTC Resin with a degree of substitution of 0.69 mmol/g was weighed and placed in a reactor, soaked with 10 mL of N,N-dimethylformamide (DMF) for 2 h and then drained, and deprotection was performed with 10 mL of 20% piperidine (Pip)/DMF. Raw materials (amino acid : N,N'-diisopropylcarbodiimide (DIC) : 1-hydroxybenzotriazole (HOBt) = 3 : 3 : 3) were charged in equivalent proportions and 10 mL of DMF was added for reaction, and then the resulting mixture was drained and washed with 15 mL of DMF. According to the polypeptide sequence, the respective amino acids were added successively, and all amino acids were linked via solid phase synthesis. The crude resin was successively washed with DMF, DCM and MeOH, and the obtained crude resin was treated with 10 mL of 1% TFA/DCM. The organic phase was respectively washed with water and saturated brine, and concentrated in vacuum to give the fully protected intermediate Hex-Cys(Trt)-Pro-Pro-Thr(tBu)-Gln(Trt)-1Nal-OH. 233 mg of the intermediate and 55 mg of HOOBt (2.0 eq.) were dissolved in 10 mL of DMF and cooled to 0°C, then 65 mg of EDC hydrochloride (2.0 eq.) and 93 mg of H-Cys(Trt)-CH₂NH-3-(4-trifluoromethylphenyl)propionic acid (1.0 eq.) were successively added, and the mixture was reacted at room temperature. Upon completion of the reaction, DMF was removed via concentration in vacuum, 10 mL of 5% aqueous phosphoric acid solution and 20 mL of ethyl acetate were added, and the mixture was stirred and separated. The organic phase was successively washed with water and saturated brine and concentrated in vacuum to give the intermediate Hex-Cys(Trt)-Pro-Pro-Thr(tBu)-Gln(Trt)-1Nal-Cys(Trt)-CH₂NH-3-(4-trifluoromethylphenyl)propionic acid. 323 mg of the intermediate was successively washed with DMF, DCM and MeOH, treated with solution E (TFA : EDT : H₂O : phenol : TIS = 90: 2.5 : 2.5 : 2.5 : 2.5) and methyl tert-butyl ether, and centrifuged, and the product was washed with methyl tert-butyl ether again, dried and purified by HPLC (separated by preparative chromatography, column: Huapu C18 10 µm 100A 20*250 mm, mobile phase: A: 0.1% TFA in water; B: 0.1% TFA in ACN, linear gradient: 34-64% over 60 min, wavelength: 220 nm) to give an intermediate Hex-Cys-Pro-Pro-Thr-Gln-1Nal-Cys-CH₂NH-3-(4-trifluoromethylphenyl)propanoic acid.

**Step 2:** 56 mg (1.0 eq.) of the final product obtained in step 1 was dissolved in 60 mL of 50 mM aqueous ammonium bicarbonate solution and acetonitrile (with a volume ratio of 1 : 1), and a solution of 27 mg TBMB (1.5 eq.) in 0.5 mL of acetonitrile was added, the mixture was reacted at room temperature for 1 h, then 38 mg of cysteamine (10 eq.) was added and stirred for another 2 h, 1 mL of acetic acid was added, and the mixture was concentrated, lyophilized and then purified by HPLC (separated by preparative chromatography, column: Huapu C18 10 µm 100A 20*250 mm, mobile phase: A: 0.1% TFA in water; B: 0.1% TFA in ACN, linear gradient: 41-71% over 60 min, wavelength: 220 nm) to give an intermediate 2 Hex-Cys-Pro-Pro-Thr-Gln-1Nal-Cys-CH₂NH-3-(4-trifluoromethylphenyl)propanoic acid (Cys bridge TBMB (H-AET)). 29 mg of the intermediate 2 (1.0 eq.) and 17 mg of DOTA-NHS (1.5 eq.) were dissolved in 5 mL of DMF, then 0.023 mL of DIPEA (6 eq.) was added, and the mixture was reacted at room temperature for 2 h, concentrated and then purified by HPLC (separated by preparative chromatography, column: Huapu C18 10 µm 100A 20*250 mm, mobile phase: A: 0.1% TFA in water; B: 0.1% TFA in ACN, linear gradient: 38-68% over 60 min, wavelength: 220 nm) to give a final product Hex-Cys-Pro-Pro-Thr-Gln-1Nal-Cys-CH₂NH-3-(4-trifluoromethylphenyl)propanoic acid (Cys bridge TBMB (DOTA-AET)) trifluoroacetate.

HPLC purity was 96.1%, MS (m/z): 853.3 [M+2H]²⁺.

### Examples 20-25

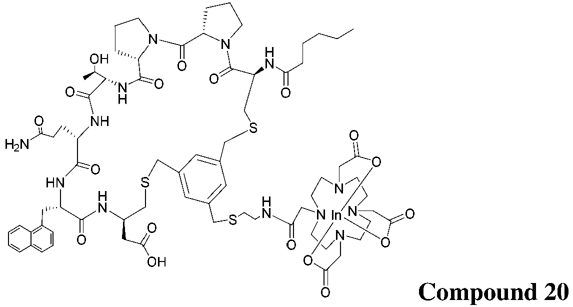

To a solution of compound 11 (0.4 mM) in 4.9 mL of sodium acetate (0.4 M, pH 5.0), 3 equivalents of indium(III) chloride (1.2 mM) was added. The resulting mixture was vortexed at 50°C for 20 min or at room temperature overnight. The resulting crude product was purified by HPLC to give 1.8 mg (61% yield) of a white solid. MS (m/z): 824.5 [M+2H]²⁺.

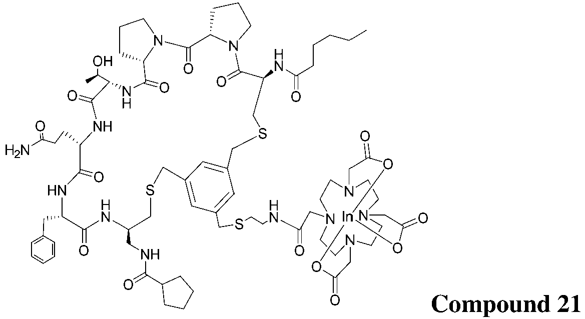

To a solution of compound 14 (0.4 mM) in 4.8 mL of sodium acetate (0.4 M, pH 5.0), 3 equivalents of indium(III) chloride (1.2 mM) was added. The resulting mixture was vortexed at 50°C for 20 min or at room temperature overnight. The resulting crude product was purified by HPLC to give 2.6 mg (81% yield) of a white solid. MS (m/z): 833.2 [M+2H]²⁺.

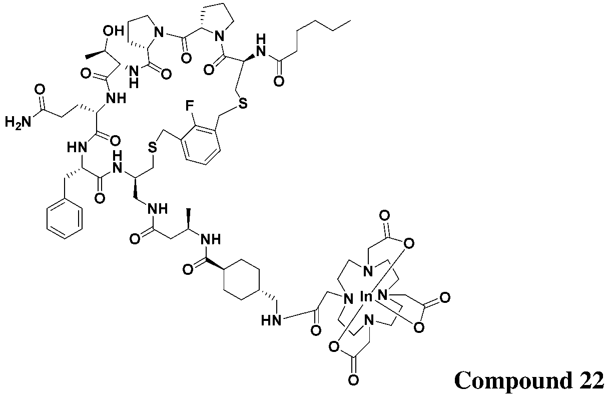

To a solution of compound 16 (0.4 mM) in 4.6 mL of sodium acetate (0.4 M, pH 5.0), 3 equivalents of indium(III) chloride (1.2 mM) was added. The resulting mixture was vortexed at 50°C for 20 min or at room temperature overnight. The resulting crude product was purified by HPLC to give 2.5 mg (78% yield) of a white solid. MS (m/z): 861.8 [M+2H]²⁺.

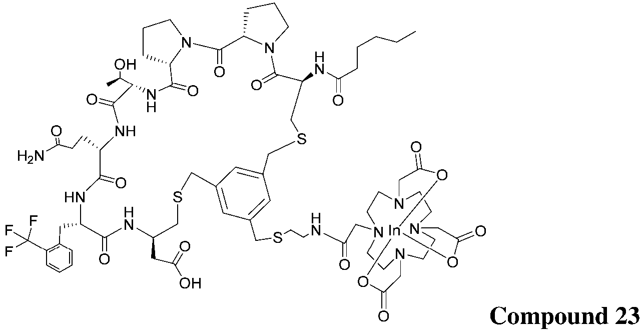

To a solution of compound 12 (0.4 mM) in 4.8 mL of sodium acetate (0.4 M, pH 5.0), 3 equivalents of indium(III) chloride (1.2 mM) was added. The resulting mixture was vortexed at 50°C for 20 min or at room temperature overnight. The resulting crude product was purified by HPLC to give 2.1 mg (66% yield) of a white solid. MS (m/z): 833.5 [M+2H]²⁺.

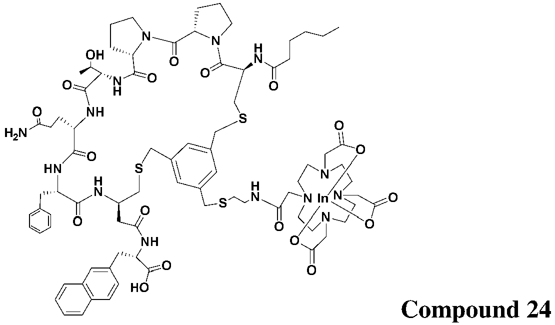

To a solution of compound 13 (0.4 mM) in 4.4 mL of sodium acetate (0.4 M, pH 5.0), 3 equivalents of indium(III) chloride (1.2 mM) was added. The resulting mixture was vortexed at 50°C for 20 min or at room temperature overnight. The resulting crude product was purified by HPLC to give 1.7 mg (53% yield) of a white solid. MS (m/z): 898.2 [M+2H]²⁺.

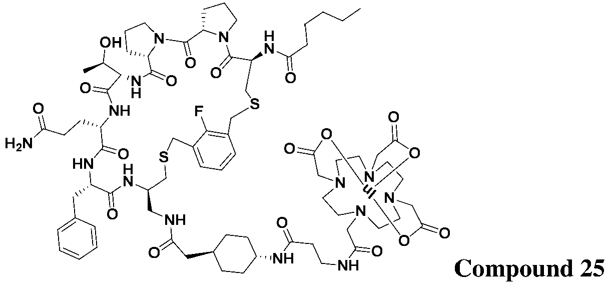

To a solution of compound 15 (0.4 mM) in 4.7 mL of sodium acetate (0.4 M, pH 5.0), 3 equivalents of indium(III) chloride (1.2 mM) was added. The resulting mixture was vortexed at 50°C for 20 min or at room temperature overnight. The resulting crude product was purified by HPLC to give 1.8 mg (56% yield) of a white solid. MS (m/z): 854.7 [M+2H]²⁺.

### Examples 26-28

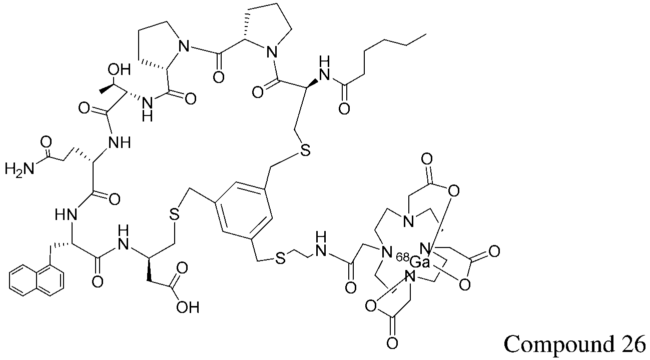

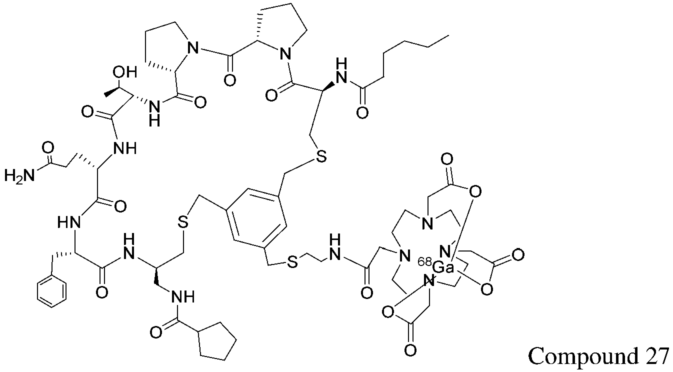

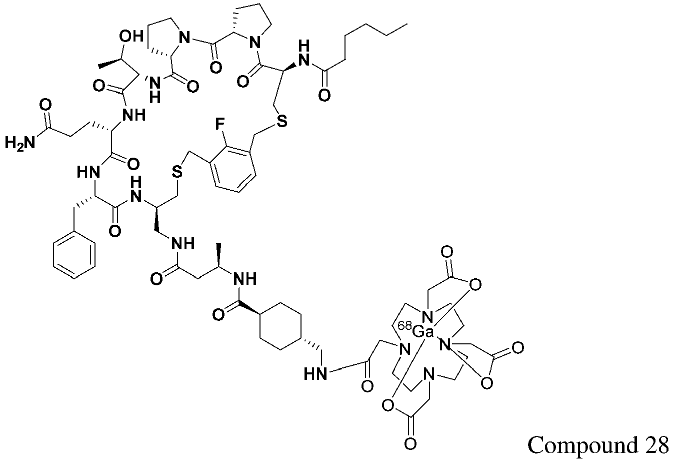

a) The compounds to be labeled (compounds 11, 14 and 16) were formulated in DMSO to a concentration of 1 mM and diluted to 0.1 mM using 0.1 M sodium acetate buffer with a pH of 4.5.
b) The germanium/gallium generator was eluted with 5 mL of 0.1 M HCl in sections, and the most active part (1 mL) was taken. To 0.25 mL of the eluent, 0.5 mL of 0.1 M sodium acetate buffer with a pH of 4.5 and 40 µL of precursors (corresponding to compounds 11, 14 and 16, respectively, 0.1 mM) were added in a 1.5 mL centrifuge tube as a reaction tube. The mixture was uniformly mixed by vortex for 10 s and heated at 95°C at 800 rpm for 15 min.
c) The C18 column was activated with anhydrous ethanol and rinsed with pure water until clean and dried.
d) The solution from the end of the reaction was passed through a C18 column, rinsed with pure water and dried, followed by rinsing with ethanol, with 3 drops of ethanol in one tube and about 10 tubes in total.

The tube with the highest radioactive dose was taken for HPLC quality control. The purity of compound 26 was 90.88%, the purity of compound 27 was 97.19%, and the purity of compound 28 was 99.79%.

### Examples 29-30

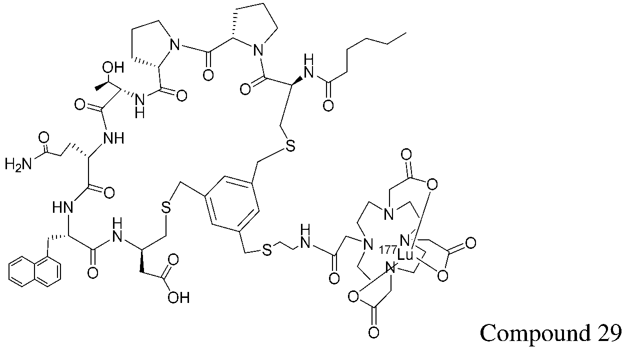

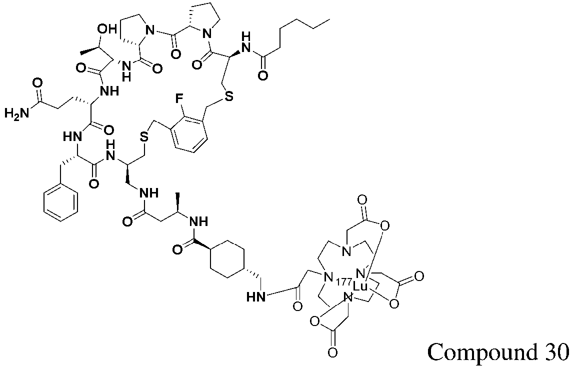

To 40 µL of the respective precursor compounds (compounds 11 and 16, 0.1 mM), 260 µL of 0.5 M acetic acid/sodium acetate buffer with a pH of 4.5 and 1.8 µL (about 2 mci) of ¹⁷⁷Lu were added respectively, and the mixture was heated at 95°C for reaction for 20 min.

The purity of the labeled compound was identified by the Radio-HPLC method.

Chromatographic conditions: mobile phase A: 0.1%TFA-H₂O; mobile phase B: 0.1% TFA-CAN; chromatographic column: Shim-pack VP-ODS 150L*4.6, 5 µm; flow rate: 1 mL/min; wavelength: 220 nm; method: time: 0 min 5% B, 15 min 95% B;

The Radio-HPLC result of compound 29 was 96.84%. The Radio-HPLC result of compound 30 was 99.54%.

### Example 31

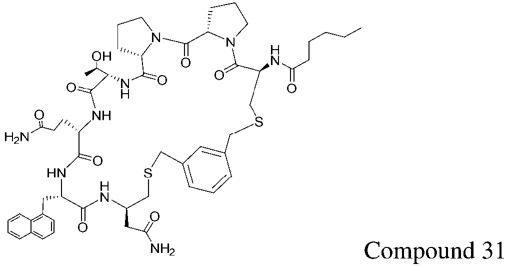

Compound 31 was synthesized with reference to the synthesis method of compound 2.

HPLC purity was 98.9%, MS (m/z): 1058.5 [M+H]⁺.

### Example 32

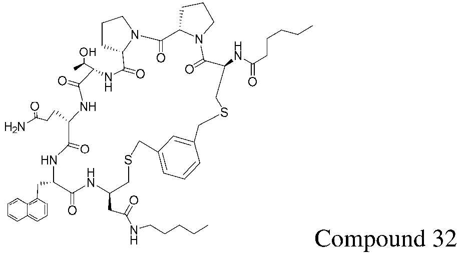

Compound 32 was synthesized with reference to the synthesis method of compound 2.

HPLC purity was 99.5%, MS (m/z): 1128.7 [M+H]⁺.

### Example 33

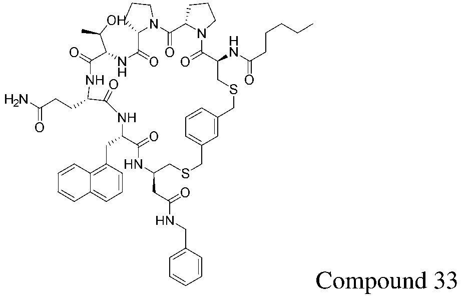

Compound 33 was synthesized with reference to the synthesis method of compound 2.

HPLC purity was 97%, MS (m/z): 1148.7 [M+H]⁺ .

### Example 34

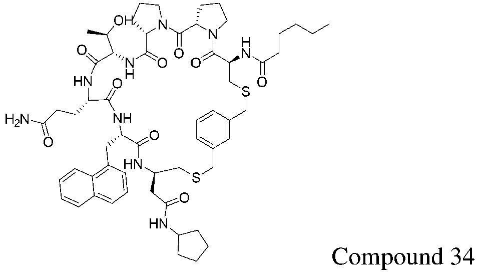

Compound 34 was synthesized with reference to the synthesis method of compound 2.

HPLC purity was 99.3%, MS (m/z): 1126.7 [M+H]⁺ .

### Example 35

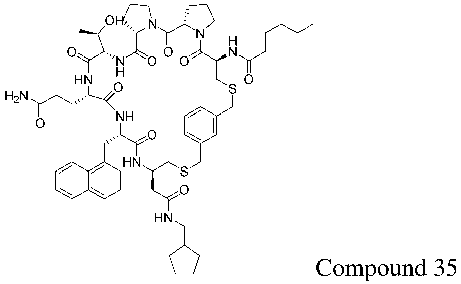

Compound 35 was synthesized with reference to the synthesis method of compound 2.

HPLC purity was 99.2%, MS (m/z): 1140.6 [M+H]⁺ .

### Example 36

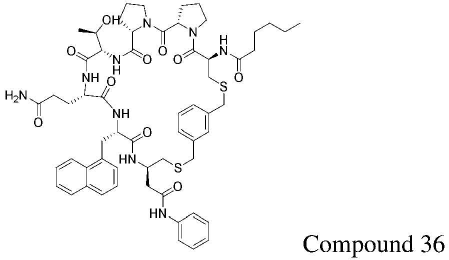

Compound 36 was synthesized with reference to the synthesis method of compound 2.

HPLC purity was 99.5%, MS (m/z): 1134.6 [M+H]⁺ .

### Example 37

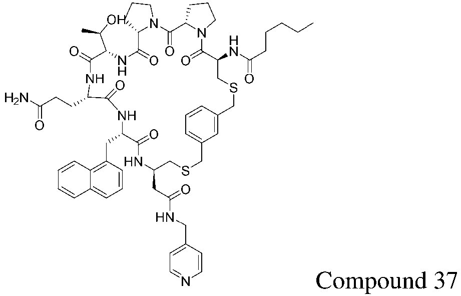

Compound 37 was synthesized with reference to the synthesis method of compound 2.

HPLC purity was 98.3%, MS (m/z): 1149.7 [M+H]⁺ .

### Example 38

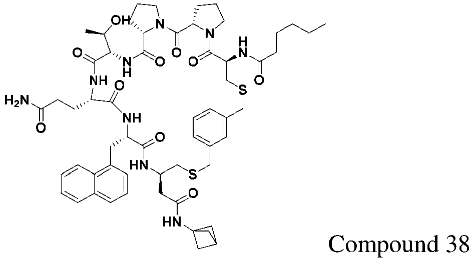

Compound 38 was synthesized with reference to the synthesis method of compound 2.

HPLC purity was 98.1%, MS (m/z): 1124.6 [M+H]⁺ .

### Example 39

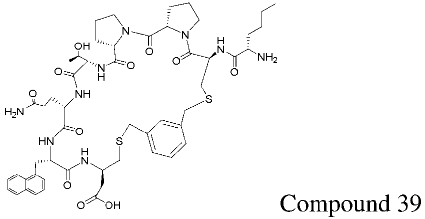

Compound 39 was synthesized with reference to the synthesis method of compound 2.

HPLC purity was 98.6%, MS (m/z): 1074.6 [M+H]⁺ .

### Example 40

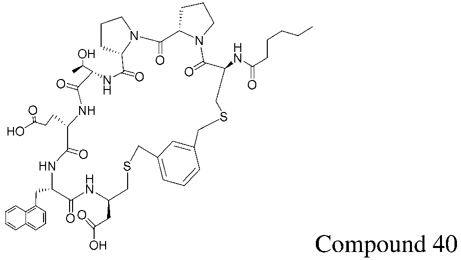

Compound 40 was synthesized with reference to the synthesis method of compound 2.

HPLC purity was 99%, MS (m/z): 1060.5 [M+H]⁺.

### Example 41

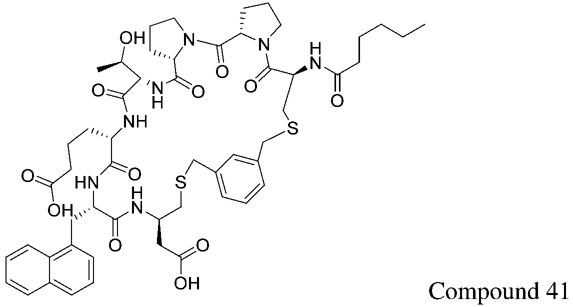

Compound 41 was synthesized with reference to the synthesis method of compound 2.

HPLC purity was 98.9%, MS (m/z): 1074.5 [M+H]⁺ .

### Example 42

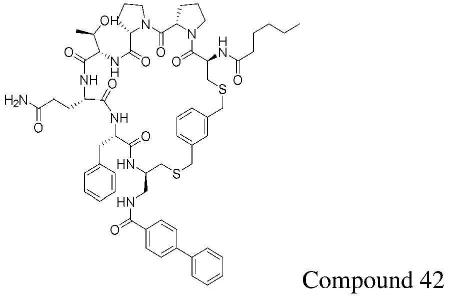

Compound 42 was synthesized with reference to the synthesis method of compound 4.

HPLC purity was 96.4%, MS (m/z): 1060.7 [M+H]⁺ .

### Example 43

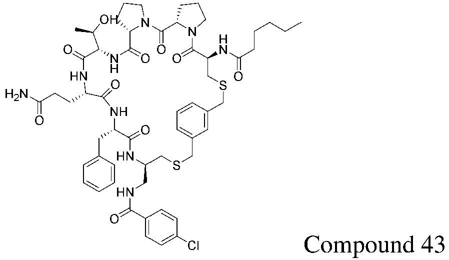

Compound 43 was synthesized with reference to the synthesis method of compound 4.

HPLC purity was 98%, MS (m/z): 1118.8 [M+H]⁺ .

### Example 44

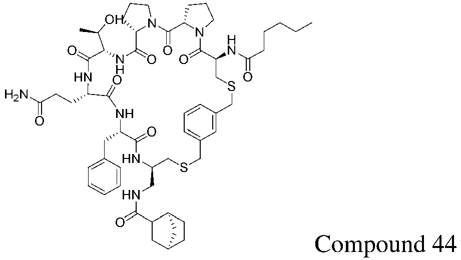

Compound 44 was synthesized with reference to the synthesis method of compound 4.

HPLC purity was 96.6%, MS (m/z): 1102.7 [M+H]⁺ .

### Example 45

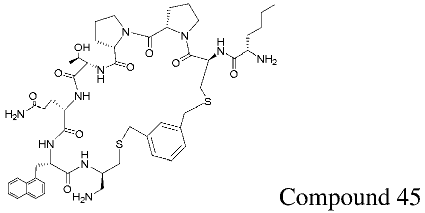

Compound 45 was synthesized with reference to the synthesis method of compound 4.

HPLC purity was 97.7%, MS (m/z): 1045.6 [M+H]⁺ .

### Example 46

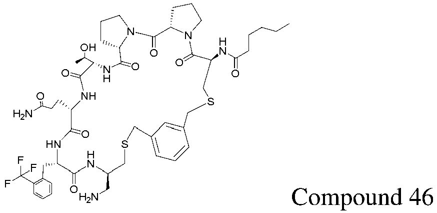

Compound 46 was synthesized with reference to the synthesis method of compound 4.

HPLC purity was 97.1%, MS (m/z): 1048.6 [M+H]⁺ .

### Example 47

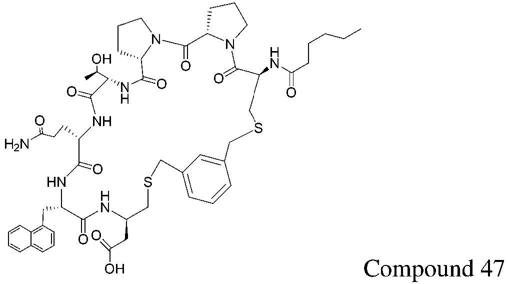

Compound 47 was synthesized with reference to the synthesis method of compound 2.

HPLC purity was 99.2%, MS (m/z): 1059.6 [M+H]⁺ .

### Example 48

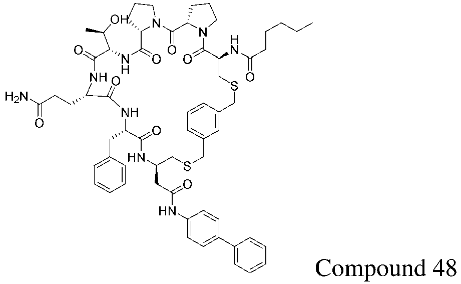

Compound 48 was synthesized with reference to the synthesis method of compound 2.

HPLC purity was 98.1%, MS (m/z): 1060.7 [M+H]⁺ .

### Example 49

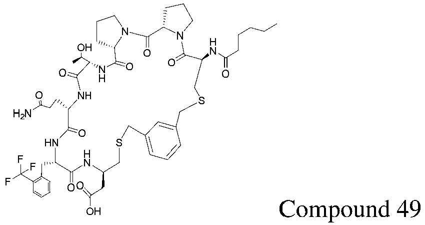

Compound 49 was synthesized with reference to the synthesis method of compound 2.

HPLC purity was 97.7%, MS (m/z): 1077.6 [M+H]⁺ .

### Example 50

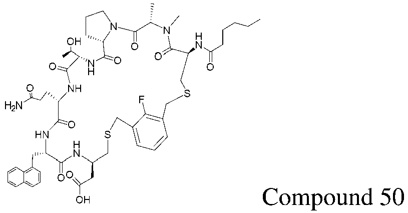

Compound 50 was synthesized with reference to the synthesis method of compound 2.

HPLC purity was 97 %, MS (m/z): 1065.6 [M+H]⁺ .

### Example 51

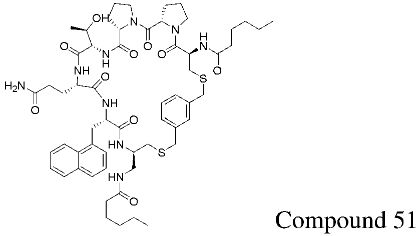

Compound 51 was synthesized with reference to the synthesis method of compound 4.

HPLC purity was 97.1%, MS (m/z): 1128.7 [M+H]⁺ .

### Example 52

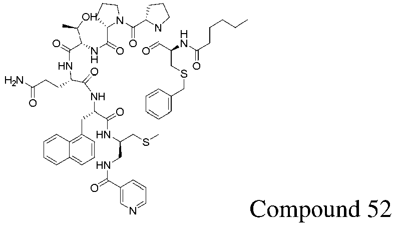

Compound 52 was synthesized with reference to the synthesis method of compound 4.

HPLC purity was 98.4%, MS (m/z): 1135.7 [M+H]⁺ .

### Example 53

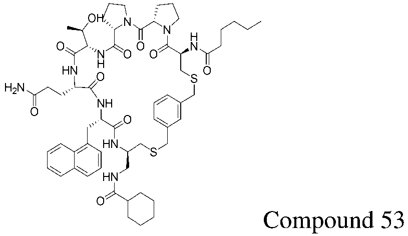

Compound 53 was synthesized with reference to the synthesis method of compound 4.

HPLC purity was 97.3%, MS (m/z): 1140.8 [M+H]⁺ .

### Example 54

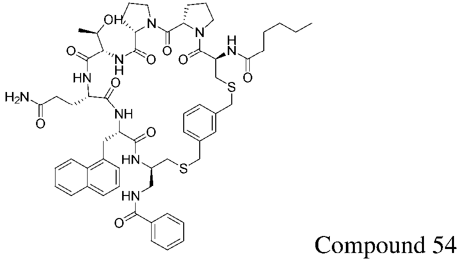

Compound 54 was synthesized with reference to the synthesis method of compound 4.

HPLC purity was 97.5%, MS (m/z): 1134.7 [M+H]⁺ .

### Example 55

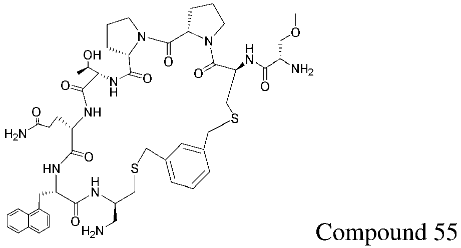

Compound 55 was synthesized with reference to the synthesis method of compound 4.

HPLC purity was 98.8%, MS (m/z): 1033.6 [M+H]⁺ .

### Example 56

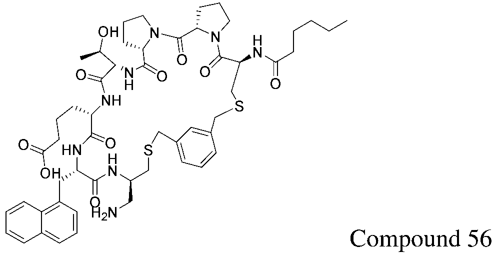

Compound 56 was synthesized with reference to the synthesis method of compound 4.

HPLC purity was 96.4%, MS (m/z): 1045.6 [M+H]⁺ .

### Example 57

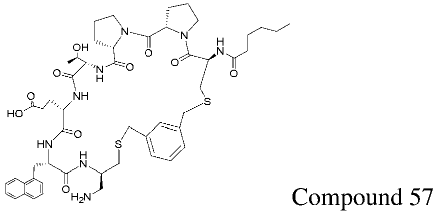

Compound 57 was synthesized with reference to the synthesis method of compound 4.

HPLC purity was 97.1%, MS (m/z): 1031.6 [M+H]⁺ .

### Example 58

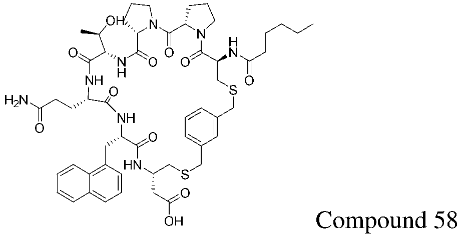

Compound 58 was synthesized with reference to the synthesis method of compound 2.

HPLC purity was 99.4%, MS (m/z): 1059.7 [M+H]⁺ .

### Example 59

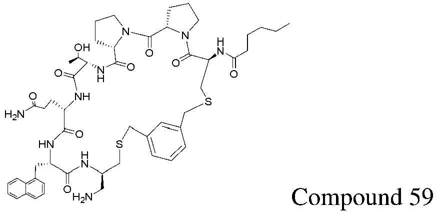

Compound 59 was synthesized with reference to the synthesis method of compound 10.

HPLC purity was 96.5%, MS (m/z): 1030.6 [M+H]⁺.

### Example 60

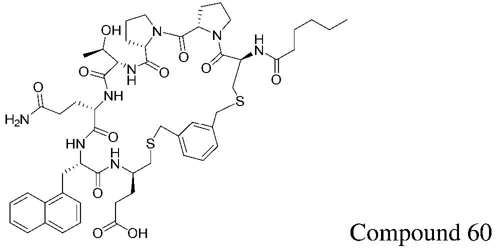

Compound 60 was synthesized with reference to the synthesis method of compound 2.

### Example 61

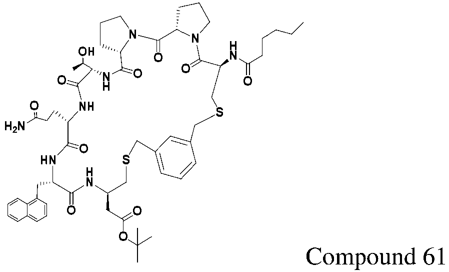

Compound 61 was synthesized with reference to the synthesis method of compound 2.

### Example 62

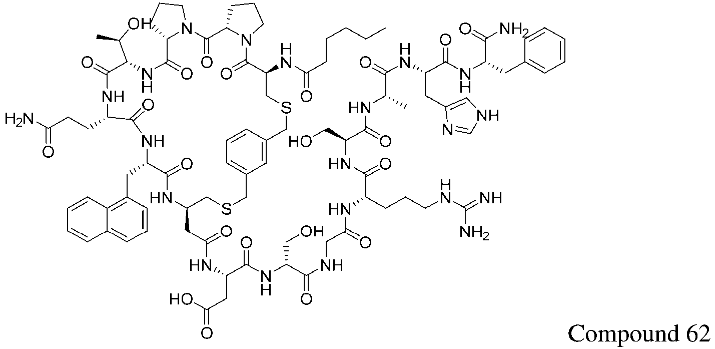

Compound 62 was synthesized with reference to the synthesis method of compound 1.

### Example 63

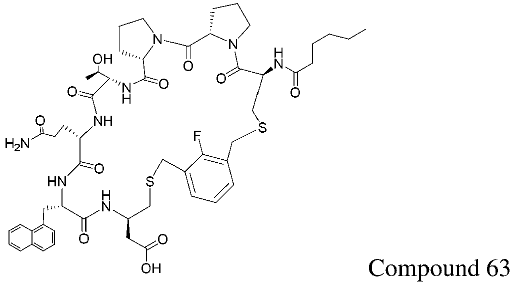

Compound 63 was synthesized with reference to the synthesis method of compound 2.

### Example 64

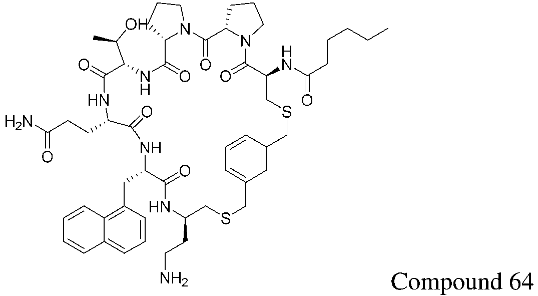

Compound 64 was synthesized with reference to the synthesis method of compound 4.

### Example 65

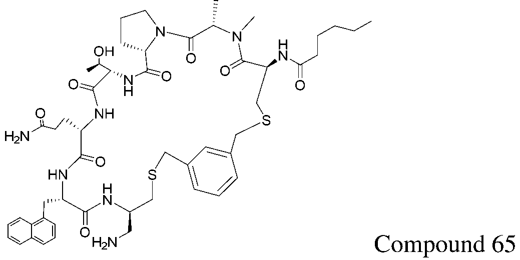

Compound 65 was synthesized with reference to the synthesis method of compound 4.

### Example 66

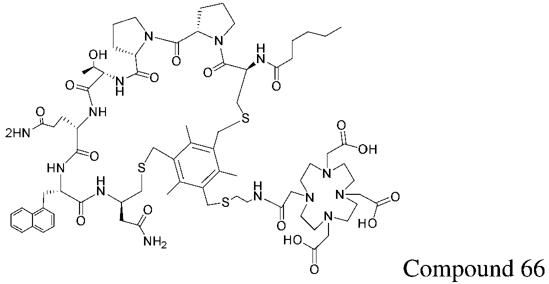

**Compound** 66 was synthesized with reference to the synthesis method of compound 11.

### Example 67

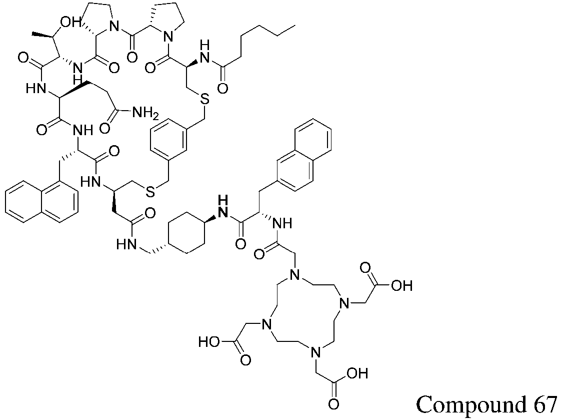

Compound 67 was synthesized with reference to the synthesis method of compound 11.

### Example 68

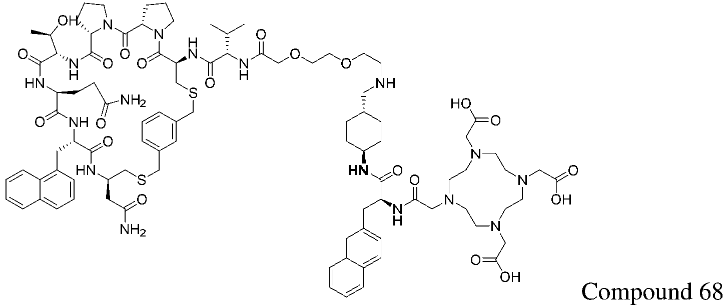

Compound 68 was synthesized with reference to the synthesis method of compound 11.

### Example 69

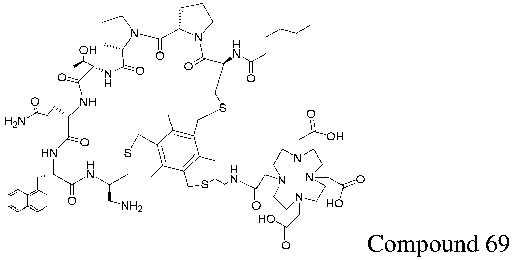

Compound 69 was synthesized with reference to the synthesis method of compound 10.

### Example 70

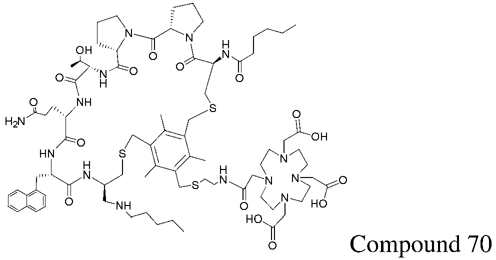

Compound 70 was synthesized with reference to the synthesis method of compound 11.

### Example 71

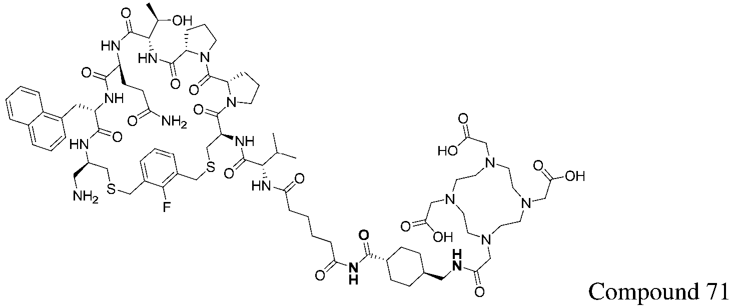

Compound 71 was synthesized with reference to the synthesis method of compound 10.

### Example 72

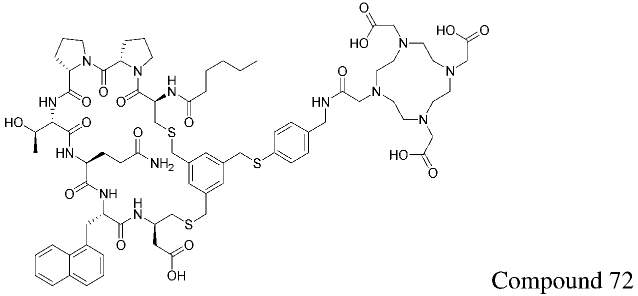

Compound 72 was synthesized with reference to the synthesis method of compound 11.

### Example 73

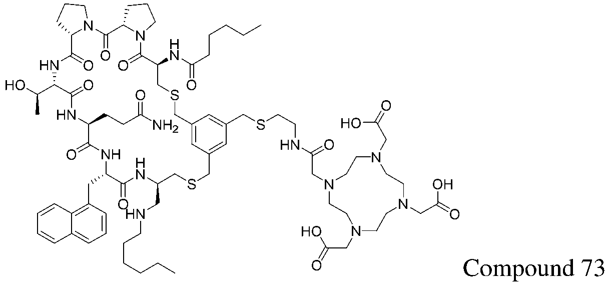

Compound 73 was synthesized with reference to the synthesis method of compound 11.

### Example 74

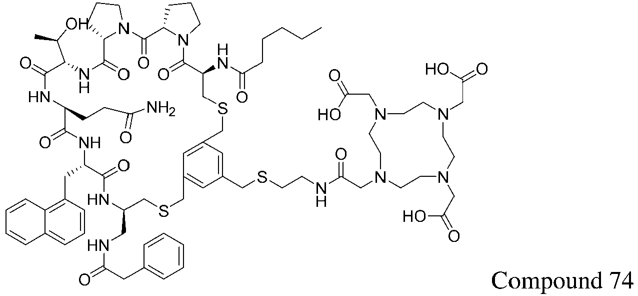

**Compound 74** was synthesized with reference to the synthesis method of compound 10.

### Example 75

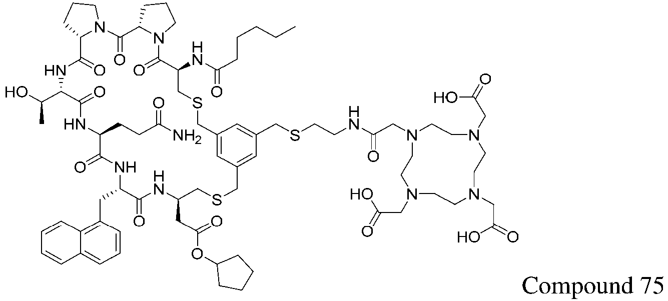

Compound 75 was synthesized with reference to the synthesis method of compound 11.

### Example 76

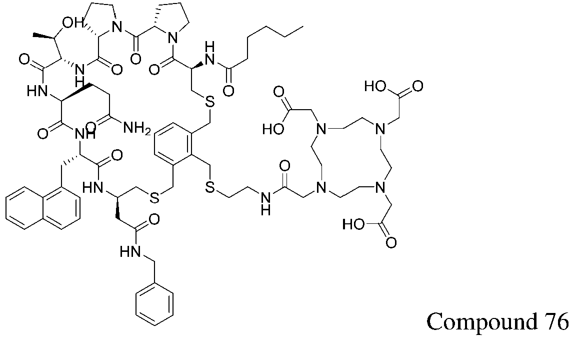

Compound 76 was synthesized with reference to the synthesis method of compound 11.

### Example 77

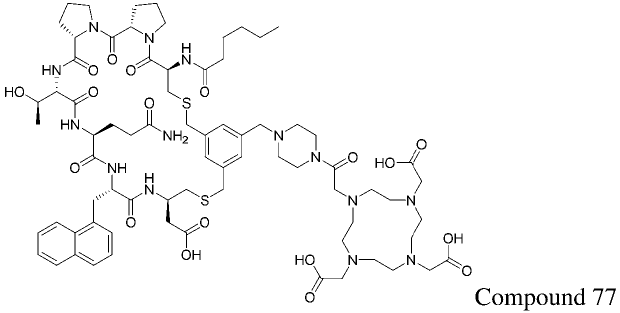

Compound 77 was synthesized with reference to the synthesis method of compound 11.

### Example 78

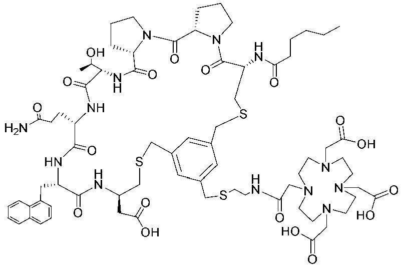

**Step 1:** 1.28 g (1 mmol) of CTC resin with a degree of substitution of 0.78 mmol/g was weighed and placed in a reactor, and the raw materials (Fmoc-betahomoCys(Trt)-OH/N,N-diisopropylethylamine (DIPEA) = 1.5/6) were dissolved in 10 mL of dichloromethane (DCM) in equivalent proportion, and the above solution was added to the reactor for reaction for 3-4 h, then 1 mL of methanol (MeOH) was added for capping, and the resulting mixture was successively washed with DCM and N,N-dimethylformamide (DMF), and then deprotected with 10 mL of 20% piperidine (Pip)/DMF. The raw materials (amino acid/1-hydroxybenzotriazole (HOBt)/N,N'-diisopropylcarbodiimide (DIC) = 2/2/2) were dissolved in 10 mL of DMF in equivalent proportion, and the above solution was added to the reactor for reaction. At the end of the reaction, the solution was drained and the product was washed with 10 mL of DMF. According to the polypeptide sequence, Fmoc-1Nal-OH, Fmoc-Gln(Trt)-OH, Fmoc-Thr(tBu)-OH, Fmoc-Pro-OH, Fmoc-Pro-OH, and Fmoc-D-Cys(Trt)-OH amino acids were added successively. Then the product was deprotected with 10 mL of 20% piperidine (Pip)/DMF, the raw materials (n-hexanoic acid/O-benzotriazazole-tetramethyluronium hexafluorophosphate (HBTU)/DIPEA = 2/1.9/4) were dissolved in 10 mL of DMF in equivalent proportion, and the above solution was added to the reactor for reaction. At the end of the reaction, the solution was drained and the peptide resin was successively washed with DMF and MeOH. Finally, the peptide resin was treated with a cut solution (trifluoroacetic acid (TFA)/ethanedithiol (EDT)/H₂O = 92.5/5/2.5) and diethyl ether, and centrifuged to give crude Hexanoyl-D-Cys-Pro-Pro-Thr-Gln-1Nal-betahomoCys-OH.

**Step 2:** 500 mg (1 eq.) of the crude product obtained in step 1 was dissolved in 100 mL of acetonitrile and water (with a volume ratio of 1/1), then a solution of 185 mg (1 eq.) of 1,3,5-tris (bromomethyl)benzene (TBMB) in 2 mL of acetonitrile was added, and 1 M (NH₄)₂CO₃ aqueous solution was added to adjust the pH of the reaction solution to alkaline. After 10 min of the reaction, a solution of 485 mg (2 eq.) of 2,2',2"-(10-(2-((2-thioethyl)amino)-2-oxoethyl)-1,4,7,10-tetraazacyclododecane-1,4,7-triyl) triacetic acid (DOTA-AET) in 2 mL of water was added, and then 1 M (NH₄)₂CO₃ aqueous solution was added to adjust the pH of the reaction solution to alkaline. The reaction was continued for 24 h, diluted by addition of 200 mL of water, purified by RP-HPLC (preparative column size: 1 inch; filler: Luna C18(3); mobile phase: phase A: 0.1% TFA in H₂O, phase B: 0.1% TFA in 80% acetonitrile in water; elution gradient: 35-65% phase B in 60 min; wavelength: 220 nm), and lyophilized to give the compound 78 Hexanoyl-(D-Cys(tMeBn(DOTA-AET))-Pro-Pro-Thr-Gln-1Nal-betahomoCys)-OH trifluoroacetate.

HPLC purity was 95.44%, MS (m/z): 1534.7 [M+H]⁺.

### Example 79

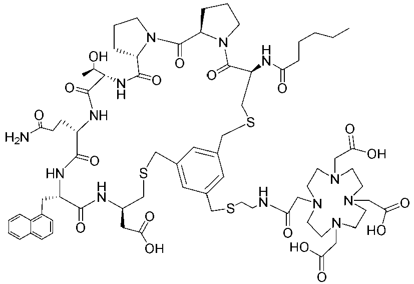

Compound 79 (Hexanoyl-(Cys(tMeBn(DOTA-AET))-D-Pro-Pro-Thr-Gln-1Nal-betahomoCys)-OH) was synthesized with reference to the synthesis method of compound 78. During synthesis, the A1 amino acid raw material was adjusted from S to R configuration, while the A2 amino acid raw material was adjusted from S to R configuration.

HPLC purity was 90.14%, MS (m/z): 1535.74 [M+H]⁺.

### Example 80

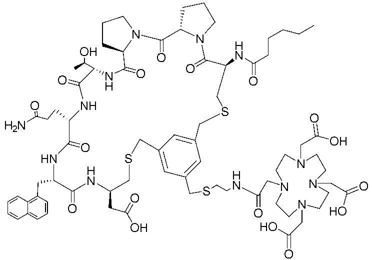

Compound 80 (Hexanoyl-(Cys(tMeBn(DOTA-AET))-Pro-D-Pro-Thr-Gln-1Nal-betahomoCys)-OH) was synthesized with reference to the synthesis method of compound 78. During synthesis, the A1 amino acid raw material was adjusted from S to R configuration, while the A3 amino acid raw material was adjusted from S to R configuration.

HPLC purity was 90.13%, MS (m/z): 1534.73 [M+H]⁺.

### Example 81

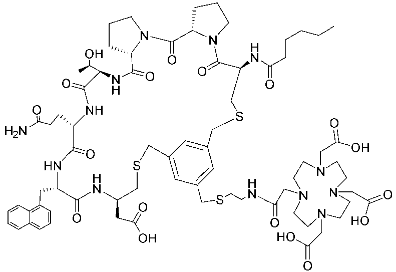

Compound 81 (Hexanoyl-(Cys(tMeBn(DOTA-AET))-Pro-Pro-D-Thr-Gln-1Nal-betahomoCys)-OH) was synthesized with reference to the synthesis method of compound 78. During synthesis, the A1 amino acid raw material was adjusted from S to R configuration, while the A4 amino acid raw material was adjusted from S to R configuration.

HPLC purity was 92.36%, MS (m/z): 1535.75 [M+H]⁺.

### Example 82

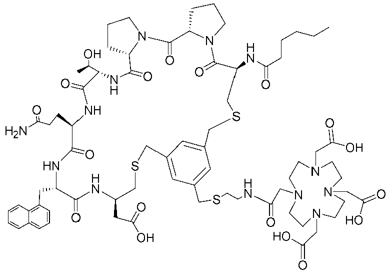

Compound 82 (Hexanoyl-(Cys(tMeBn(DOTA-AET))-Pro-Pro-Thr-D-Gln-1Nal-betahomoCys)-OH) was synthesized with reference to the synthesis method of compound 78. During synthesis, the A1 amino acid raw material was adjusted from S to R configuration, while the A5 amino acid raw material was adjusted from S to R configuration.

HPLC purity was 94.36%, MS (m/z): 1534.75 [M+H]⁺.

### Example 83

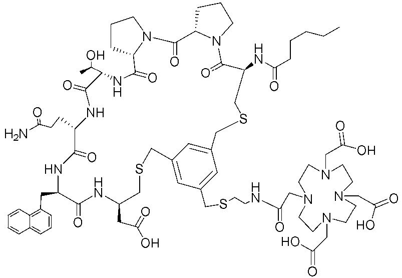

Compound 83 (Hexanoyl-(Cys(tMeBn(DOTA-AET))-Pro-Pro-Thr-Gln-D-1Nal-betahomoCys)-OH) was synthesized with reference to the synthesis method of compound 78. During synthesis, the A1 amino acid raw material was adjusted from S to R configuration, while the A6 amino acid raw material was adjusted from S to R configuration.

HPLC purity was 94.76%, MS (m/z): 1534.66 [M+H]⁺.

### Example 84

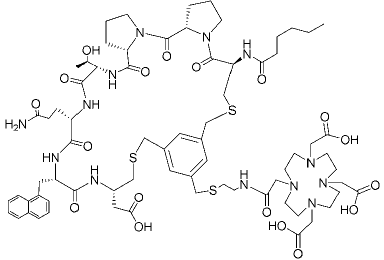

Compound 84 (Hexanoyl-(Cys(tMeBn(DOTA-AET))-Pro-Pro-Thr-Gln-1Nal-D-betahomoCys)-OH) was synthesized with reference to the synthesis method of compound 78. During synthesis, only the A1 amino acid raw material was adjusted from S to R configuration, while the A3 amino acid raw material was adjusted from R to S configuration.

HPLC purity was 90%, MS (m/z): 1534.73 [M+H]⁺.

Abbreviation of structural formula of compound

| Abbreviation | Structure |
|---|---|
| DBMB | 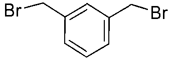 |
| Beta-homo-Cys | 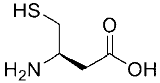 |
| Cha | 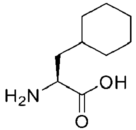 |
| TBMB | 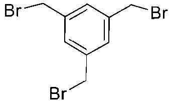 |
| DOTA-NHS | 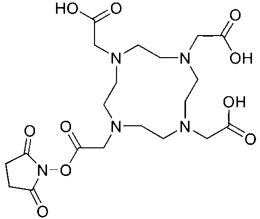 |
| AET | 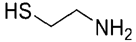 |
| 1Nal | 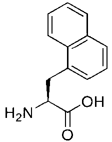 |
| Phe(2-CF3) | 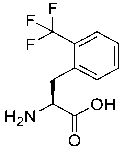 |
| 2Nal | 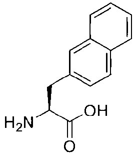 |
| Hex | 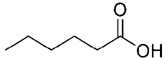 |

The human recombinant FAP protein (Acrobiosystems, FAP-H82Q6) was diluted to a 125 pM FAP working solution with an assay buffer (50 mM Tris-HCl, 1 M NaCl, 1 mg/mL BSA, pH 7.5), and the FAP substrate (Z-Gly-Pro-AMC (LEONBIO, 68542-93-8)) was diluted to a 150 µM substrate working solution with the assay buffer. 40 µl of FAP working solution and 20 µl of compounds at different concentrations (4-fold dilution) were taken, then 40 µl of substrate working solution was added, and the mixture was uniformly mixed on a shaker at room temperature for 1 min. The uniformly mixed mixture was reacted at room temperature in the dark for 90 min. The fluorescence value Ex = 380 nm/Em = 460 nm was read by using a microplate reader. The original data was saved, and the IC₅₀ value of the compound was obtained by using the original data or the Inhibition% via calculation and analyzing the data with Graphpad PRISM. When the compound has a IC₅₀ ≤ 100 nM, it is indicated that the compound has a significant inhibitory activity against FAP protease activity and meets the protease activity index of the candidate compound.

3BP-4186 and 3BP-3554 are compounds in CN 114341158 A and have the following structures:

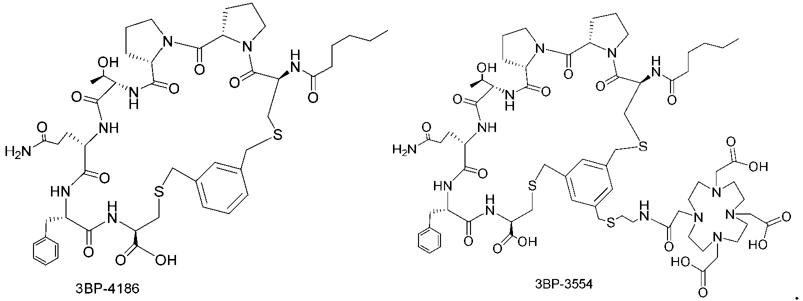

| Compound No. | FAP IC₅₀ (nM) | Compound No. | FAP IC₅₀ (nM) |
|---|---|---|---|
| 3BP-4186 | 98.82 | Compound 40 | 11.4 |
| Compound 1 | 2.91 | Compound 42 | 16.2 |
| Compound 2 | 4.24 | Compound 43 | 3.11 |
| Compound 3 | 25.06 | Compound 44 | 2.6 |
| Compound 4 | 11.3 | Compound 45 | 10.8 |
| Compound 5 | 1.8 | Compound 46 | 15.6 |
| Compound 6 | 7.25 | Compound 47 | 2.9 |
| Compound 31 | 1.61 | Compound 48 | 35.2 |
| Compound 32 | 3.35 | Compound 49 | 4.98 |
| Compound 33 | 2.9 | Compound 50 | 14.4 |
| Compound 34 | 5.92 | Compound 51 | 2.73 |
| Compound 35 | 6.62 | Compound 52 | 3.93 |
| Compound 36 | 4.18 | Compound 53 | 5.78 |
| Compound 37 | 4.67 | Compound 54 | 7.05 |
| Compound 38 | 4.12 | Compound 58 | 2.8 |
| Compound 39 | 5.31 | Compound 59 | 11.5 |

The results showed that the compounds of the present disclosure that are not linked with a chelating agent have a stronger inhibitory activity against FAP protease activity than 3BP-4186.

| Compound No. | FAP IC₅₀ (nM) | Compound No. | FAP IC₅₀ (nM) |
|---|---|---|---|
| 3BP-3554 | 1.8 | Compound 13 | 0.97 |
| Compound 7 | 1.53 | Compound 14 | 0.28 |
| Compound 8 | 1.18 | Compound 15 | 0.67 |
| Compound 10 | 0.23 | Compound 16 | 0.41 |
| Compound 11 | 0.26 | Compound 19 | 0.98 |
| Compound 12 | 0.46 | | |

The results showed that the compounds of the present disclosure that are linked with a chelating agent have a stronger inhibitory activity against FAP protease activity than 3BP-3554.

| Compound No. | FAP IC₅₀ (nM) | Compound No. | FAP IC₅₀ (nM) |
|---|---|---|---|
| Compound 78 | 14.75 | Compound 82 | 209.3 |
| Compound 79 | > 5000 | Compound 83 | 1042 |
| Compound 80 | 566.4 | Compound 84 | 6.70 |
| Compound 81 | 70.58 | | |

The results showed that among compounds 78-84 formed by replacing 7 amino acids of compound 11 with enantiomers, compounds 78, 81 and 84 still showed good inhibitory activity against FAP protease activity.

### Effect example 2 Test of inhibitory activity of compounds against DPP4 protease activity

The human recombinant DPP4 protein (Acrobiosystems, DP4-H5211) was formulated into a 125 pM working solution with an assay buffer (20 mM Tris-HCl, 100 mM NaCl), and the DPP4 substrate (H-Gly-Pro-AMC.HBr (LEONBIO, 115035-46-6)) was diluted to a 250 µM DPP4 substrate working solution with the assay buffer. 40 µl of DPP4 working solution and 20 µl of compounds at different concentrations (4-fold dilution) were taken, then 40 µl of DPP4 substrate working solution was added, and the mixture was uniformly mixed on a shaker at room temperature for 1 min. The uniformly mixed mixture was reacted at room temperature in the dark for 90 min. Reading was made by microplate reader at excitation and emission wavelengths of 380 nm and 460 nm. The original data was saved, and the IC₅₀ value of the compound was obtained by analyzing the data with Graphpad PRISM.

| Compound No. | DPP4 IC50 (µM) | Compound No. | DPP4 IC50 (µM) |
|---|---|---|---|
| 3BP-4186 | > 10 | Compound 40 | > 10 |
| Compound 1 | > 10 | Compound 42 | > 10 |
| Compound 2 | > 10 | Compound 43 | > 10 |
| Compound 3 | > 10 | Compound 44 | > 10 |
| Compound 5 | > 10 | Compound 46 | > 10 |
| Compound 6 | > 10 | Compound 47 | > 10 |
| Compound 31 | > 10 | Compound 48 | > 10 |
| Compound 32 | > 10 | Compound 49 | > 10 |
| Compound 33 | > 10 | Compound 50 | > 10 |
| Compound 34 | > 10 | Compound 51 | > 10 |
| Compound 35 | > 10 | Compound 52 | > 10 |
| Compound 36 | > 10 | Compound 53 | >10 |
| Compound 37 | > 10 | Compound 54 | > 10 |
| Compound 38 | > 10 | Compound 58 | >10 |
| Compound 39 | > 10 | | |

The results showed that the compounds of the present disclosure that are not linked with a chelating agent have an inhibitory activity against DPP4 protease activity comparable to that of 3BP-4186.

| Compound No. | DPP4 IC50 (µM) | Compound No. | DPP4 IC50 (µM) |
|---|---|---|---|
| 3BP-3554 | > 10 | Compound 13 | > 10 |
| Compound 7 | > 10 | Compound 14 | > 10 |
| Compound 8 | > 10 | Compound 15 | > 10 |
| Compound 10 | > 10 | Compound 16 | > 10 |
| Compound 11 | > 10 | Compound 19 | > 10 |
| Compound 12 | > 10 | | |

The results showed that the compounds of the present disclosure that are linked with a chelating agent have an inhibitory activity against DPP4 protease activity comparable to that of 3BP-3554.

### Effect example 3 Test of inhibitory activity of compounds against PERP protease activity

The human recombinant PREP protein (R&D systems, 4308-SE-010) was formulated into a 125 pM working solution with a assay buffer (25 mM Tris-HCl, 250 mM NaCl, 2.5 mM DTT, 1 mg/mL BSA), and the PREP substrate (Z-Gly-Pro-AMC (LEONBIO, 68542-93-8)) was diluted to a 125 µM PREP substrate working solution with the assay buffer. 40 µl of PREP working solution and 20 µl of compounds at different concentrations (4-fold dilution) were taken, then 40 µl of PREP substrate working solution was added, and the mixture was uniformly mixed on a shaker at room temperature for 1 min. The uniformly mixed mixture was reacted at room temperature in the dark for 90 min. Reading was made by microplate reader at excitation and emission wavelengths of 380 nm and 460 nm. The original data was saved, and the IC₅₀ value of the compound was obtained by analyzing the data with Graphpad PRISM.

The ratio of the IC₅₀ value of inhibitory activity of a compound on PREP protease activity to the IC₅₀ value of inhibitory activity of the compound on FAP protease activity reflects the selectivity of the compound to PREP protease activity: A > 10000, 10000 > B > 3300, 3300 > C > 1000, 1000 > D > 330, 330 > E > 100, and 100 > F.

| Compound No. | PREP/FAP | Compound No. | PREP/FAP |
|---|---|---|---|
| 3BP-4186 | F | Compound 44 | C |
| Compound 4 | D | Compound 45 | D |
| Compound 31 | C | Compound 46 | D |
| Compound 32 | D | Compound 47 | E |
| Compound 33 | D | Compound 48 | D |
| Compound 34 | E | Compound 49 | E |
| Compound 35 | D | Compound 50 | C |
| Compound 36 | E | Compound 51 | D |
| Compound 37 | D | Compound 52 | D |
| Compound 38 | D | Compound 53 | D |
| Compound 39 | D | Compound 54 | E |
| Compound 40 | D | Compound 57 | E |
| Compound 42 | E | Compound 58 | E |
| Compound 43 | D | Compound 59 | E |

The results showed that the compounds of the present disclosure that are not linked with a chelating agent have a higher selectivity for PREP than 3BP-4186.

| Compound No. | PREP/FAP | Compound No. | PREP/FAP |
|---|---|---|---|
| 3BP-3554 | C | Compound 12 | A |
| Compound 8 | B | Compound 14 | B |
| Compound 10 | A | Compound 15 | B |
| Compound 11 | A | Compound 16 | B |

The results showed that the compounds of the present disclosure that are linked with a chelating agent have a higher selectivity for PREP than 3BP-3554.

### Effect Example 4 Plasma stability experiment of compounds in mice

The plasma stability experiment was performed using plasma from CD-1 mice. The compounds were diluted to a concentration of 1 mM with methanol. To 0.792 mL of plasma, 8.00 µL of 1 mM of each test compound was added, and 50.0 µL of the plasma sample was taken after uniform mixing. The remaining sample was placed in a 37°C water bath and incubated for 24 h. Then 50.0 µL of the plasma sample was taken, and 10 µL of an internal standard (100 µM 3BP-3554 as the internal standard of compound 13, and 100 µM compound 13 as the internal standard of other test compounds) was added to the above samples, respectively, and uniformly mixed. After centrifugation, the sample was diluted 1-fold with 0.2% trifluoroacetic acid and analyzed by LC-MS/MS.

| Compound No. | 24 h remaining amount (%) |
|---|---|
| 3BP-3554 | 87 |
| Compound 11 | 94 |
| Compound 12 | 94 |
| Compound 13 | 100 |
| Compound 15 | 93 |
| Compound 16 | 96 |

The results showed that the compounds of the present disclosure have a higher plasma stability in mice than 3BP-3554.

### Effect Example 5 Pharmacokinetics of compounds

Male CD-1 mice weighing 30-35 g were selected, and a test compound was injected into the tail vein at 0.2 mg/kg. Plasma samples were collected at 5, 15, 30, and 60 min and 2, 4, 8, and 24 h after administration and processed accordingly. The drug content was analyzed by LC-MS/MS.

Among them, compounds 20, 21 and 22 were compounds formed by chelating nonradioactive in with compounds 11, 14 and 16, respectively.

| Parameter | 3BP-3623 | Compound 20 | Compound 21 | Compound 22 |
|---|---|---|---|---|
| Cₘₐₓ (ng/mL) | 594 | 772 | 1010 | 1120 |
| T_{1/2} (h) | 0.37 | 1.63 | 1.26 | 7.93 |
| AUC₀₋ₜ(h*ng/mL) | 195 | 308 | 634 | 740 |
| Vss (L/kg) | 0.24 | 0.52 | 0.22 | 1.18 |
| Cl (L/h/kg) | 1.02 | 0.61 | 0.31 | 0.26 |

The results showed that the half-life, AUC and clearance rate of the compounds of the present disclosure were better than those of 3BP-3623.

3BP-3623 is a compound in CN 114341158 A and has the following structure:

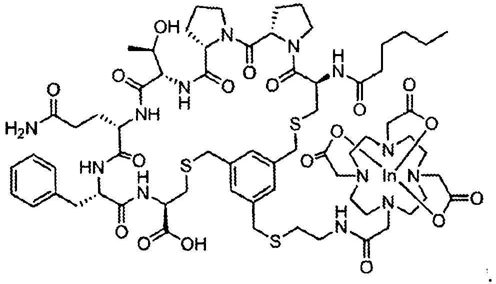

### Effect Example 6 Experiment on distribution of compounds in tissues

Male SCID mice (6-8 weeks old) were subcutaneously inoculated with 5 × 10⁶ HEK293-FAP cells (FAP was highly expressed in HEK293 cells). 10 days later, 30 mice with tumor size of 200-300 mm³ were randomly divided into 3 groups. Each mouse in the 3 groups was administered with compound ¹⁷⁷Lu-3BP-3554, compound 29 and compound 30 with a radiation dose of 100 µCi, respectively. Blood, kidney and tumor tissues were collected at time points of 4 h, 8 h, 24 h, 72 h and 168 h, and all blood samples and tissue samples were measured for radioactive counts by gamma counter.

| **Compound** | **Time (h)** | **Tumor (ID %/g)** | **Kidney (ID %/g)** | **Blood (ID %/g)** | **Tumor/kid ney** | **Tumor/blo od** |
|---|---|---|---|---|---|---|
| ¹⁷⁷Lu-3BP-3554 | 4 | 21.21 | 6.65 | 0.07 | 3.23 | 290.35 |
| | 8 | 18.42 | 6.37 | 0.02 | 2.88 | 1180.18 |
| | 24 | 10.37 | 2.92 | 0.01 | 3.55 | 731.53 |
| | 72 | 5.05 | 0.86 | 0.0038 | 5.75 | 1330 |
| | 168 | 1.52 | 0.16 | 0.0091 | 9.44 | 167.79 |
| Compound 29 | 4 | 25.62 | 11.06 | 0.99 | 2.32 | 26.16 |
| | 8 | 27.45 | 8.63 | 0.39 | 3.18 | 70.12 |
| | 24 | 22.70 | 5.69 | 0.03 | 3.99 | 1634.37 |
| | 72 | 19.21 | 2.43 | 0.0079 | 7.12 | 2405.35 |
| | 168 | 13.11 | 0.47 | 0.011 | 27.58 | 1161.77 |
| Compound 30 | 4 | 34.29 | 4.98 | 2.11 | 6.88 | 16.27 |
| | 8 | 30.94 | 5.57 | 0.98 | 5.56 | 32.32 |
| | 24 | 21.64 | 3.64 | 0.10 | 5.95 | 212.10 |
| | 72 | 16.79 | 2.20 | 0.0151 | 6.98 | 1103.52 |
| | 168 | 8.81 | 0.65 | 0.018 | 11.85 | 483.67 |
| Note | Compounds 29 and 30 were compounds formed by chelating radioactive ¹⁷⁷Lu with compounds 11 and 16, respectively. | | | | | |

The results showed that the injection dose%/g tissue of compounds 29 and 30 at different time points were significantly higher than that of ¹⁷⁷Lu-3BP-3554, indicating that compounds 29 and 30 are more highly enriched and persist longer in tumor tissues compared with ¹⁷⁷Lu-3BP-3554. Compounds 29 and 30 also had better tissue selectivity for kidney and blood tissues than ¹⁷⁷Lu-3BP-3554; therefore, compounds 29 and 30 had better safety compared with ¹⁷⁷Lu-3BP-3554.

¹⁷⁷Lu-3BP-3554 is a compound in CN 114341158 A and has the following structure:

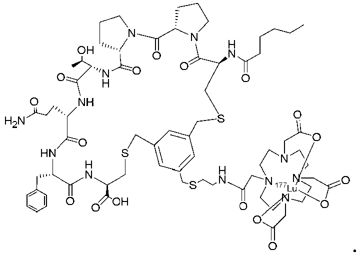

### Effect Example 7 Compound efficacy experiment

The stable transgenic cell line HEK-293-FAP with high expression of FAP was constructed and 1 × 10⁶ HEK-293-FAP cells were injected subcutaneously to establish an NOG tumor-bearing mouse model, which was used in the experiment when the tumor grew to about 300 mm³. The tumor-bearing mice were randomly divided into 4 groups: control group, compound 29 0.4 mCi group (0.4 mCi/mouse), compound 29 0.8 mCi group (0.8 mCi/mouse) and ¹⁷⁷Lu-3BP-3554 0.8 mCi group (0.8 mCi/mouse), with 6 mice in each group. On day 0 of the experiment, each group of mice was injected with 0.1 ml of a compound solution according to the dosage, and the volume of tumor was measured on days 2, 4, 7, 11, 14, 17, and 21 of the experiment. When the volume of tumor was greater than 2000 mm³, the tumor-bearing mice were euthanized.

The experimental results showed: compounds 29 and ¹⁷⁷Lu-3BP-3554 could significantly inhibit tumor growth. The inhibitory effect of compound 29 on tumors had a dose-response relationship, and the inhibitory effect of the 0.8 mCi group on tumors was better than that of the 0.4 mCi group. Compared with positive molecule ¹⁷⁷Lu-3BP-3554, on the 21st day of the experiment, the inhibitory effects of both compound 29 0.8 mCi group and 0.4 mCi group on tumors were better than those of ¹⁷⁷Lu-3BP-3554 0.8 mCi group.

## Claims

1. A polypeptide compound, a pharmaceutically acceptable salt thereof, a solvate thereof or a solvate of a pharmaceutically acceptable salt thereof, wherein the polypeptide compound is a compound represented by formula I; wherein A₁, A₂, A₃, A₄, A₅, A₆ and A₇ are sequentially connected via a peptide bond (-CO-NH-); 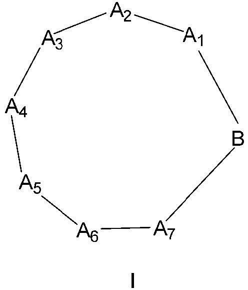
A₇ has a structure represented by formula a-7, 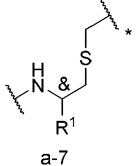
"&" denotes that the carbon atom herein is a chiral carbon, that is, 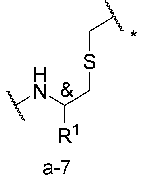 is 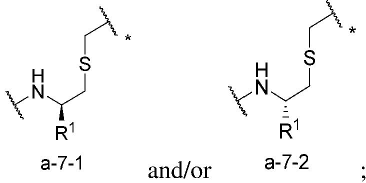 "*" end denotes attachment to B;
R¹ is -(CH₂)ₘM;
m is 1, 2, 3, 4, 5 or 6;
M is -COR¹⁻¹ or -NR¹⁻²R¹⁻³;
R¹⁻¹ is -OH, -NR^{a}R^{b}, -OC₁₋₁₂alkyl, -OC₃₋₁₂cycloalkyl, -OC₆₋₁₀oaryl, -O-5-10-membered heteroaryl, a group formed by the loss of a H atom by an amino group on an amino acid, a group formed by the loss of a H atom by an amino group on a peptide formed by 2 or more amino acids, -OC₁₋₁₂alkyl substituted with 1, 2 or 3 R^{e}, -OC₃₋₁₂cycloalkyl substituted with 1, 2 or 3 R^{f}, -OC₆₋₁₀aryl substituted with 1, 2 or 3 R^{e-1}, or -O-5-10-membered heteroaryl substituted with 1, 2 or 3 R^{f-1};
R^{a} and R^{b} are independently H, C₁₋₁₂alkyl, C₃₋₁₂cycloalkyl, C₆₋₁₀aryl, 5-10-membered heteroaryl, C₁₋₁₂alkyl substituted with 1, 2 or 3 R^{b-1}, C₃₋₁₂cycloalkyl substituted with 1, 2 or 3 R^{b-2}, C₆₋₁₀aryl substituted with 1, 2 or 3 R^{b-3}, or 5-10-membered heteroaryl substituted with 1, 2 or 3 R^{b-4};
R^{b-1}, R^{b-2}, R^{b-3} and R^{b-4} are independently deuterium, -OH, -NH₂, -COOH, -CONH₂, -CN, halogen, C₁₋₁₂alkyl, C₃₋₁₂cycloalkyl, C₆₋₁₀aryl, 5-10-membered heteroaryl, C₆₋₁₀aryl substituted with 1, 2 or 3 R^{b-1-1}, 5-10-membered heteroaryl substituted with 1, 2 or 3 R ^{b-1-2} C₃₋₁₂cycloalkyl substituted with 1, 2 or 3 R^{b-1-3}, or C₁₋₁₂alkyl substituted with 1, 2 or 3 R^{b-1-4}.
R¹¹ R^{b-1-2}, R^{b-1-3} and R^{b-1-4} are independently C₁₋₁₂alkyl, halogen, or C₁₋₁₂alkyl substituted with 1, 2 or 3 halogen;
R^{e}, R^{f}, R^{e-1} and R^{f-l} are independently halogen, -OH or -NH₂;
R¹⁻² and R¹⁻³ are independently H, C₁₋₁₂alkyl, C₃₋₁₂cycloalkyl, C₆₋₁₀aryl, 5-10-membered heteroaryl, -COR^{g}, a group formed by the loss of a -OH group by a carboxyl group on an amino acid, a group formed by the loss of a -OH group by a carboxyl group on a peptide formed by 2 or more amino acids, C₁₋₁₂alkyl substituted with 1, 2 or 3 R^{h}, C₃₋₁₂cycloalkyl substituted with 1, 2 or 3 R^{h-1}, C₆₋₁₀aryl substituted with 1, 2 or 3 R^{h-2}, or 5-10-membered heteroaryl substituted with 1, 2 or 3 R^{h-3};
R^{g} is independently C₁₋₁₂alkyl, C₃₋₁₂cycloalkyl, C₆₋₁₀aryl, 5-10-membered heteroaryl, C₁₋₁₂alkyl substituted with 1, 2 or 3 R^{g-1}, C₃₋₁₂cycloalkyl substituted with 1, 2 or 3 R^{g-2}, C₆₋₁₀aryl substituted with 1, 2 or 3 R^{g-3}, or 5-10-membered heteroaryl substituted with 1, 2 or 3 R^{g-4};
R^{g-1}, R^{g-2}, R^{g-3} and R^{g-4} are independently deuterium, -OH, -NH₂, -COOH, -CN, halogen, C₁₋₁₂alkyl, C₃₋₁₂cycloalkyl, C₆₋₁₀aryl, or C₆₋₁₀aryl substituted with 1, 2 or 3 R^{g-1-1};
R^{g-1-1} is independently -OH, -NH₂, -COOH, -CN, halogen, C₁₋₁₂alkyl, or C₁₋₁₂alkyl substituted with 1, 2 or 3 halogen;
R^{h}, R^{h-1}, R^{h-2} and R^{h-3} are independently deuterium, -COOH, -CN, halogen, -OH, or -NH₂;
B is 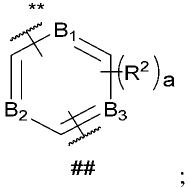 B₁, B₂ and B₃ are independently N or C;
a is 1, 2 or 3;
R² is independently H, C₁₋₁₂alkyl or halogen;
"**" end denotes attachment to A₇; "##" end denotes attachment to A₁;
A₁ has a structure represented by formula a-1, 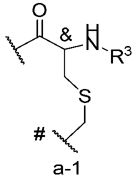
"&" denotes that the carbon atom herein is a chiral carbon, that is, 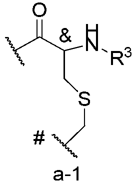
is 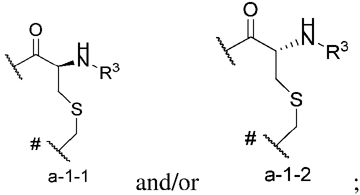
"#" end denotes attachment to B;
wherein R³ is -COC₁₋₁₂alkyl, -COOC₁₋₁₂alkyl, -COC₁₋₁₂alkyl substituted with 1, 2 or 3 R³⁻¹, - COOC₁₋₁₂alkyl substituted with 1, 2 or 3 R³⁻², a group formed by the loss of a -OH group by a carboxyl group on an amino acid, or a group formed by the loss of a -OH group by a carboxyl group on a peptide formed by 2 or more amino acids;
R³⁻¹ and R³⁻² are independently deuterium, -NH₂, -COOH, -CN, halogen, -OH, -NR³⁻¹⁻¹R³⁻¹⁻², or -OC₁₋₁₂alkyl;
R³⁻¹⁻¹ and R³⁻¹⁻² are independently H or C₁₋₁₂alkyl;
A₂ has a structure represented by formula a-2 or a-2-2, the nitrogen end of which is attached to A₁, and the carbonyl end of which is attached to A₃; 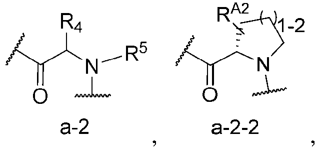
R⁴ and R⁵ are independently H, C₁₋₁₂alkyl or C₁₋₁₂alkyl substituted with 1, 2 or 3 R⁴⁻¹;
R⁴⁻¹ is independently deuterium, -OH, halogen, -NH₂, or -COOH;
R^{A2} is independently H, deuterium, -CH₃, -OH, -NH₂, or F; the number of R^{A2} is 1, 2, 3 or 4; A₃ has a structure represented by formula a-3, the nitrogen end of which is attached to A₂, and the carbonyl end of which is attached to A₄; 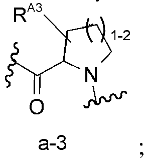
R^{A3} is H, deuterium, -CH₃, -OH, -NH₂ or F; the number of R^{A3} is 1, 2, 3 or 4;
A₄ has a structure represented by formula a-4, the nitrogen end of which is attached to A₃, and the carbonyl end of which is attached to A₅; 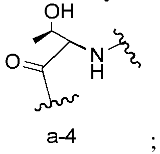
As has a structure represented by formula a-5, the nitrogen end of which is attached to A₄, and the carbonyl end of which is attached to A₆; 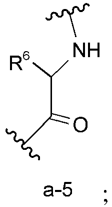
wherein R⁶ is -(CH₂)ₓCOR⁶⁻¹, in which x is 1, 2 or 3, and R⁶⁻¹ is -OH or -NH₂;
A₆ has a structure represented by formula a-6, the nitrogen end of which is attached to A₅, and the carbonyl end of which is attached to A₇; 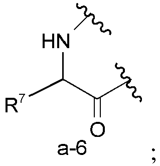
wherein R⁷ is -CH₂C₆₋₁₀aryl, -CH₂-5-10-membered heteroaryl, -CH₂C₃₋₁₂cycloalkyl, -CH₂C₆₋₁₀aryl substituted with 1, 2 or 3 R⁷⁻¹, -CH₂-5-10-membered heteroaryl substituted with 1, 2 or 3 R⁷⁻², or -CH₂C₃₋₁₂cycloalkyl substituted with 1, 2 or 3 R⁷⁻³;
R⁷⁻¹, R⁷⁻² and R⁷⁻³ are independently deuterium, -OH, -NH₂, halogen, -CN, C₁₋₁₂alkyl, or C₁₋₁₂alkyl substituted with 1, 2 or 3 halogen;
in the 5-10-membered heteroaryl, the heteroatom in each 5-10-membered heteroaryl is independently selected from one or more of N, O and S, and the number of the heteroatom is independently 1, 2 or 3.

2. The polypeptide compound of claim 1, a pharmaceutically acceptable salt thereof, a solvate thereof or a solvate of a pharmaceutically acceptable salt thereof, wherein the polypeptide compound satisfies one or more of the following:
(1) 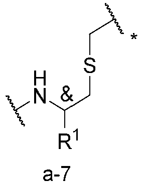 is 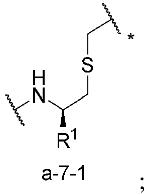
(2) m is 1 or 2;
(3) R¹⁻¹ is -OH, -NR^{a}R^{b}, -OC₁₋₁₂alkyl, -OC₃₋₁₂cycloalkyl, -OC₆₋₁₀aryl, a group formed by the loss of a H atom by an amino group on an amino acid, or a group formed by the loss of a H atom by an amino group on a peptide formed by 2 or more amino acids;
(4) R^{a} and R^{b} are independently H, C₁₋₁₂alkyl, C₃₋₁₂cycloalkyl, C₆₋₁₀aryl, 5-10-membered heteroaryl, or C₁₋₁₂alkyl substituted with 1, 2 or 3 R^{b-1}, C₆₋₁₀aryl substituted with 1, 2 or 3 R^{b-3};
(5) R^{b-1} and R^{b-3} are independently deuterium, -OH, -NH₂, -COOH, -CONH₂, -CN, halogen, C₃₋₁₂cycloalkyl, C₆₋₁₀aryl, 5-10-membered heteroaryl, or C₆₋₁₀aryl substituted with 1, 2 or 3 R^{b-1-1};
(6) R^{b-1-1} is independently C₁₋₁₂alkyl substituted with 1, 2 or 3 halogen;
(7) R¹⁻² and R¹⁻³ are independently H, C₁₋₁₂alkyl, -COR^{g}, a group formed by the loss of a - OH group by a carboxyl group on an amino acid, or a group formed by the loss of a -OH group by a carboxyl group on a peptide formed by 2 or more amino acids;
(8) R^{g} is independently C₁₋₁₂alkyl, C₃₋₁₂cycloalkyl, C₆₋₁₀aryl, 5-10-membered heteroaryl, C₁₋₁₂alkyl substituted with 1, 2 or 3 R^{g-1}, C₆₋₁₀aryl substituted with 1, 2 or 3 R^{g-3}, or 5-10-membered heteroaryl substituted with 1, 2 or 3 R^{g-4};
(9) R^{g-1}, R^{g-3} and R^{g-4} are independently -OH, -NH₂, halogen, C₁₋₁₂alkyl, C₆₋₁₀aryl, or C₆₋₁₀aryl substituted with 1, 2 or 3 R^{g-1-1};
(10) R^{g-1-1} is independently C₁₋₁₂alkyl substituted with 1, 2 or 3 halogen;
(11) R³ is -COC₁₋₁₂alkyl, -COC₁₋₁₂alkyl substituted with 1, 2 or 3 R³⁻¹, a group formed by the loss of a -OH group by a carboxyl group on an amino acid, or a group formed by the loss of a -OH group by a carboxyl group on a peptide formed by 2 or more amino acids;
(12) R³⁻¹ is independently -NH₂ or -OC₁₋₁₂alkyl;
(13) R⁴ and R⁵ are independently C₁₋₁₂alkyl; or the nitrogen-containing heterocycle in the structure represented by formula a-2-2 is a five-membered ring;
(14) x is 2 or 3;
*(15)* R⁷ is -CH₂C₆₋₁₀aryl, -CH₂C₆₋₁₀heteroaryl, -CH₂C₃₋₁₂cycloalkyl, -CH₂C₆₋₁₀aryl substituted with 1, 2 or 3 R⁷⁻¹, or -CH₂-5-10-membered heteroaryl substituted with 1, 2 or 3 R⁷⁻²; and
(16) R⁷⁻¹ and R⁷⁻² are independently deuterium, -OH, -NH₂, halogen, C₁₋₃alkyl, or C₁₋₁₂alkyl substituted with 1, 2 or 3 halogen.

3. The polypeptide compound of claim 1 or 2, a pharmaceutically acceptable salt thereof, a solvate thereof or a solvate of a pharmaceutically acceptable salt thereof, wherein the polypeptide compound satisfies one or more of the following:
(1) R¹⁻¹ is -OH, -NR^{a}R^{b}, -OC₁₋₁₂alkyl, -OC₃₋₁₂cycloalkyl, a group formed by the loss of a H atom by an amino group on an amino acid, or a group formed by the loss of a H atom by an amino group on a peptide formed by 2 or more amino acids;
(2) R^{b-1} is independently C₃₋₁₂cycloalkyl, C₆₋₁₀aryl, 5-10-membered heteroaryl, or C₆₋₁₀aryl substituted with 1, 2 or 3 R^{b-1-1};
(3) R^{g} is independently C₁₋₁₂alkyl, C₃₋₁₂cycloalkyl, C₆₋₁₀aryl, 5-10-membered heteroaryl, C₁₋₁₂alkyl substituted with 1, 2 or 3 R^{g-1}, or C₆₋₁₀aryl substituted with 1, 2 or 3 R^{g-3};
(4) R^{g-1} and R^{g-3} are independently halogen, C₆₋₁₀aryl, or C₆₋₁₀aryl substituted with 1, 2 or 3 R^{g-l-l};
(5) R⁷ is -CH₂C₆₋₁₀aryl, -CH₂C₃₋₁₂cycloalkyl, or -CH₂C₆₋₁₀aryl substituted with 1, 2 or 3 R⁷⁻¹; and
(6) R⁷⁻¹ is independently C₁₋₁₂alkyl substituted with 1, 2 or 3 halogen.

4. The polypeptide compound of claim 1 or 2, a pharmaceutically acceptable salt thereof, a solvate thereof or a solvate of a pharmaceutically acceptable salt thereof, wherein 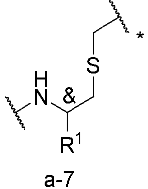 is 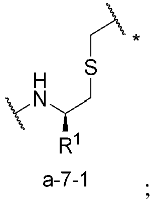
m is 1 or 2;
R¹⁻¹ is -OH, -NR^{a}R^{b}, -OC₁₋₁₂alkyl, -OC₃₋₁₂cycloalkyl, a group formed by the loss of a H atom by an amino group on an amino acid, or a group formed by the loss of a H atom by an amino group on a peptide formed by 2 or more amino acids;
R^{a} and R^{b} are independently H, C₁₋₁₂alkyl, C₃₋₁₂cycloalkyl, C₆₋₁₀aryl, or C₁₋₁₂alkyl substituted with 1, 2 or 3 R^{b-l};
R^{b-1} is independently C₃₋₁₂cycloalkyl, C₆₋₁₀aryl, 5-10-membered heteroaryl, or C₆₋₁₀aryl substituted with 1, 2 or 3 R^{b-1-1};
R^{b-1-l} is independently C₁₋₁₂alkyl substituted with 1, 2 or 3 halogen;
R¹⁻² and R¹⁻³ are independently H, C₁₋₁₂alkyl, -COR^{g}, a group formed by the loss of a -OH group by a carboxyl group on an amino acid, or a group formed by the loss of a -OH group by a carboxyl group on a peptide formed by 2 or more amino acids;
R^{g} is independently C₁₋₁₂alkyl, C₃₋₁₂cycloalkyl, C₆₋₁₀aryl, 5-10-membered heteroaryl, C₁₋₁₂alkyl substituted with 1, 2 or 3 R^{g-1}, or C₆₋₁₀aryl substituted with 1, 2 or 3 R^{g-3};
R^{g-1} and R^{g-3} are independently halogen, C₆₋₁₀aryl, or C₆₋₁₀aryl substituted with 1, 2 or 3 R^{g-1-1};
R^{g-l-l} is independently C₁₋₁₂alkyl substituted with 1, 2 or 3 halogen;
R³ is -COC₁₋₁₂alkyl, -COC₁₋₁₂alkyl substituted with 1, 2 or 3 R³⁻¹, a group formed by the loss of a -OH group by a carboxyl group on an amino acid, or a group formed by the loss of a -OH group by a carboxyl group on a peptide formed by 2 or more amino acids;
R³⁻¹ is independently -NH₂ or -OC₁₋₁₂alkyl;
R⁴ and R⁵ are independently C₁₋₁₂alkyl; or the nitrogen-containing heterocycle in formula a-2-2 is a five-membered ring, and R^{A2} is independently H, deuterium, -CH₃, -OH, -NH₂, or F; the number of R^{A2} is 1, 2 or 3;
x is 1 or 2;
R⁷ is -CH₂C₆₋₁₀aryl, -CH₂C₃₋₁₂cycloalkyl, or -CH₂C₆₋ioaryl substituted with 1, 2 or 3 R⁷⁻¹;
R⁷⁻¹ is independently C₁₋₁₂alkyl substituted with 1, 2 or 3 halogen.

5. The polypeptide compound of claim 1 or 2, a pharmaceutically acceptable salt thereof, a solvate thereof or a solvate of a pharmaceutically acceptable salt thereof, wherein the polypeptide compound satisfies one or more of the following:
(1) in R¹⁻¹, the C₁₋₁₂alkyl in the -OC₁₋₁₂alkyl is C₁₋₆alkyl;
(2) in R¹⁻¹, the C₃₋₁₂cycloalkyl in the -OC₃₋₁₂cycloalkyl is C₃₋₆cycloalkyl;
(3) in R¹⁻¹, the C₆₋₁₀aryl in the -OC₆₋₁₀aryl is phenyl or naphthyl;
(4) in R¹⁻¹, the group formed by the loss of a H atom by an amino group on an amino acid is 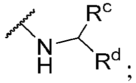
R^{c} is -(CH₂)ₒCOOH or -(CH₂)_{pCONH2};
o and p are independently 0, 1, 2, 3 or 4;
R^{d} is hydrogen, deuterium, C₁₋₁₂alkyl, C₃₋₁₂cycloalkyl, C₆₋₁₀aryl, 5-10-membered heteroaryl, C₁₋₁₂alkyl substituted with 1, 2 or 3 R^{d-1}, C₃₋₁₂cycloalkyl substituted with 1, 2 or 3 R^{d-2}, C₆₋₁₀aryl substituted with 1, 2 or 3 R^{d-3}, or 5-10-membered heteroaryl substituted with 1, 2 or 3 R^{d-4};
R^{d-1}, R^{d-2}, R^{d-3} and R^{d-4} are independently C₁₋₁₂alkyl, C₃₋₁₂cycloalkyl, or C₆₋₁₀aryl;
(5) in R¹⁻¹, the peptide formed by 2 or more amino acids is a peptide formed by 2 to 20 amino acids;
(6) in R^{a} and R^{b}, the C₁₋₁₂alkyl or the C₁₋₁₂alkyl in the C₁₋₁₂alkyl substituted with 1, 2 or 3 R^{b-1} is independently C₁₋₆alkyl;
(7) in R^{a} and R^{b}, the C₃₋₁₂cycloalkyl is independently C₃₋₆cycloalkyl;
(8) in R^{a} and R^{b}, the C₆₋₁₀aryl is independently phenyl, biphenyl or naphthyl;
(9) in R^{a} and R^{b}, the number of the heteroatom in the 5-10-membered heteroaryl is 1 or 2;
(10) in R^{b-1}, the C₃₋₁₂cycloalkyl is independently C₃₋₆cycloalkyl;
(11) in R^{b-1}, the C₆₋₁₀aryl or the C₆₋₁₀aryl in the C₆₋₁₀aryl substituted with 1, 2 or 3 R^{b-1-l} is independently phenyl or naphthyl;
(12) in R^{b-1}, the 5-10-membered heteroaryl is independently 5-6 membered heteroaryl;
(13) in R^{b-1}, the number of the heteroatom in the 5-10-membered heteroaryl is 1 or 2;
(14) in R^{b-l-1}, the halogen or the halogen in the C₁₋₁₂alkyl substituted with 1, 2 or 3 halogen is independently F, Cl, Br or **I,**
(15) in R^{b-l-1}, the C₁₋₁₂alkyl in the C₁₋₁₂alkyl substituted with 1, 2 or 3 halogen is independently C₁₋₆alkyl;
(16) in R¹⁻² and R¹⁻³, the C₁₋₁₂alkyl is independently C₁₋₆alkyl;
(17) in R¹⁻² and R¹⁻³, the C₃₋₁₂cycloalkyl or the C₃₋₁₂cycloalkyl in the C₃₋₁₂cycloalkyl substituted with 1, 2 or 3 R^{h-1} is independently C₃₋₆cycloalkyl;
(18) in R¹⁻² and R¹⁻³, the C₆₋₁₀aryl or the C₆₋₁₀aryl in the C₆₋₁₀aryl substituted with 1, 2 or 3 R^{h-2} is independently phenyl or naphthyl;
(19) in R¹⁻² and R¹⁻³, the number of the heteroatom in the 5-10-membered heteroaryl or the 5-10-membered heteroaryl in the 5-10-membered heteroaryl substituted with 1, 2 or 3 R^{h-3} is 1 or 2;
(20) in R¹⁻² and R¹⁻³, the peptide formed by 2 or more amino acids is a peptide formed by 2 to 20 amino acids;
(21) in R^{g}, the C₁₋₁₂alkyl or the C₁₋₁₂alkyl in the C₁₋₁₂alkyl substituted with 1, 2 or 3 R^{g-1} is independently C₁₋₆alkyl;
(22) in R^{g}, the C₃₋₁₂cycloalkyl is independently C₃₋₆cycloalkyl;
(23) in R^{g}, the C₆₋₁₀aryl or the C₆₋₁₀aryl in the C₆₋₁₀aryl substituted with 1, 2 or 3 R^{g-3} is independently phenyl or naphthyl;
(24) in R^{g}, the 5-10-membered heteroaryl or the 5-10-membered heteroaryl in the 5-10-membered heteroaryl substituted with 1, 2 or 3 R^{g-4} is 5-6-membered heteroaryl;
(25) in R^{g}, the number of the heteroatom in the 5-10-membered heteroaryl or the 5-10-membered heteroaryl in the 5-10-membered heteroaryl substituted with 1, 2 or 3 R^{g-4} is 1 or 2;
(26) in R^{g-1}, R^{g-3} and R^{g-4}, the halogen is independently F, Cl, Br or I;
(27) in R^{g-1}, R^{g-3} and R^{g-4}, the C₆₋₁₀aryl or the C₆₋₁₀aryl in the C₆₋₁₀aryl substituted with 1, 2 or 3 R^{g-1-1} is independently phenyl or naphthyl;
(28) in R^{g-1-1}, the C₁₋₁₂alkyl in the C₁₋₁₂alkyl substituted with 1, 2 or 3 halogen is independently C₁₋₆alkyl;
(29) in R^{g-1-1}, the halogen in the C₁₋₁₂alkyl substituted with 1, 2 or 3 halogen is independently F, Cl, Br or I;
(30) in R², the C₁₋₁₂alkyl is independently C₁₋₆alkyl;
(31) in R², the halogen is independently F, Cl, Br or I;
(32) in R³, the -COC₁₋₁₂alkyl or the C₁₋₁₂alkyl in the -COC₁₋₁₂alkyl substituted with 1, 2 or 3 R³⁻¹ is independently C₁₋₆alkyl;
(33) in R³, the peptide formed by 2 or more amino acids is a peptide formed by 2 to 20 amino acids;
(34) in R³⁻¹, the C₁₋₁₂alkyl in the -OC₁₋₁₂alkyl is independently C₁₋₆alkyl;
(35) in R⁴ and R⁵, the C₁₋₁₂alkyl is independently C₁₋₆alkyl;
(36) in R⁷, the C₃₋₁₂cycloalkyl in the -CH₂C₃₋₁₂cycloalkyl is independently C₃₋₆cycloalkyl;
(37) in R⁷, the -CH₂C₆₋₁₀aryl or the C₆₋₁₀aryl in the C₆₋₁₀aryl substituted with 1, 2 or 3 R⁷⁻¹ is independently phenyl or naphthyl;
(38) in R⁷, the 5-10-membered heteroaryl in the -CH₂-5-10-membered heteroaryl substituted with 1, 2 or 3 R⁷⁻² is 5-6-membered heteroaryl;
(39) in R⁷, the number of the heteroatom in the 5-10-membered heteroaryl in the -CH₂-5-10-membered heteroaryl substituted with 1, 2 or 3 R⁷⁻² is 1 or 2;
(40) in R⁷⁻¹, the halogen in the C₁₋₁₂alkyl substituted with 1, 2 or 3 halogen is independently F, Cl, Br or I;
(41) in R⁷⁻¹, the C₁₋₁₂alkyl in the C₁₋₁₂alkyl substituted with 1, 2 or 3 halogen is independently C₁₋₆alkyl; and
(42) B is

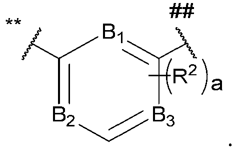

6. The polypeptide compound of claim 5, a pharmaceutically acceptable salt thereof, a solvate thereof or a solvate of a pharmaceutically acceptable salt thereof, wherein the polypeptide compound satisfies one or more of the following:
(1) in R¹⁻¹, the C₁₋₁₂alkyl in the -OC₁₋₁₂alkyl is n-propyl, isopropyl, n-butyl, isobutyl, or tert-butyl;
(2) in R¹⁻¹, the C₃₋₁₂cycloalkyl in the -OC₃₋₁₂cycloalkyl is cyclopentyl;
(3) o and p are independently 0 or 1;
(4) R^{d} is C₆₋₁₀aryl;
(5) in R¹⁻¹, the peptide formed by 2 or more amino acids is a peptide formed by 2 to 10 amino acids;
(6) in R^{a} and R^{b}, the C₁₋₁₂alkyl or the C₁₋₁₂alkyl in the C₁₋₁₂alkyl substituted with 1, 2 or 3 R^{b-1} is independently methyl, ethyl, n-propyl, isopropyl, n-butyl, isobutyl, tert-butyl, or n-pentyl;
(7) in R^{a} and R^{b}, the C₃₋₁₂cycloalkyl is independently cyclopropyl, cyclobutyl, cyclopentyl, cyclohexyl, or 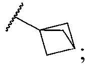
(8) in R^{a} and R^{b}, the C₆₋₁₀aryl is independently phenyl;
(9) in R^{a} and R^{b}, the 5-10-membered heteroaryl is furyl, thienyl, imidazolyl, pyridyl, or quinolinyl;
(10) in R^{b-1}, the C₃₋₁₂cycloalkyl is independently cyclopropyl, cyclobutyl, cyclopentyl, or cyclohexyl;
(11) in R^{b-1}, the C₆₋₁₀aryl or the C₆₋₁₀aryl in the C₆₋₁₀aryl substituted with 1, 2 or 3 R^{b-1-l} is independently phenyl;
(12) in R^{b-1}, the 5-10-membered heteroaryl is independently 5-6 membered heteroaryl;
(13) in R^{b-l-1}, the halogen or the halogen in the C₁₋₁₂alkyl substituted with 1, 2 or 3 halogen is F;
(14) in R^{b-l-1}, the C₁₋₁₂alkyl in the C₁₋₁₂alkyl substituted with 1, 2 or 3 halogen is independently methyl, ethyl, n-propyl, isopropyl, n-butyl, isobutyl, tert-butyl, or n-pentyl;
(15) in R¹⁻² and R¹⁻³, the C₁₋₁₂alkyl is independently methyl, ethyl, n-propyl, isopropyl, n-butyl, isobutyl, tert-butyl, n-pentyl, or n-hexyl;
(16) in R¹⁻² and R¹⁻³, the C₃₋₁₂cycloalkyl or the C₃₋₁₂cycloalkyl in the C₃₋₁₂cycloalkyl substituted with 1, 2 or 3 R^{h-1} is independently cyclopropyl, cyclobutyl, cyclopentyl, or cyclohexyl;
(17) in R¹⁻² and R¹⁻³, the peptide formed by 2 or more amino acids is a peptide formed by 2 to 10 amino acids;
(18) in R^{g}, the C₁₋₁₂alkyl or the C₁₋₁₂alkyl in the C₁₋₁₂alkyl substituted with 1, 2 or 3 R^{g-1} is independently methyl, ethyl, n-propyl, isopropyl, n-butyl, isobutyl, tert-butyl, n-pentyl, or n-hexyl;
(19) in R^{g}, the C₃₋₁₂cycloalkyl is independently cyclopropyl, cyclobutyl, cyclopentyl, cyclohexyl, or 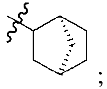
(20) in R^{g}, the C₆₋₁₀aryl or the C₆₋₁₀aryl in the C₆₋₁₀aryl substituted with 1, 2 or 3 R^{g-3} is phenyl;
(21) in R^{g-1}, R^{g-3} and R^{g-4}, the halogen is Cl;
(22) in R^{g-1}, R^{g-3} and R^{g-4}, the C₆₋₁₀aryl or the C₆₋₁₀aryl in the C₆₋₁₀aryl substituted with 1, 2 or 3 R^{g-l-l} is phenyl;
(23) in R^{g-1-1}, the C₁₋₁₂alkyl in the C₁₋₁₂alkyl substituted with 1, 2 or 3 halogen is independently methyl, ethyl, n-propyl, isopropyl, n-butyl, isobutyl, tert-butyl, or n-pentyl;
(24) in R^{g-l-l}, the halogen in the C₁₋₁₂alkyl substituted with 1, 2 or 3 halogen is F;
(25) in R², the C₁₋₁₂alkyl is independently methyl, ethyl, n-propyl, isopropyl, n-butyl, isobutyl, tert-butyl, n-pentyl, or n-hexyl;
(26) in R², the halogen is F;
(27) in R³, the -COC₁₋₁₂alkyl or the C₁₋₁₂alkyl in the -COC₁₋₁₂alkyl substituted with 1, 2 or 3 R³⁻¹ is independently methyl, ethyl, n-propyl, isopropyl, n-butyl, isobutyl, tert-butyl, n-pentyl, or n-hexyl;
(28) in R³, the peptide formed by 2 or more amino acids is a peptide formed by 2 to 10 amino acids;
(29) in R³⁻¹, the C₁₋₁₂alkyl in the -OC₁₋₁₂alkyl is independently methyl, ethyl, n-propyl, isopropyl, n-butyl, isobutyl, tert-butyl, n-pentyl, or n-hexyl;
(30) in R⁴ and R³, the C₁₋₁₂alkyl is independently methyl, ethyl, n-propyl, isopropyl, n-butyl, isobutyl, tert-butyl, n-pentyl, or n-hexyl;
(31) in R⁷, the C₃₋₁₂cycloalkyl in the -CH₂C₃₋₁₂cycloalkyl is independently cyclopropyl, cyclobutyl, cyclopentyl, or cyclohexyl;
(32) in R⁷⁻¹, the halogen in the C₁₋₁₂alkyl substituted with 1, 2 or 3 halogen is F; and
(33) in R⁷⁻¹, the C₁₋₁₂alkyl in the C₁₋₁₂alkyl substituted with 1, 2 or 3 halogen is independently methyl, ethyl, n-propyl, isopropyl, n-butyl, isobutyl, t-butyl, or n-pentyl.

7. The polypeptide compound of claim 5, a pharmaceutically acceptable salt thereof, a solvate thereof or a solvate of a pharmaceutically acceptable salt thereof, wherein the polypeptide compound satisfies one or more of the following:
(1) in R¹⁻¹, the C₁₋₁₂alkyl in the -OC₁₋₁₂alkyl is tert-butyl;
(2) R^{d} is naphthyl;
(3) in R^{a} and R^{b}, the C₁₋₁₂alkyl or the C₁₋₁₂alkyl in the C₁₋₁₂alkyl substituted with 1, 2 or 3 R^{b-1} is independently methyl or n-pentyl;
(4) in R^{a} and R^{b}, the C₃₋₁₂cycloalkyl is independently cyclopentyl or 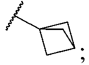
(5) in R^{b-1}, the C₃₋₁₂cycloalkyl is cyclopentyl;
(6) in R^{b-l-1}, the C₁₋₁₂alkyl in the C₁₋₁₂alkyl substituted with 1, 2 or 3 halogen is methyl;
(7) in R¹⁻² and R¹⁻³, the C₁₋₁₂alkyl is independently methyl, isopropyl, n-pentyl, or n-hexyl;
(8) in R^{g}, the C₁₋₁₂alkyl or the C₁₋₁₂alkyl in the C₁₋₁₂alkyl substituted with 1, 2 or 3 R^{g-1} is independently methyl, ethyl or n-pentyl;
(9) in R^{g}, the C₃₋₁₂cycloalkyl is independently cyclopentyl, cyclohexyl or 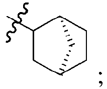
(10) in R^{g-1-1}, the C₁₋₁₂alkyl in the C₁₋₁₂alkyl substituted with 1, 2 or 3 halogen is methyl;
(11) in R², the C₁₋₁₂alkyl is methyl;
(12) in R³, the -COC₁₋₁₂alkyl or the C₁₋₁₂alkyl in the -COC₁₋₁₂alkyl substituted with 1, 2 or 3 R³⁻¹ is independently methyl, ethyl or n-pentyl;
(13) in R³⁻¹, the C₁₋₁₂alkyl in the -OC₁₋₁₂alkyl is methyl;
(14) in R⁴ and R⁵, the C₁₋₁₂alkyl is methyl;
(15) in R⁷, the C₃₋₁₂cycloalkyl in the -CH₂C₃₋₁₂cycloalkyl is cyclohexyl; and
(16) in R⁷⁻¹, the C₁₋₁₂alkyl in the C₁₋₁₂alkyl substituted with 1, 2 or 3 halogen is methyl.

8. The polypeptide compound of claim 1 or 2, a pharmaceutically acceptable salt thereof, a solvate thereof or a solvate of a pharmaceutically acceptable salt thereof, wherein the polypeptide compound satisfies one or more of the following:
(1) R¹ is -CH₂COOH, -CH₂CH₂COOH, 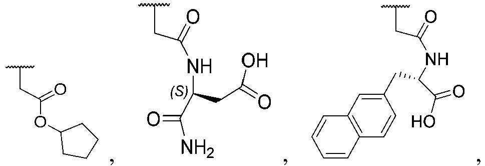 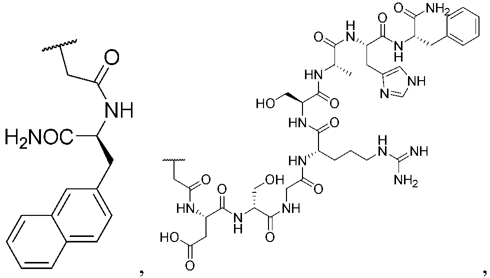 -CH₂CONH₂, 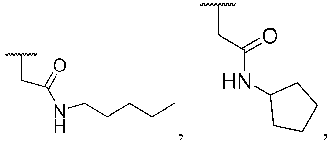 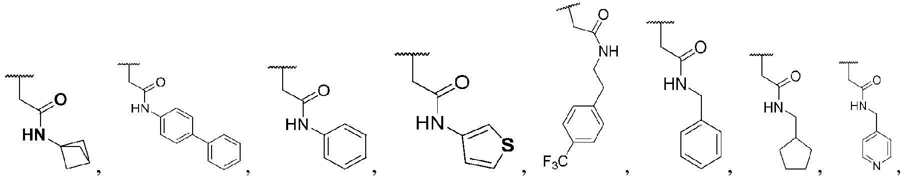 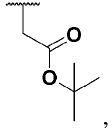 -CH₂NH₂, -CH₂NHCOCH₃, -CH₂CH₂NH₂, -CH₂NH(CH₂)₄CH₃, -CH₂NH(CH₂)₅CH₃, 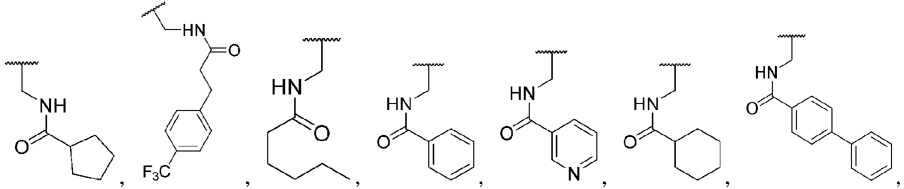 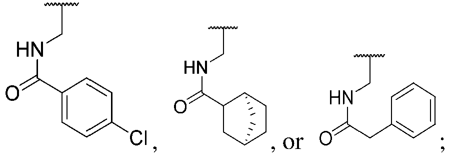
(2) **R³ is** 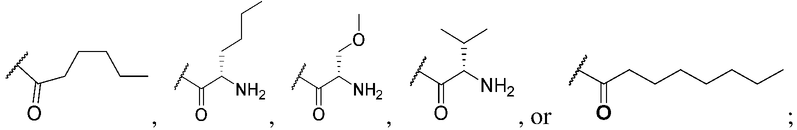
(3) B is

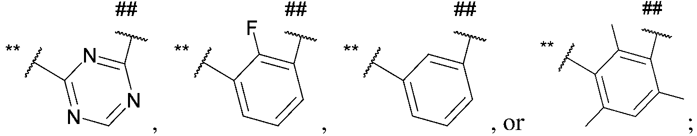

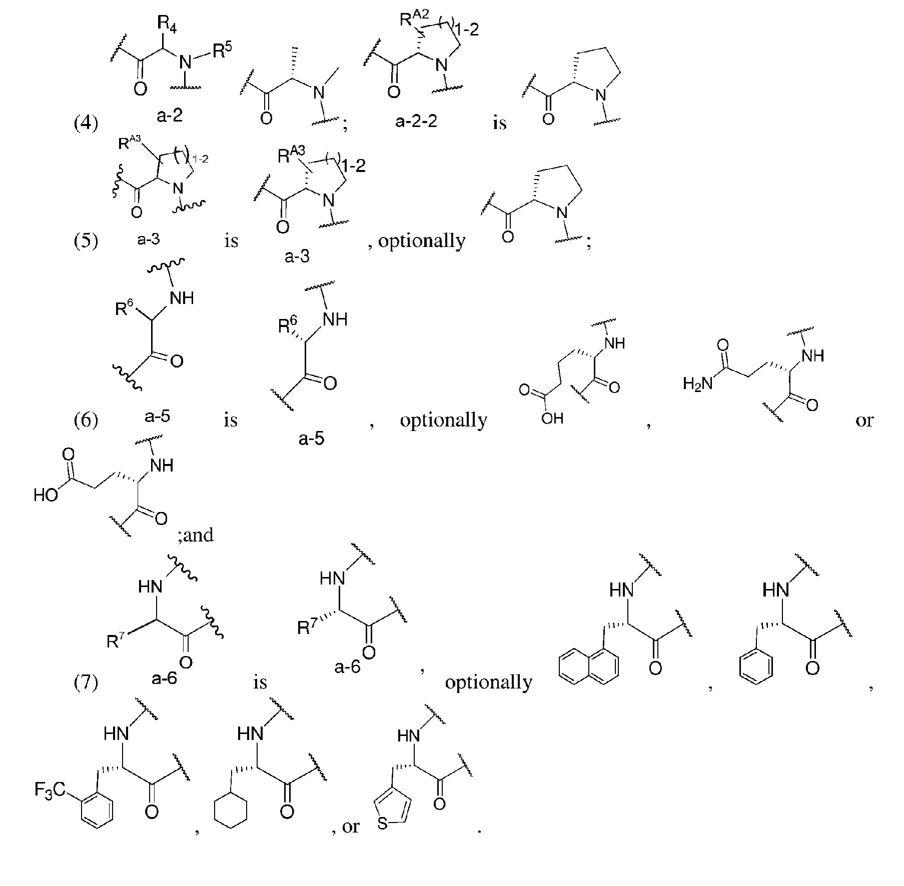

9. The polypeptide compound of claim 8, a pharmaceutically acceptable salt thereof, a solvate thereof or a solvate of a pharmaceutically acceptable salt thereof, wherein the polypeptide compound satisfies one or more of the following:
(1) R³ is 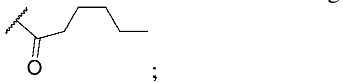
(2) B is 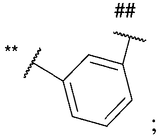
(3) 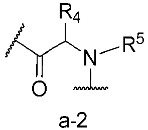 is 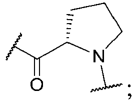
(4) 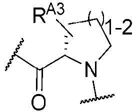 is 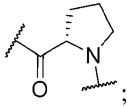
*(5)* 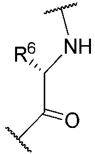 is 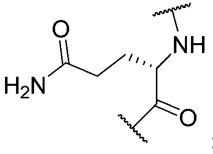 and
(6) 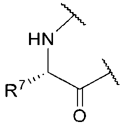 is

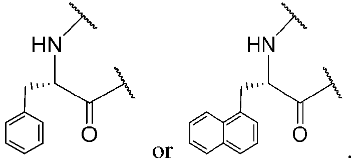

10. The polypeptide compound of claim 1, a pharmaceutically acceptable salt thereof, a solvate thereof or a solvate of a pharmaceutically acceptable salt thereof, wherein the polypeptide compound is selected from any of the following compounds: 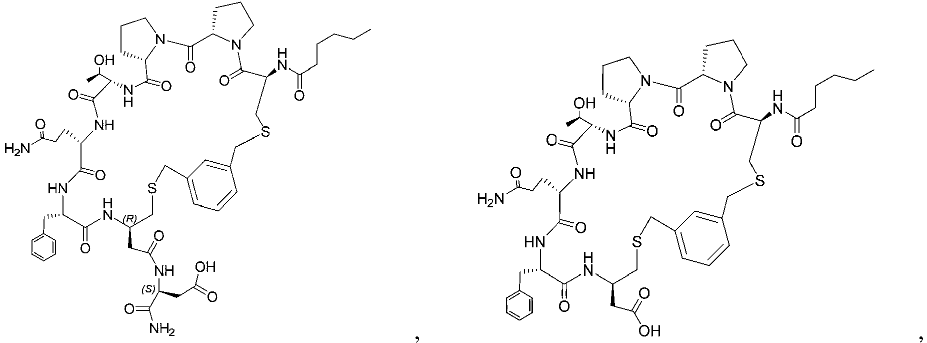 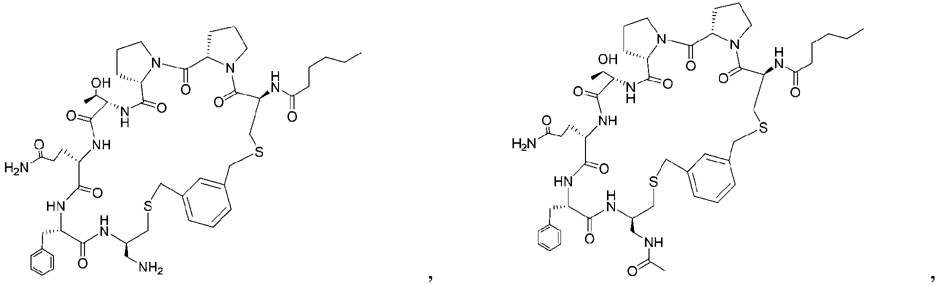 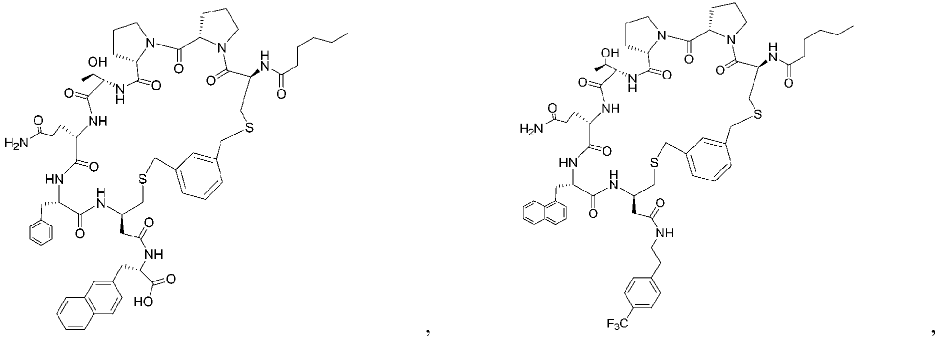 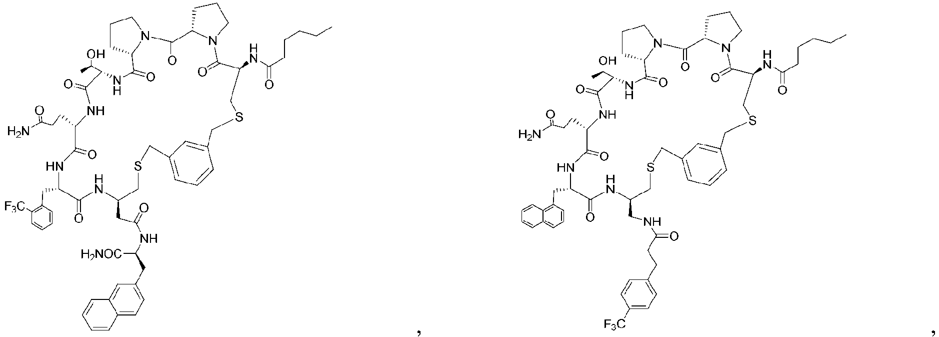 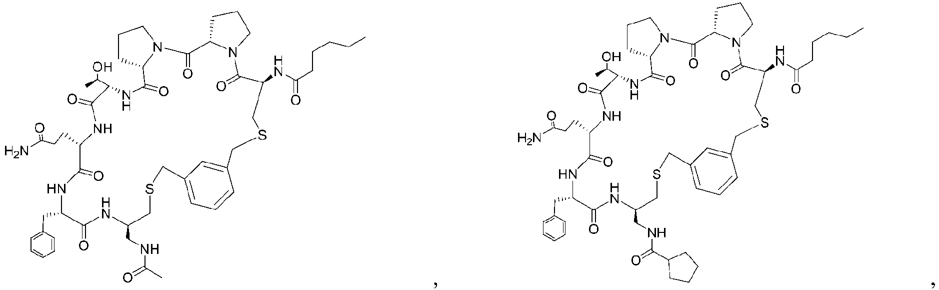 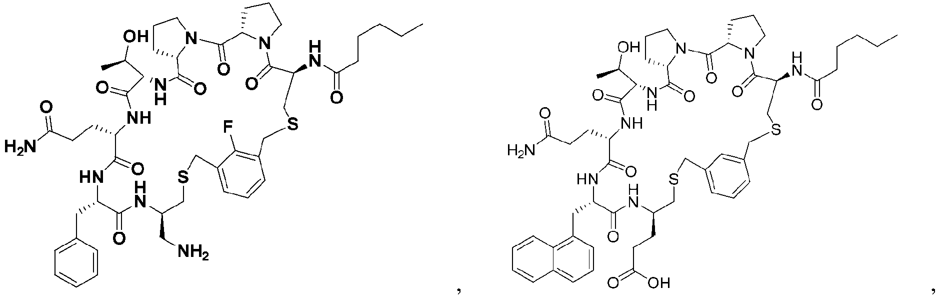 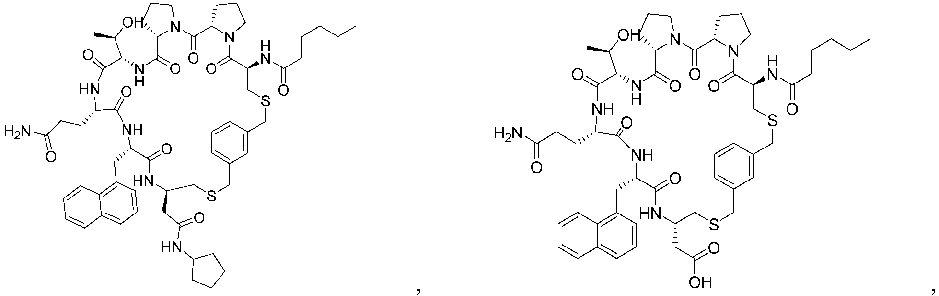 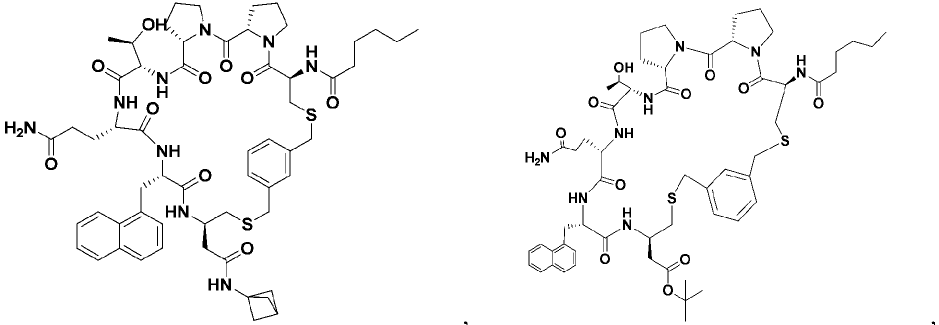 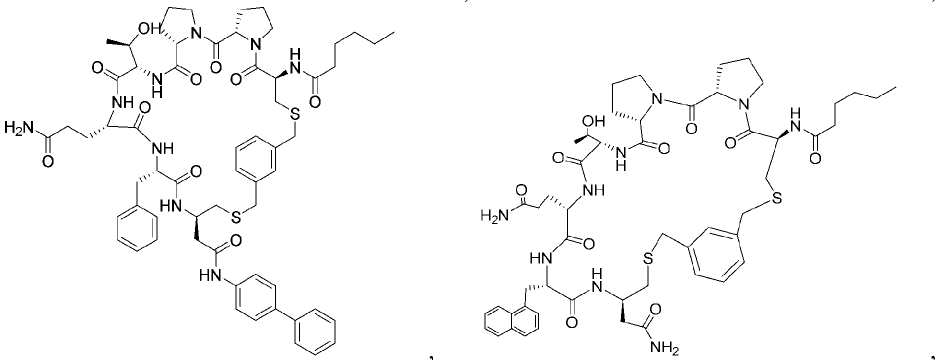 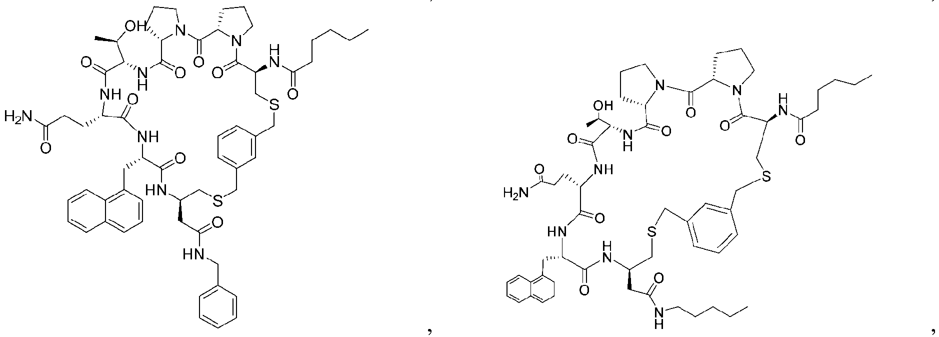 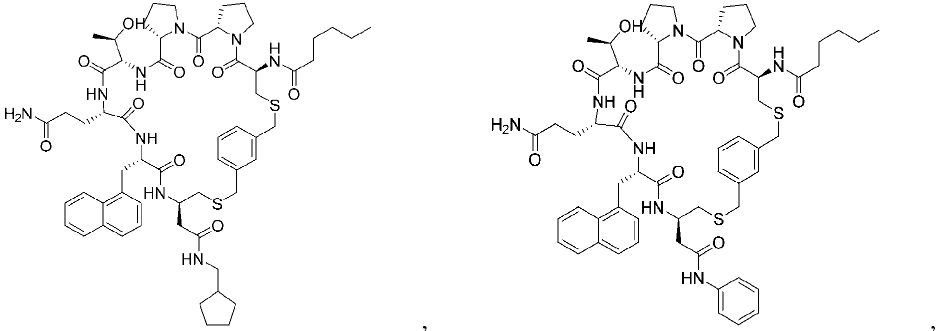 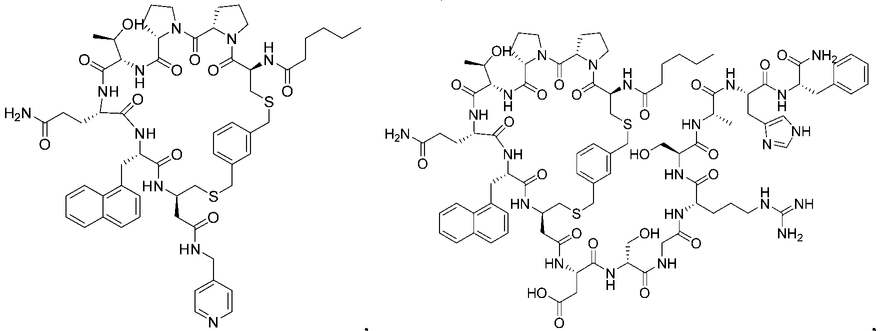 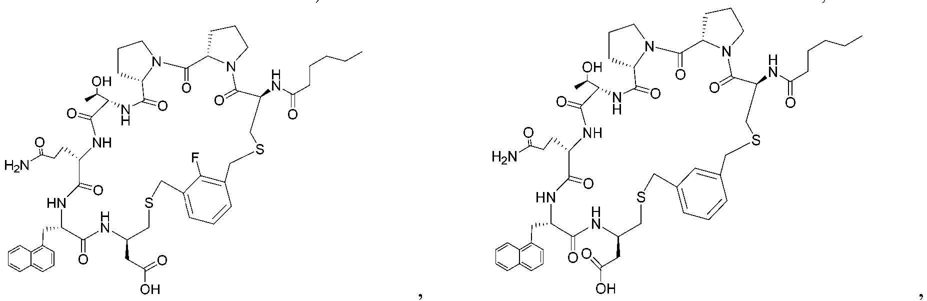 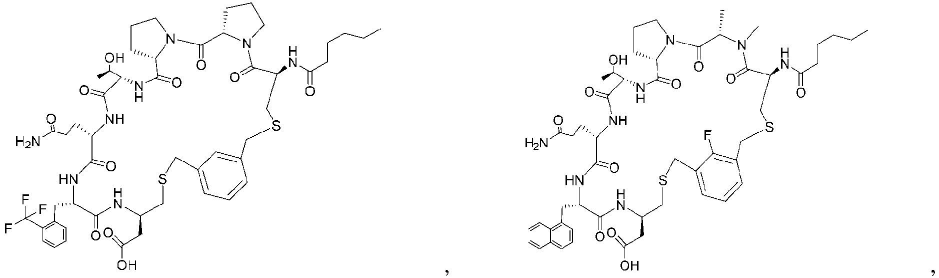 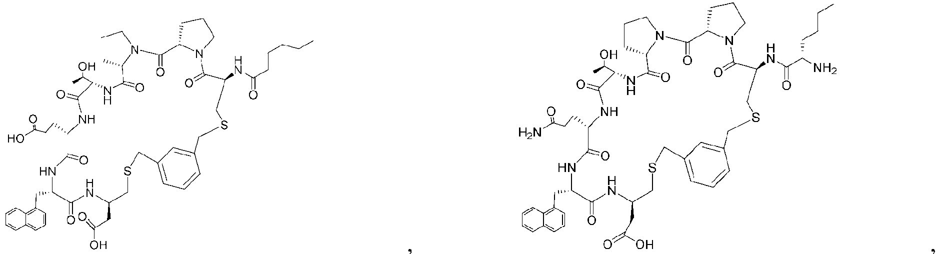 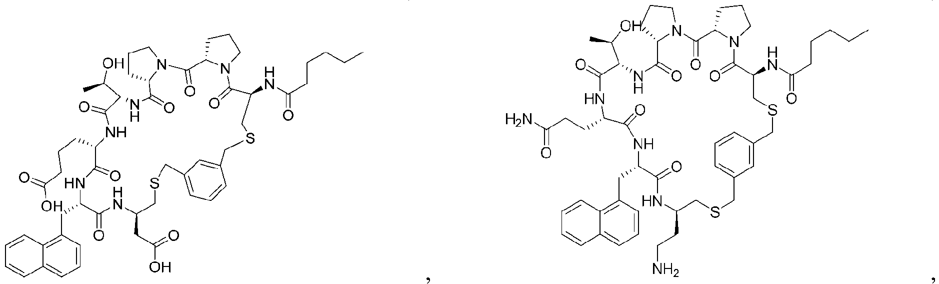 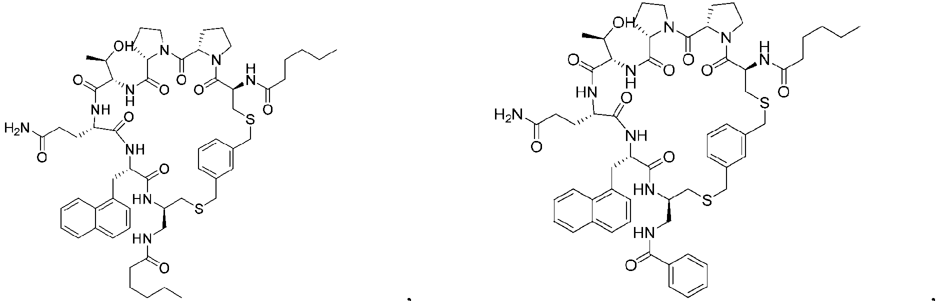 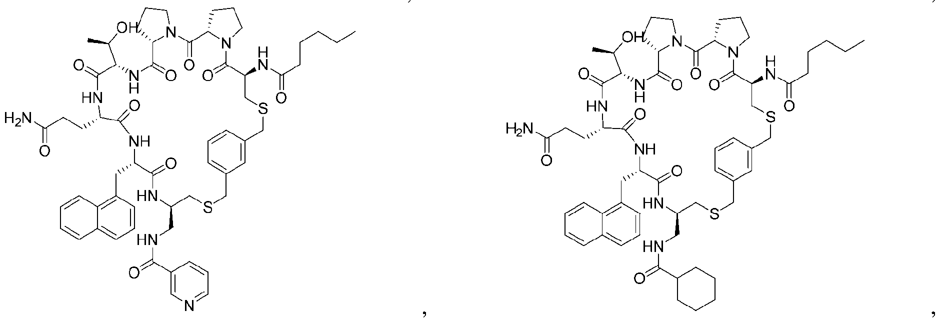 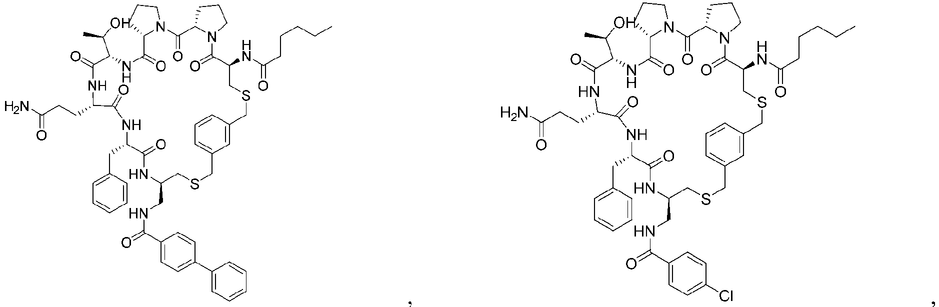 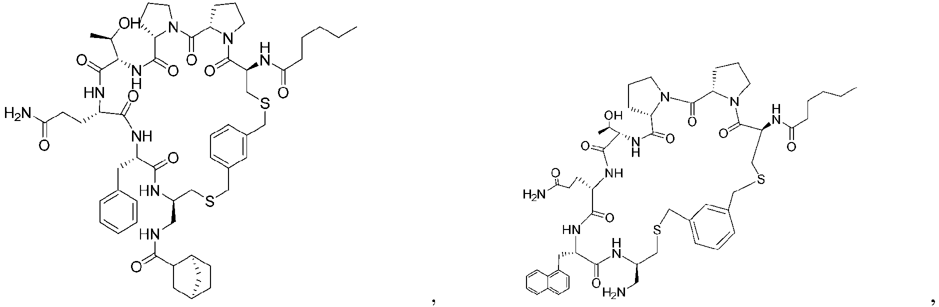 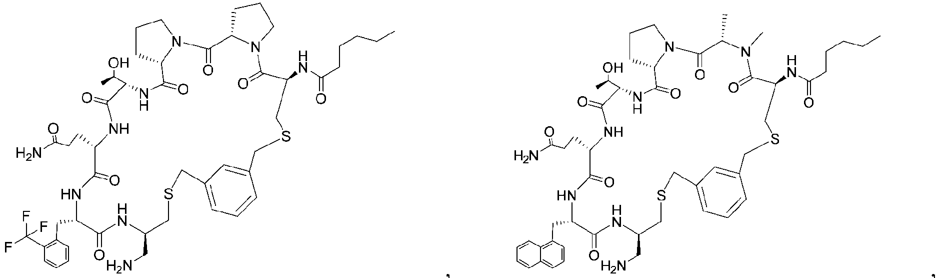 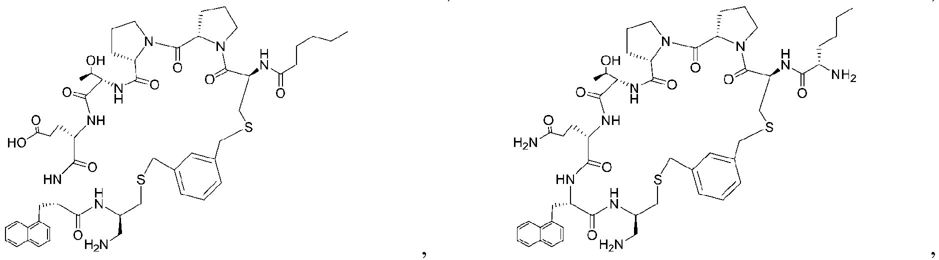 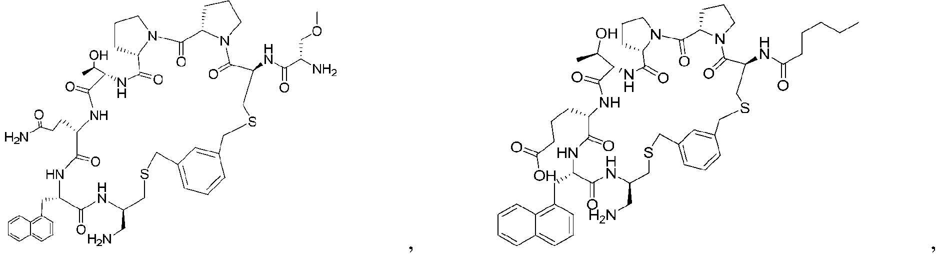 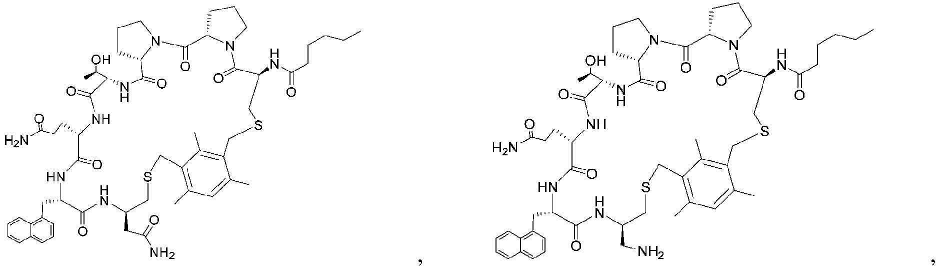 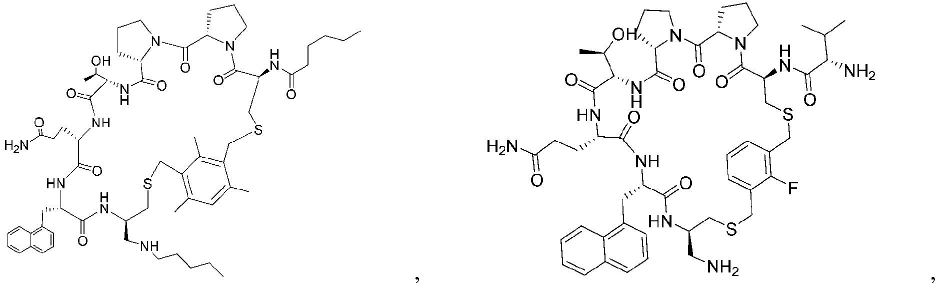 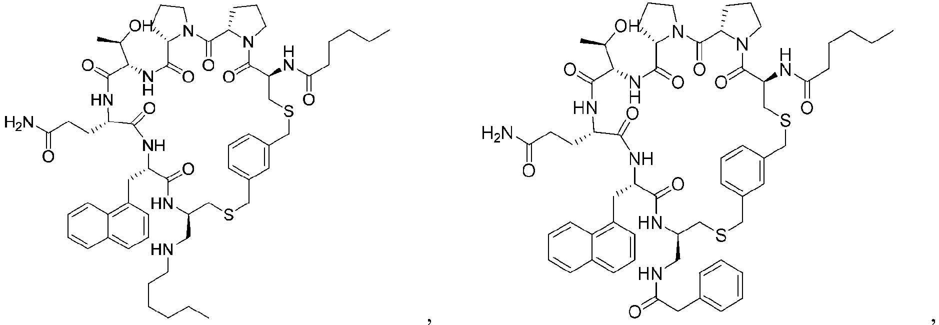 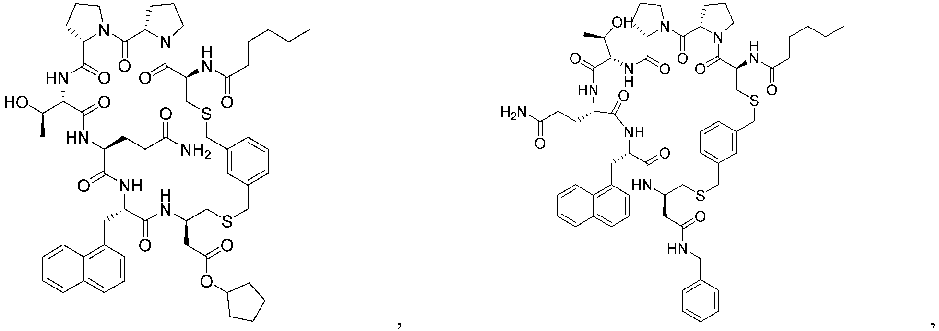 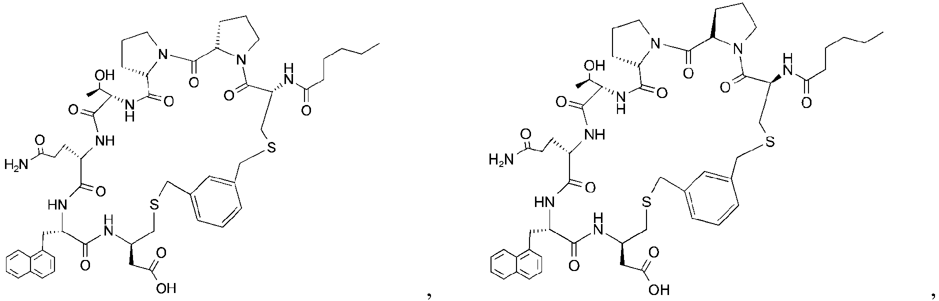 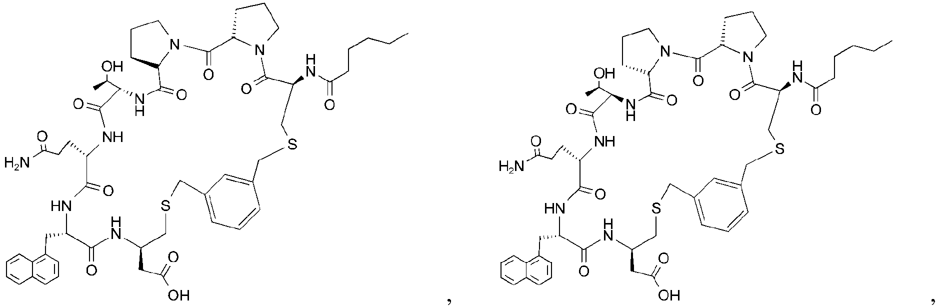 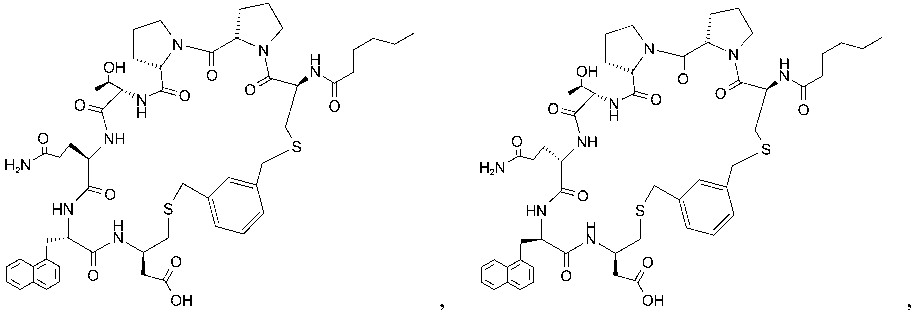 and

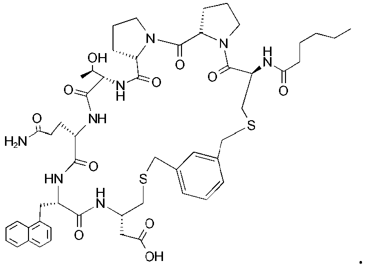

11. A polypeptide compound, a pharmaceutically acceptable salt thereof, a solvate thereof or a solvate of a pharmaceutically acceptable salt thereof, wherein the polypeptide compound is a compound represented by formula I-B; wherein A₁, A₂, A₃, A₄, A₅, A₆ and A₇ are sequentially connected via a peptide bond (-CO-NH-); 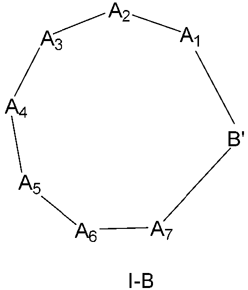
R¹ is -(CH₂)_{m'}M;
m' is 0, 1, 2, 3, 4, 5, or 6;
**B' is** 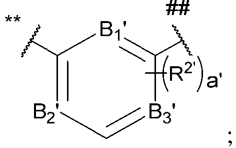 a' is 1, 2 or 3;
B₁' is CH, and B₂' and B₃' are independently N or C; R²' is independently H, C₁₋₁₂alkyl or halogen, and at least one R^{2,} is halogen;
or B₁', B₂' and B₃' are independently N or CH; R²' is independently C₁₋₁₂alkyl or halogen; and
other groups are as defined in any one of claims 1-10.

12. The polypeptide compound of claim 11, a pharmaceutically acceptable salt thereof, a solvate thereof or a solvate of a pharmaceutically acceptable salt thereof, wherein the polypeptide compound satisfies one or both of the following:
(1) B' is 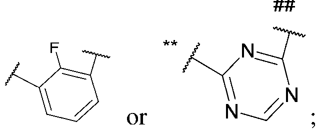 and
(2) R¹ is -COOH.

13. The polypeptide compound of claim 12, a pharmaceutically acceptable salt thereof, a solvate thereof or a solvate of a pharmaceutically acceptable salt thereof, wherein the compound is selected from any of the following structures:

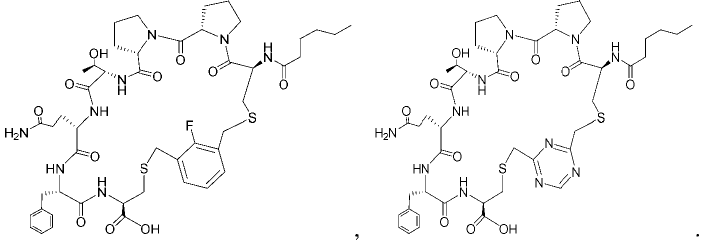

14. A polypeptide compound, a pharmaceutically acceptable salt thereof, a solvate thereof or a solvate of a pharmaceutically acceptable salt thereof, wherein the polypeptide compound is a compound represented by formula I-C; wherein A₁, A₂, A₃, A₄, A₅, A₆' and A₇ are sequentially connected via a peptide bond (-CO-NH-); 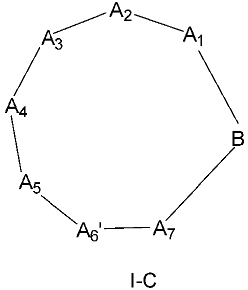
R¹ is -(CH₂)_{m'}M;
m' is 0, 1, 2, 3, 4, 5, or 6;
A₆' has a structure represented by formula a-6', 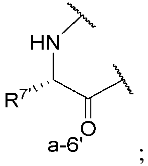
wherein R^{7'} is -CH₂C₃₋₁₂cycloalkyl, C₆₋₁₀aryl substituted with 1, 2 or 3 R⁷⁻¹, or -CH₂C₃₋₁₂cycloalkyl substituted with 1, 2 or 3 R⁷⁻²;
R⁷⁻¹ and R⁷⁻² are independently C₁₋₁₂alkyl or C₁₋₁₂alkyl substituted with 1, 2 or 3 halogen; and
other groups are as defined in any one of claims 1-10.

15. The polypeptide compound of claim 14, a pharmaceutically acceptable salt thereof, a solvate thereof or a solvate of a pharmaceutically acceptable salt thereof, wherein the polypeptide compound satisfies one or both of the following:
(1) R^{7'} is -CH₂C₃₋₁₂cycloalkyl, or C₆₋₁₀aryl substituted with 1, 2 or 3 R⁷⁻¹, and
(2) R¹ is -COOH.

16. The polypeptide compound of claim 14, a pharmaceutically acceptable salt thereof, a solvate thereof or a solvate of a pharmaceutically acceptable salt thereof, wherein R^{7'} is - CH₂cyclohexyl or

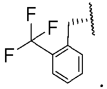

17. The polypeptide compound of claim 14, a pharmaceutically acceptable salt thereof, a solvate thereof or a solvate of a pharmaceutically acceptable salt thereof, wherein the polypeptide compound is the following compound:

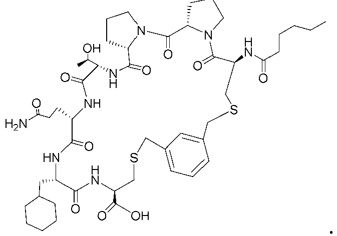

18. A polypeptide compound, a pharmaceutically acceptable salt thereof, a solvate thereof or a solvate of a pharmaceutically acceptable salt thereof, wherein the polypeptide compound is represented by formula II,
D-L; II
D is the compound represented by formula I of any one of preceding embodiments or a group formed by the loss of a H atom or OH group at the position of B, R¹ or R³ of the compound represented by formula I of any one of claims 1-10, a group formed by the loss of a H atom or OH group at the position of B', R¹ or R³ of the compound represented by formula I-B of any one of claims 11-13, or a group formed by the loss of a H atom or OH group at the position of B, R¹ or R³ of the compound represented by formula I-C of any one of claims 14-17; and
L is a non-cleavable linker.

19. The polypeptide compound of claim 18, a pharmaceutically acceptable salt thereof, a solvate thereof or a solvate of a pharmaceutically acceptable salt thereof, wherein L is: -A, -W, - W-A, -A-W, -W-A-W, or -A-W-A;
A is a group formed by the loss of a OH group by a carboxyl group on an amino acid, a group formed by the loss of a H atom by an amino group on an amino acid, a group formed by the loss of a -OH group by a carboxyl group on a peptide formed by 2 or more amino acids, or a group formed by the loss of a H atom by an amino group on a peptide formed by 2 or more amino acids;
W is -(Y)_{g-}(CH₂)_{c}-NHR^{t};
Y is independently selected from any one or a combination of the following groups: -(CH₂)_{b}-X-, -(CH₂)_{c}-C₆₋₁₀arylene-(CH₂)a-, -(CH₂)_{c}-5-12-membered heterocycloalkyl-(CH₂)_{d}-, -(CH₂)_{c}-C₃₋₁₂cycloalkylene-(CH₂)_{d}-, and -(CH₂)_{c}-CO-(CH₂)_{d}-; the heteroatom of the 5 to 12-membered heterocycloalkyl is selected from one or more of N, O and S; the number of the heteroatom is 1, 2 or 3;
X is S, O or NH;
R^{t} is H or C₁₋₁₂alkyl, or R^{t} together with -NH forms a 5-10-membered nitrogen-containing heterocycloalkyl;
g, b, c and d are independently 0, 1, 2, 3, 4, 5, 6, 7, 8, 9, and 10.

20. The polypeptide compound of claim 18, a pharmaceutically acceptable salt thereof, a solvate thereof or a solvate of a pharmaceutically acceptable salt thereof, wherein the polypeptide compound satisfies one or both of the following:
(1) D is selected from any of the following segments: 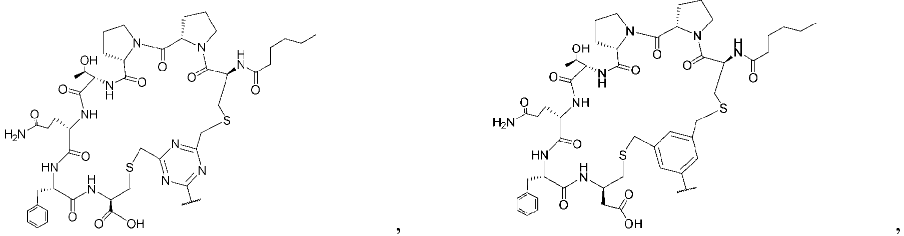 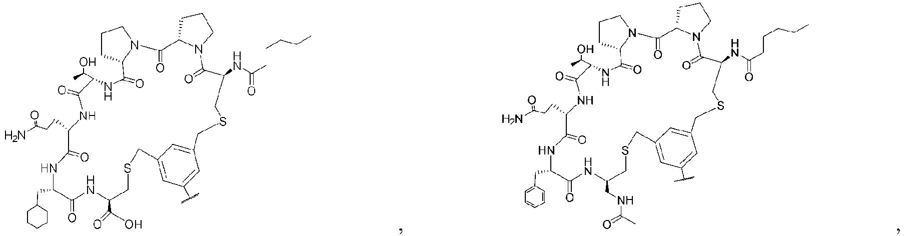 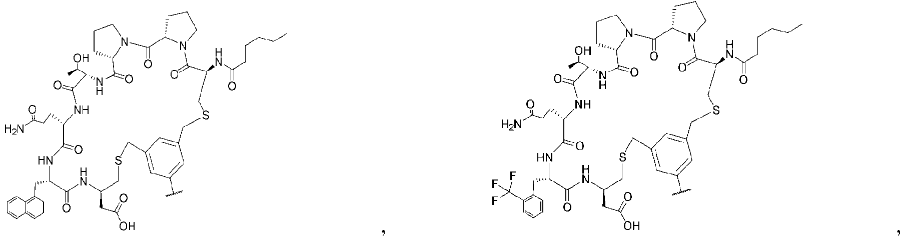 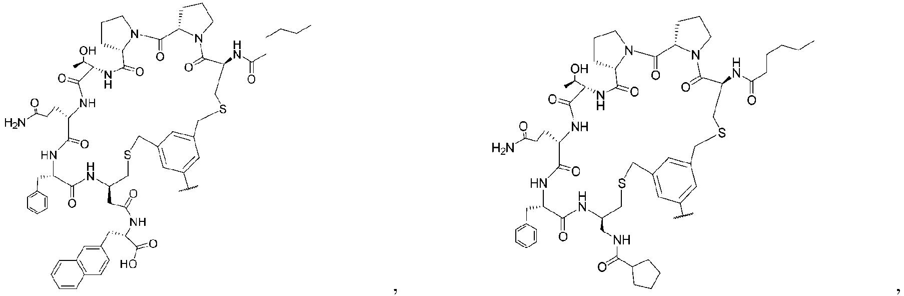 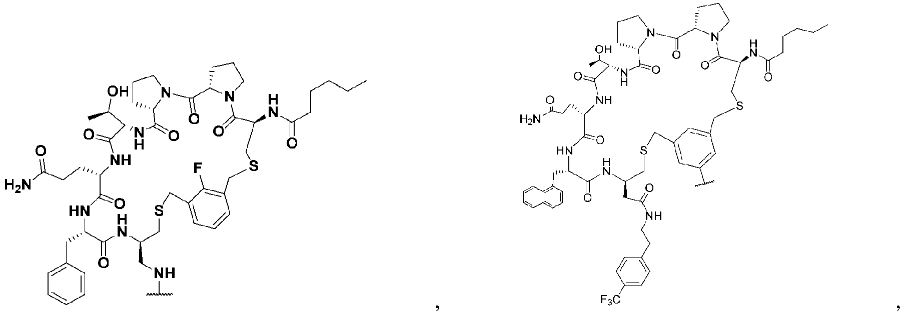 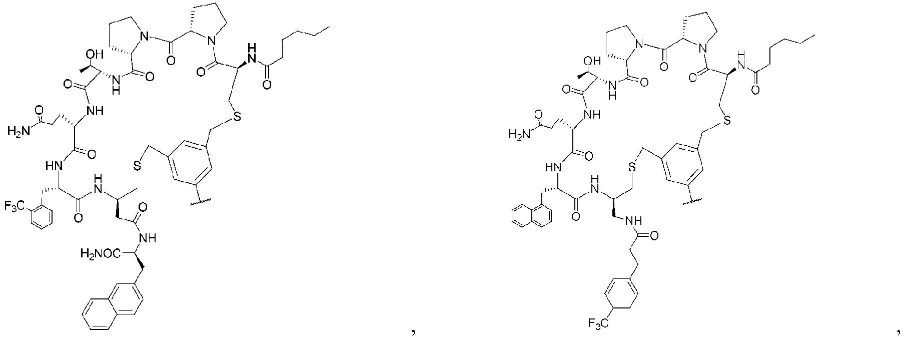 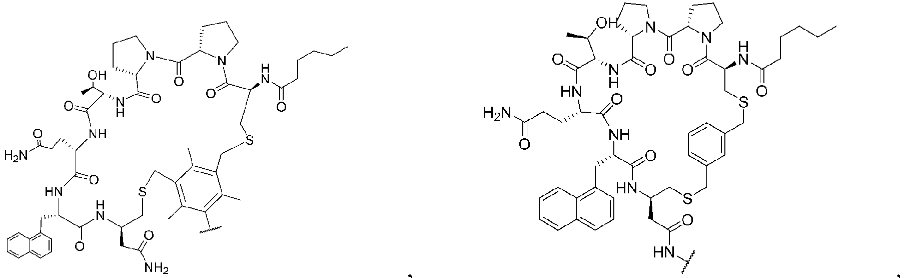 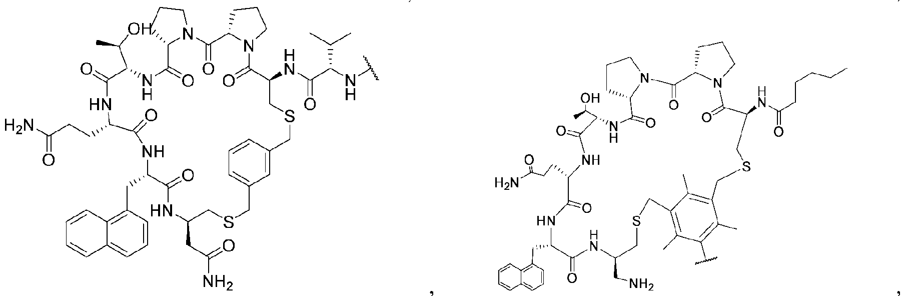 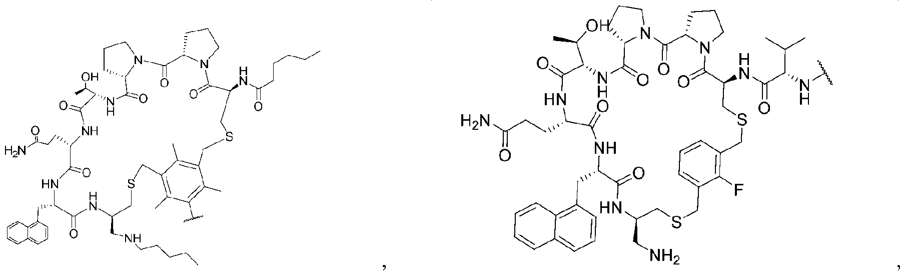 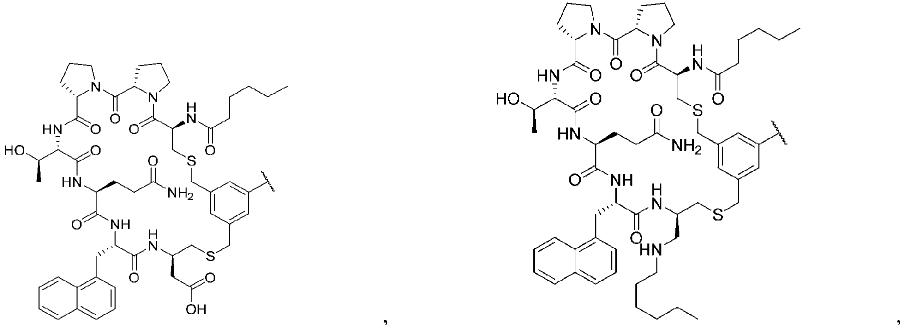 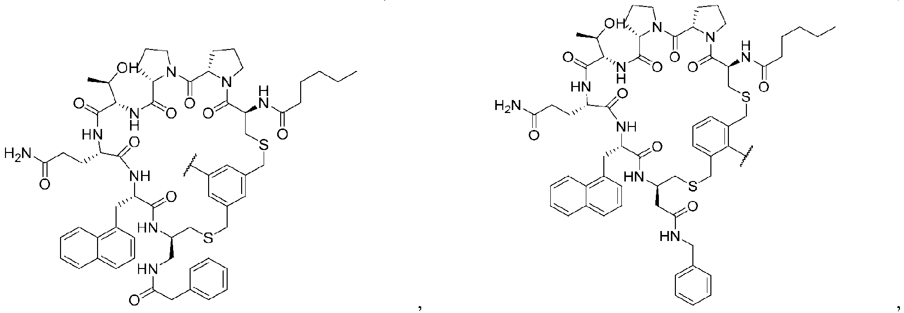 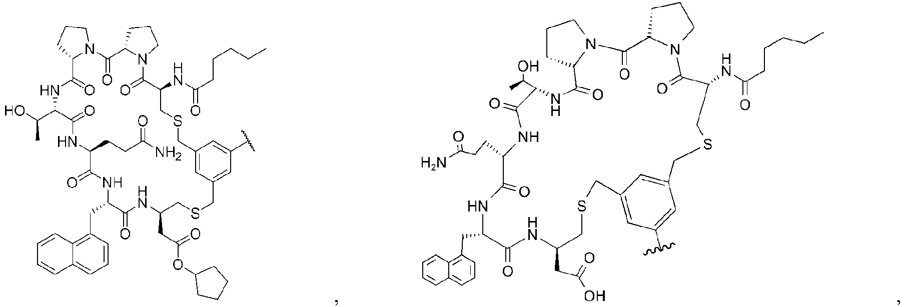 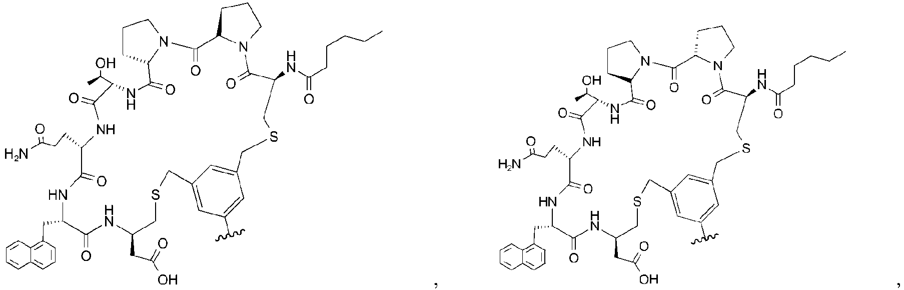 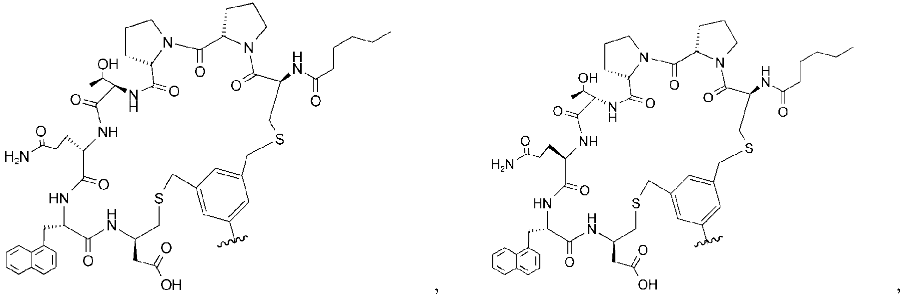 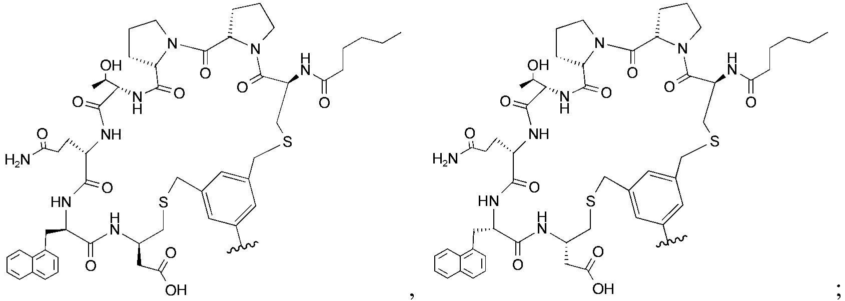 and
(2) L is selected from any of the following segments:

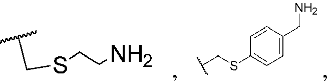

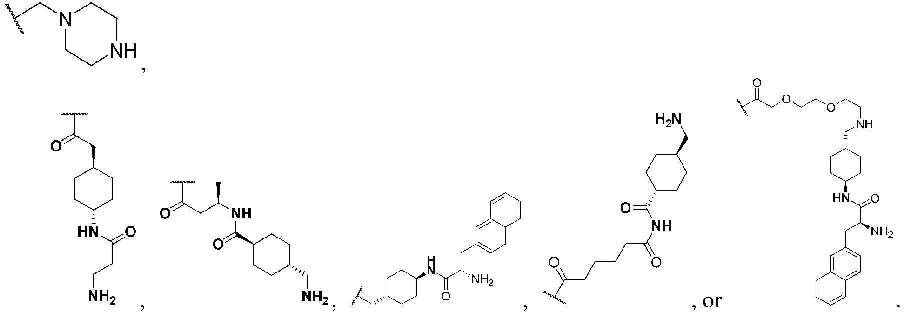

21. The polypeptide compound of claim 18, a pharmaceutically acceptable salt thereof, a solvate thereof or a solvate of a pharmaceutically acceptable salt thereof, wherein the polypeptide compound is selected from any of the following compounds:

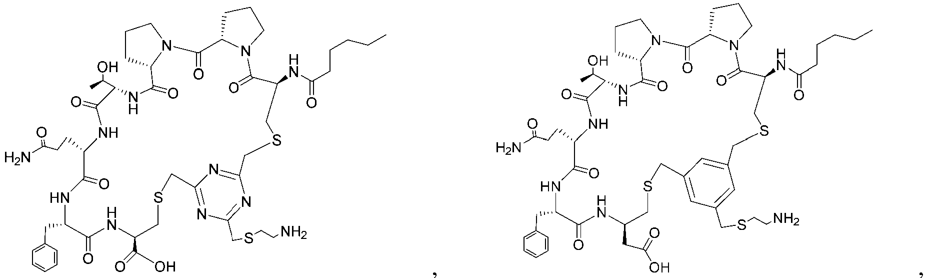

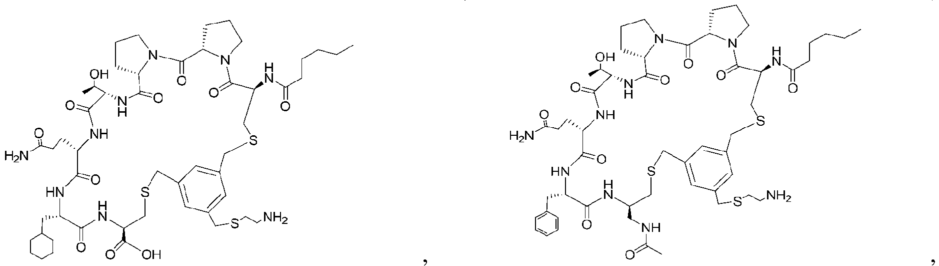

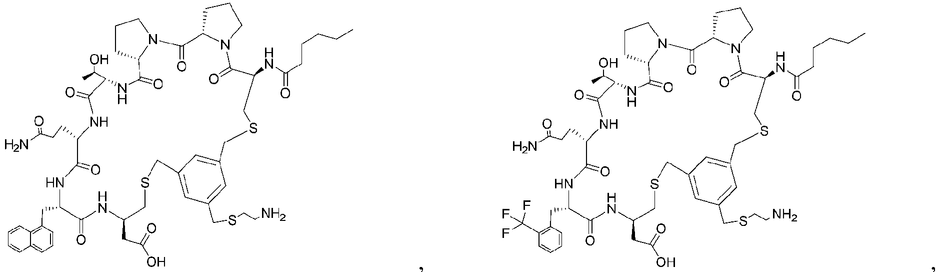

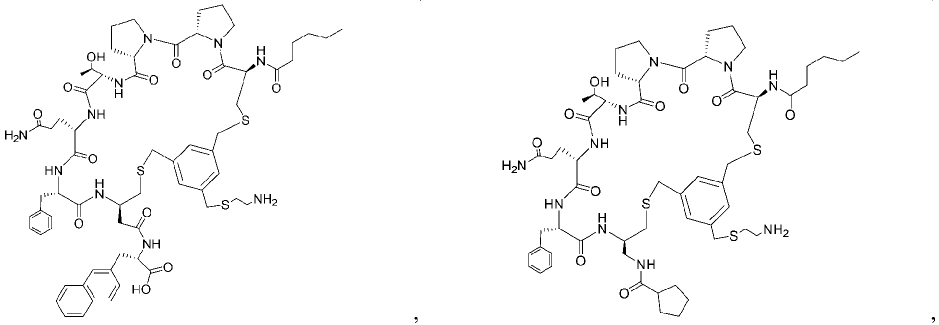

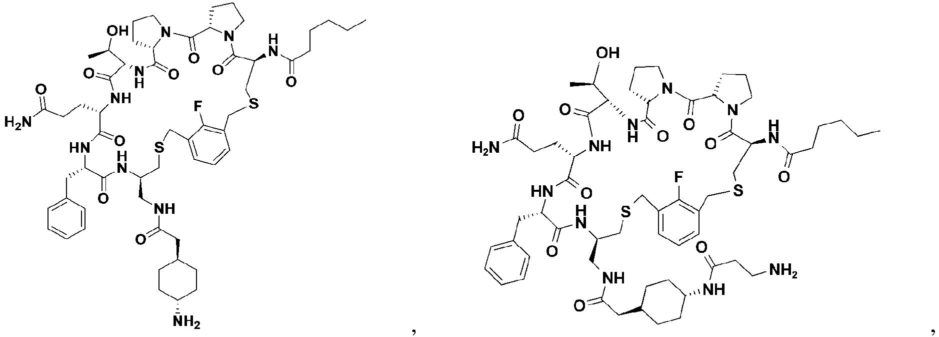

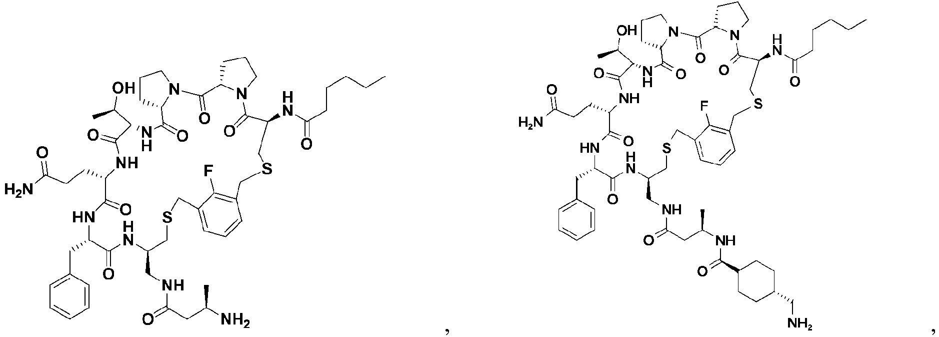

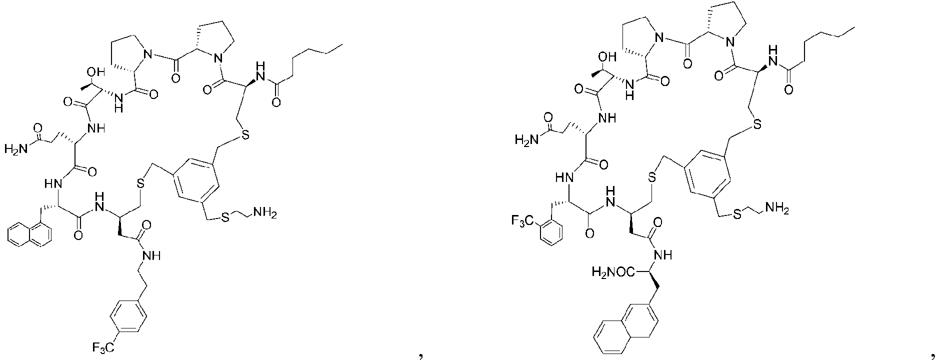

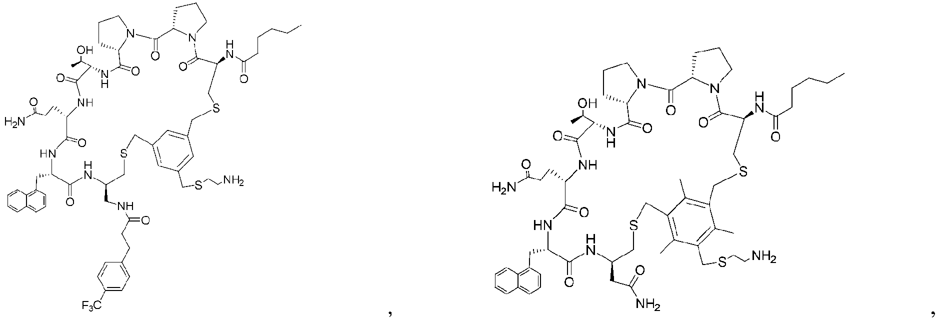

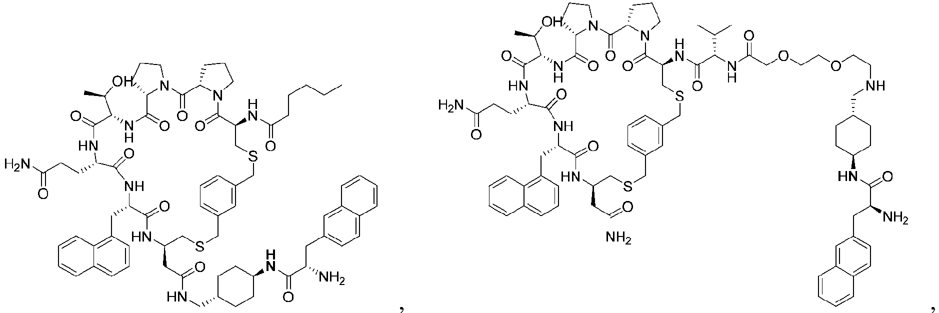

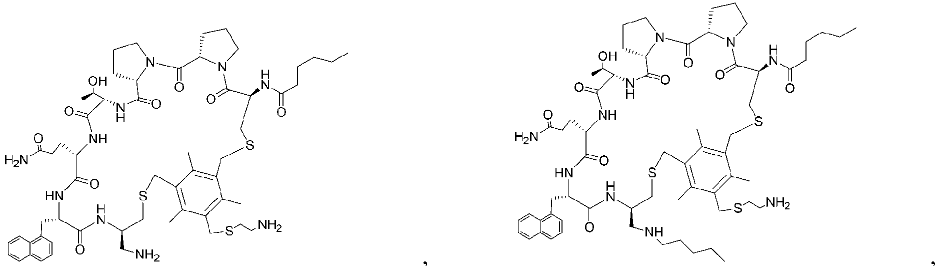

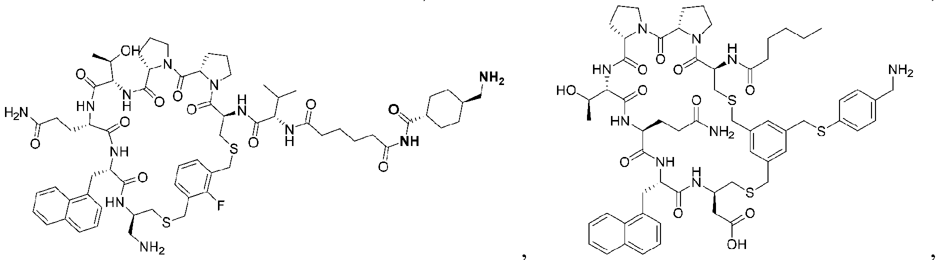

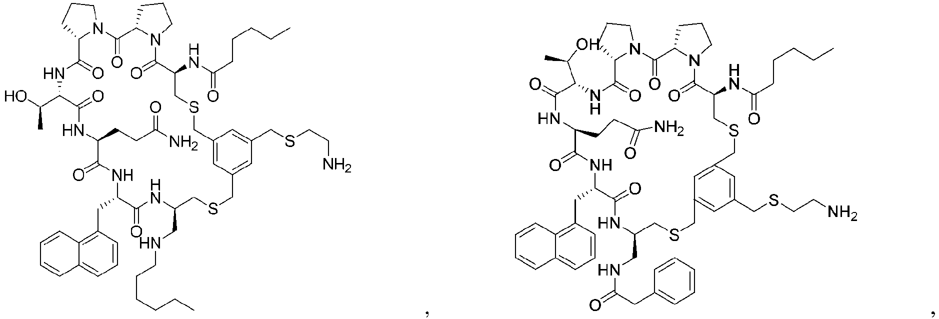

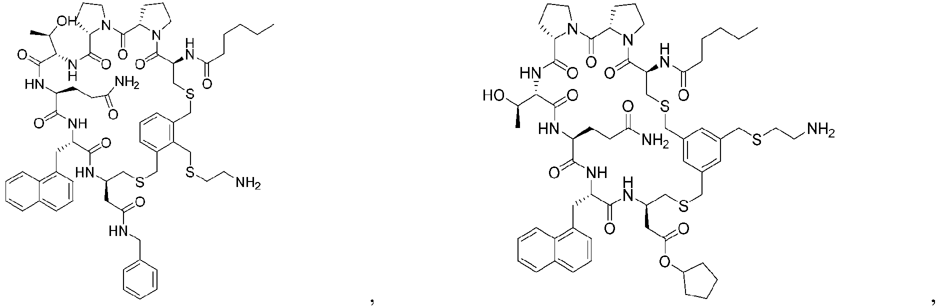

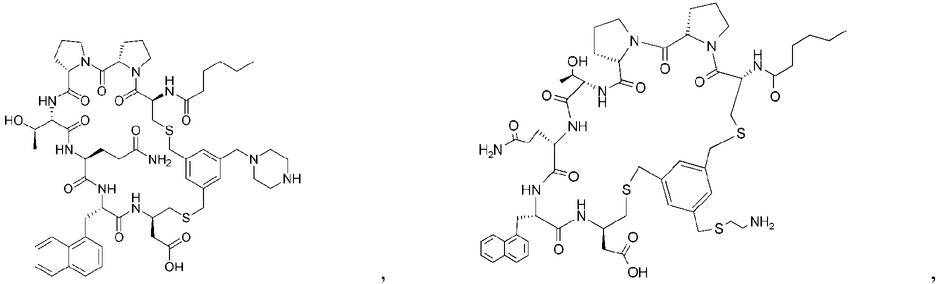

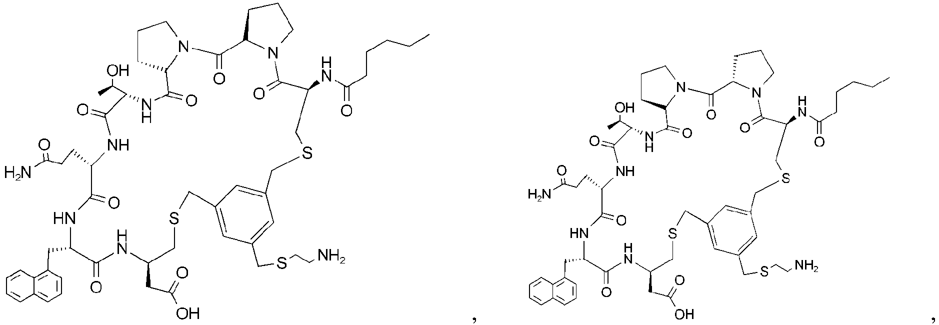

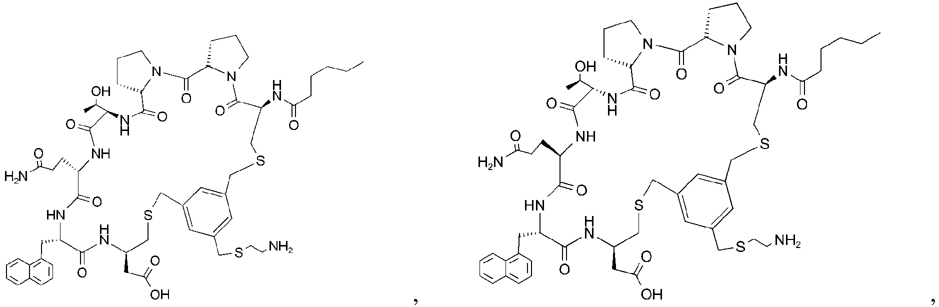

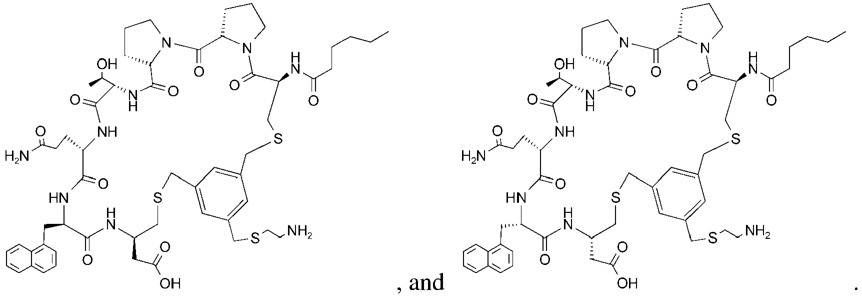

22. A polypeptide compound, a pharmaceutically acceptable salt thereof, a solvate thereof or a solvate of a pharmaceutically acceptable salt thereof, wherein the polypeptide compound is represented by formula III:
D-L'-R **III**
D is as defined in any one of claims 18-21;
L' is a group formed by the loss of a H atom by an amino group on L as defined in any one of claims 18-21; and
R is a group with the function of chelating metal ions.

23. The polypeptide compound of claim 22, a pharmaceutically acceptable salt thereof, a solvate thereof or a solvate of a pharmaceutically acceptable salt thereof, wherein R is a group formed by the loss of H or hydroxyl by a chelating agent shown below: 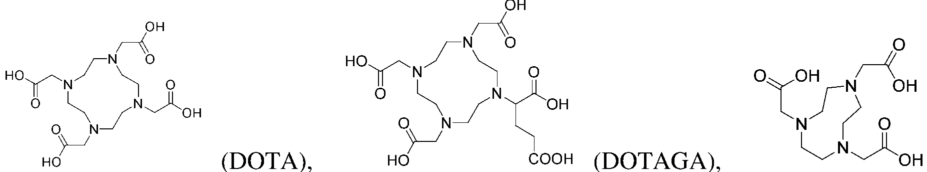 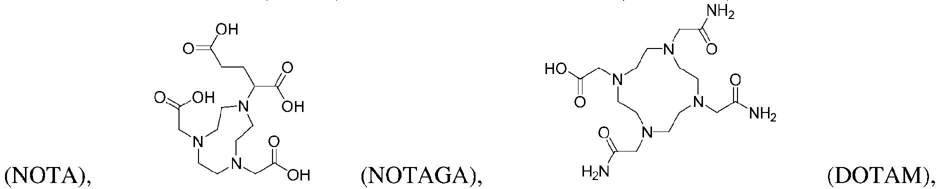 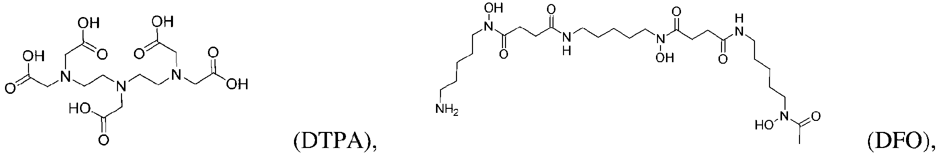 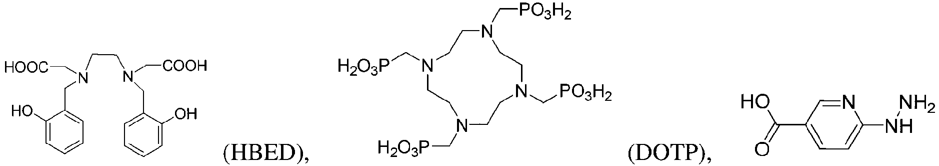 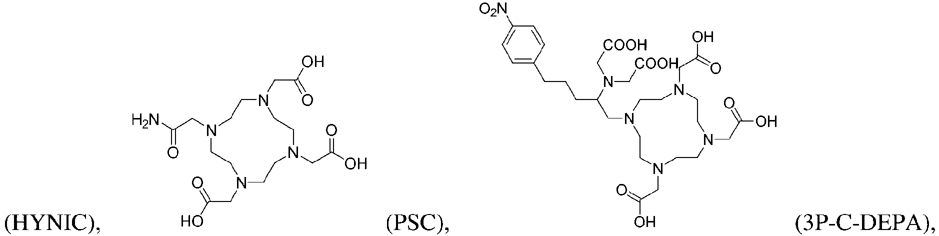 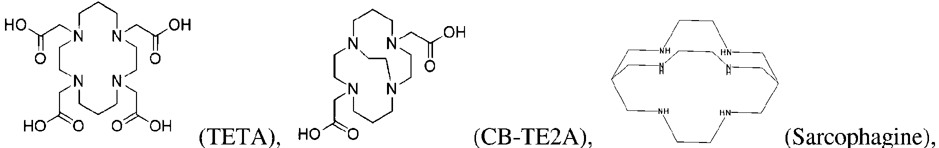 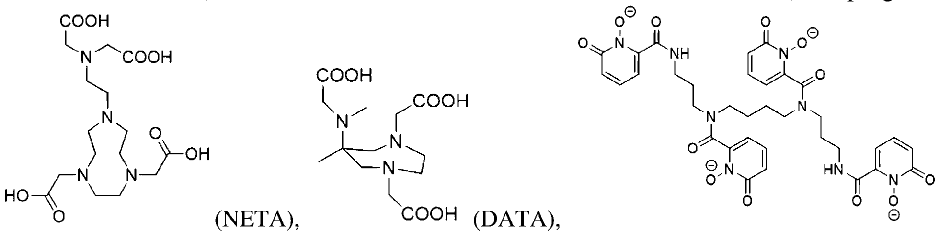 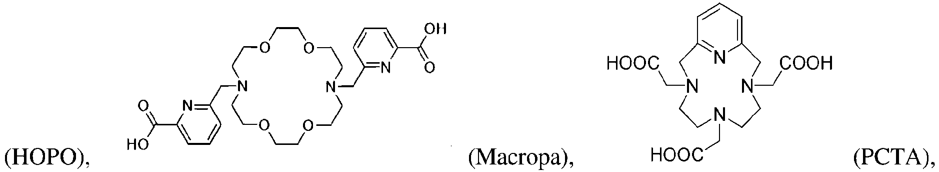 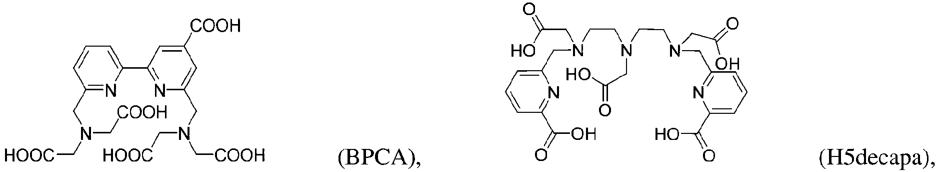 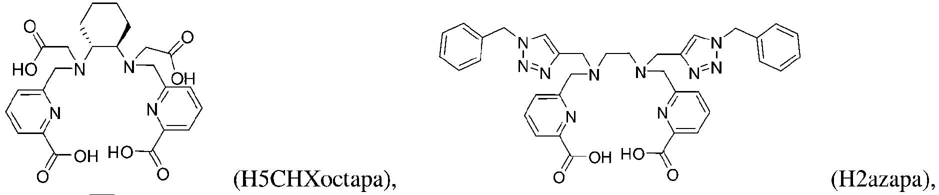 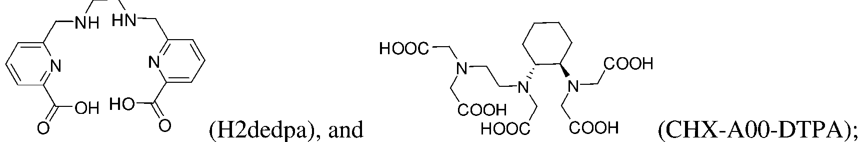 wherein R is preferably

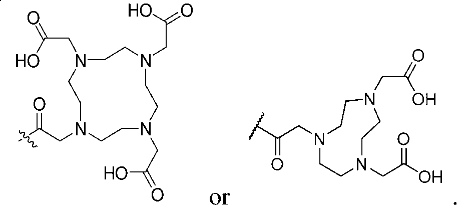

24. The polypeptide compound of claim 22, a pharmaceutically acceptable salt thereof, a solvate thereof or a solvate of a pharmaceutically acceptable salt thereof, wherein the polypeptide compound is selected from any of the following compounds:

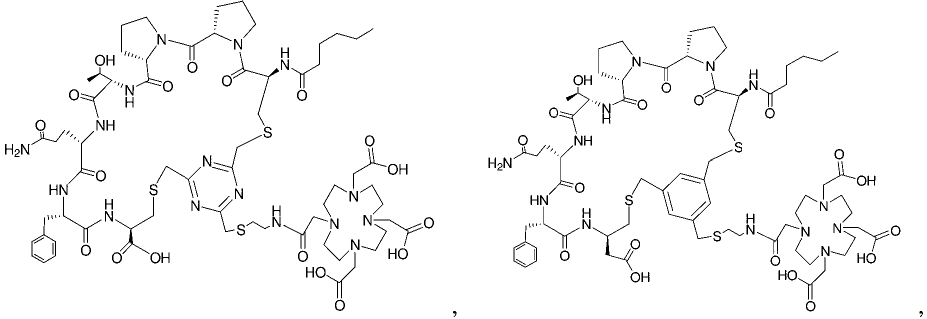

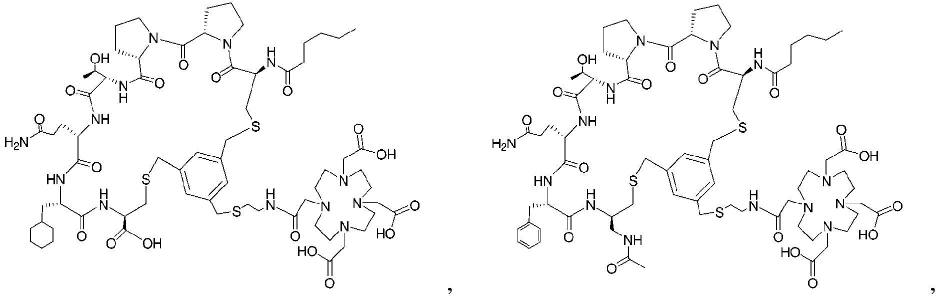

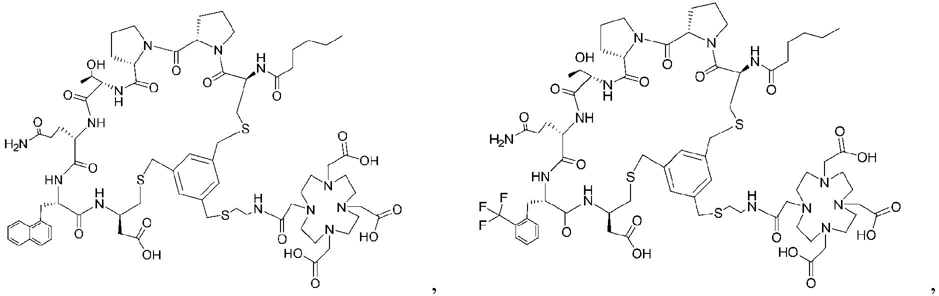

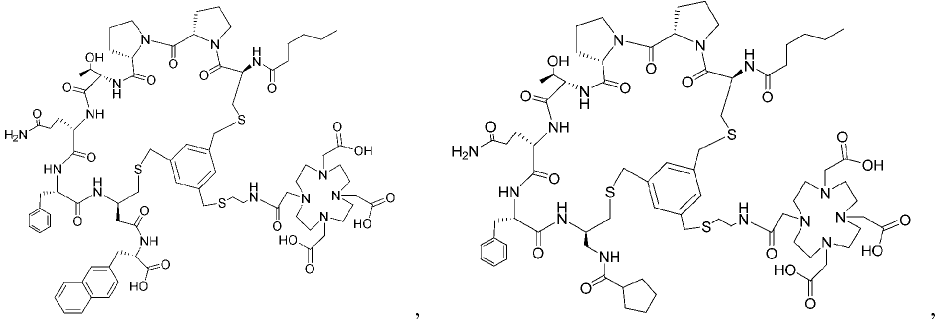

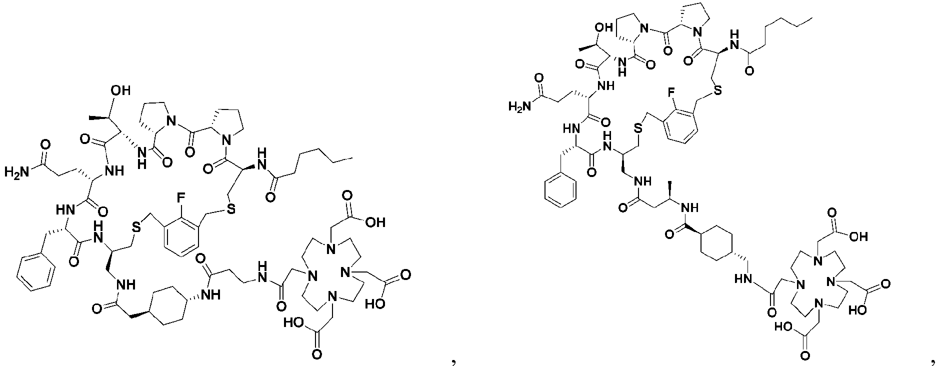

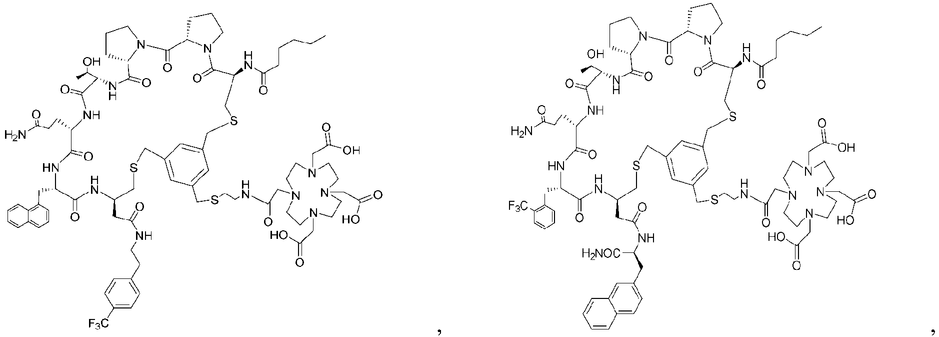

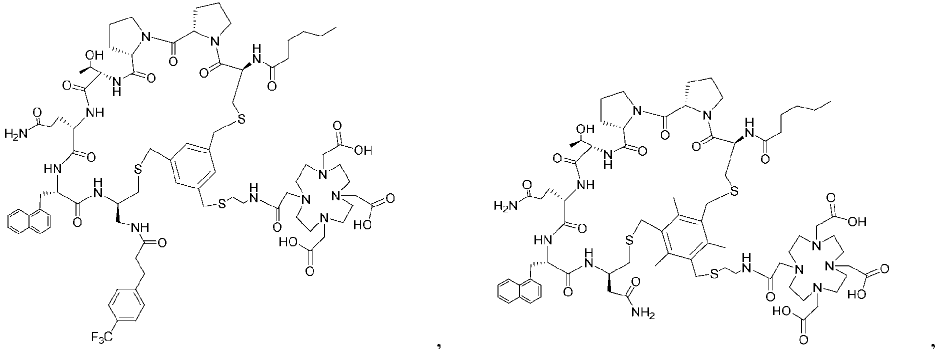

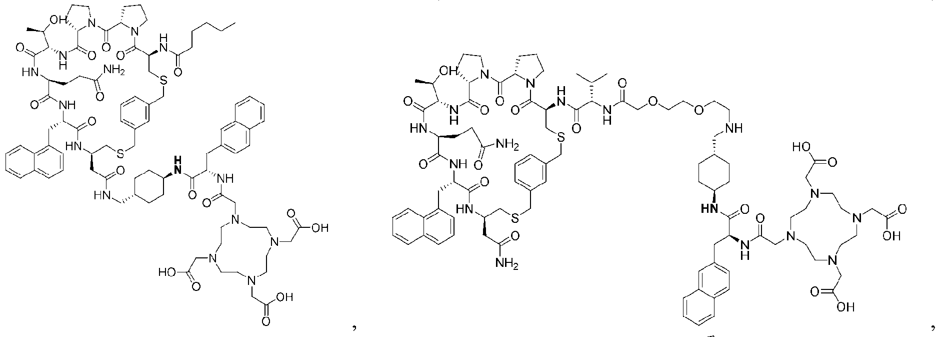

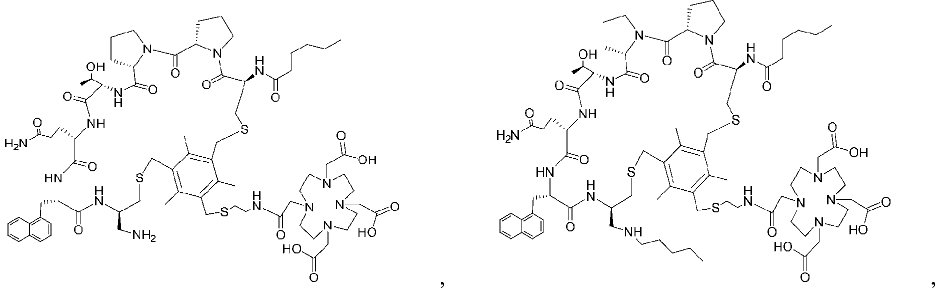

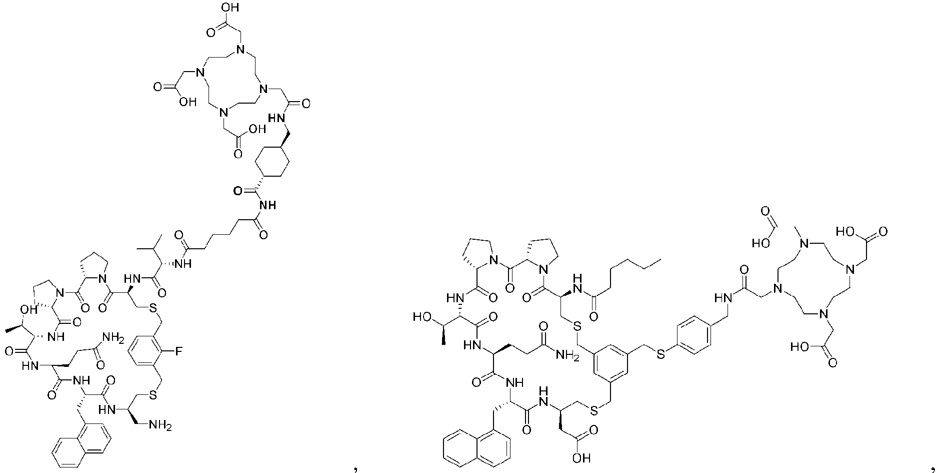

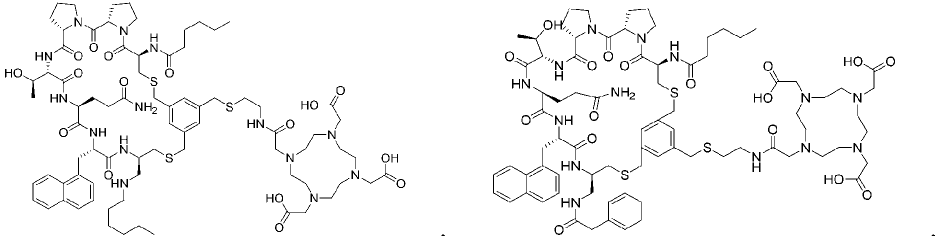

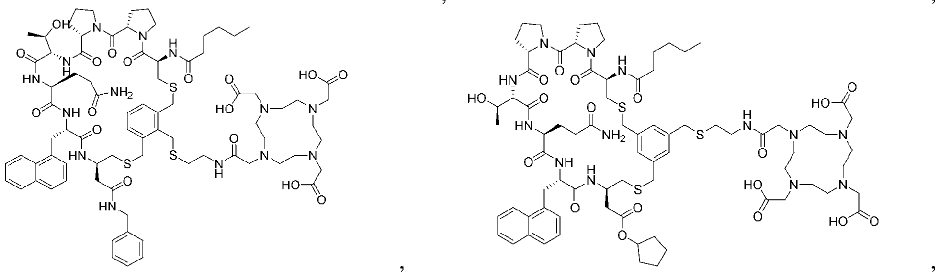

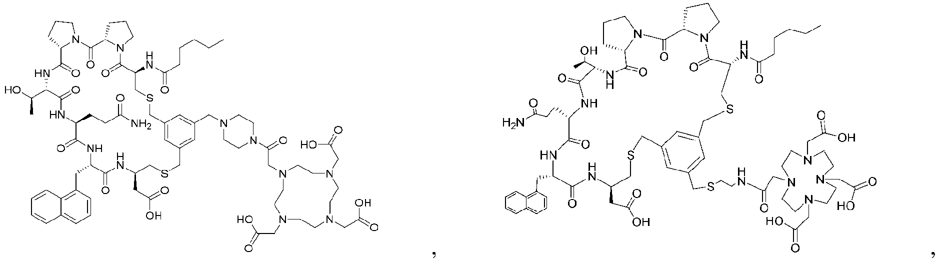

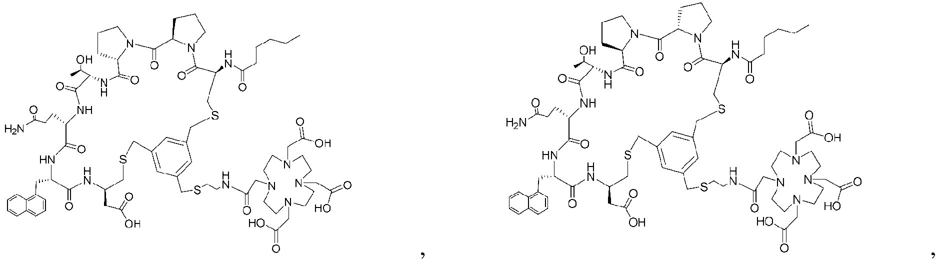

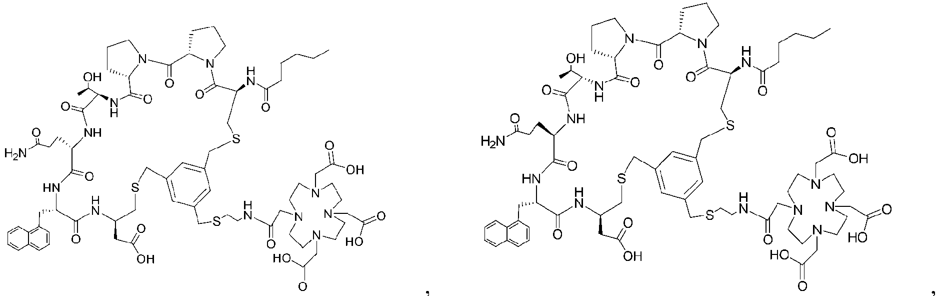

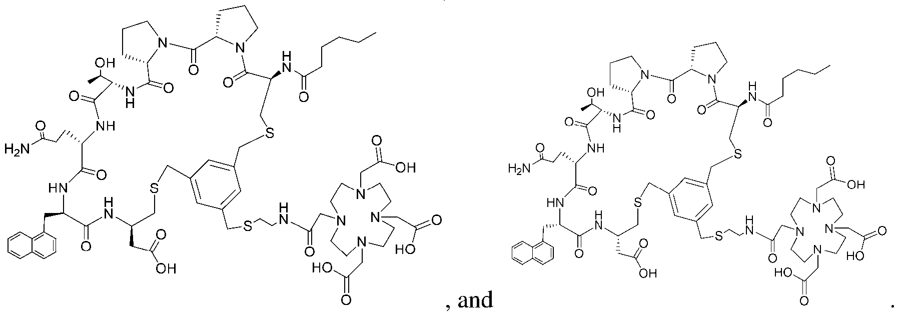

25. A polypeptide compound, a pharmaceutically acceptable salt thereof, a solvate thereof or a solvate of a pharmaceutically acceptable salt thereof, wherein the polypeptide compound is represented by formula V:
[D-L'-R]·M V
D is as defined in any one of claims 18-21;
L' is as defined in any one of claims 18-21;
R is as defined in any one of claims 22-24;
M is a diagnostically active radionuclide or a therapeutically active radionuclide; the diagnostically active radionuclide is selected from ¹⁸F, ^{99m}Tc, ⁸⁹Zr, ¹¹¹In, ⁶⁷Ga, ⁶⁸Ga, ⁴³Sc, ⁴⁴Sc, ⁶⁴Cu, ⁸⁶Y, ¹⁵²Tb, ¹⁵⁵Tb, ²⁰³Pb, ¹²³I, ¹²⁴I, ¹²⁵I, optionally ⁶⁴Cu, ⁶⁸Ga, ¹¹¹In, ¹⁸F, ^{99m}Tc, ²⁰³Pb, and ⁸⁹Zr; and the therapeutically active radionuclide is selected from ⁹⁰Y, ¹⁷⁷Lu, ^{22S} Ac, ²¹²Pb, ¹⁸⁸Re, ²²⁷Th, ¹³¹I, ²¹¹At, ¹⁶¹Tb, ¹⁴⁹Tb, ¹⁵³Sm, ⁶⁷Cu, ⁴⁷Sc, optionally ⁹⁰Y, ¹⁷⁷Lu, ²²⁵Ac, ²¹²Pb, and ¹⁶¹Tb.

26. The polypeptide compound of claim 25, a pharmaceutically acceptable salt thereof, a solvate thereof or a solvate of a pharmaceutically acceptable salt thereof, wherein the polypeptide compound is selected from any of the following compounds: 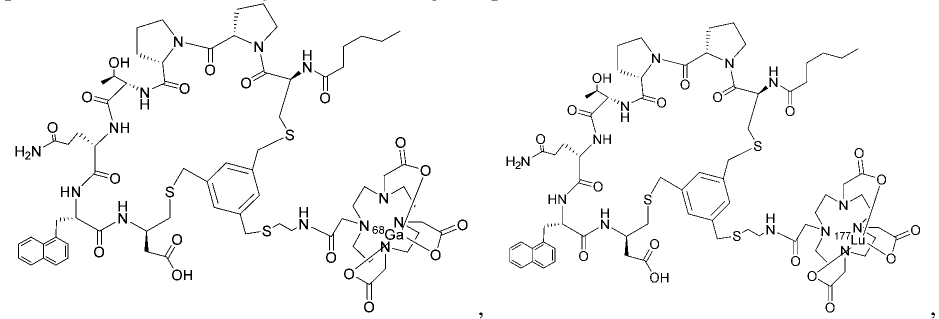 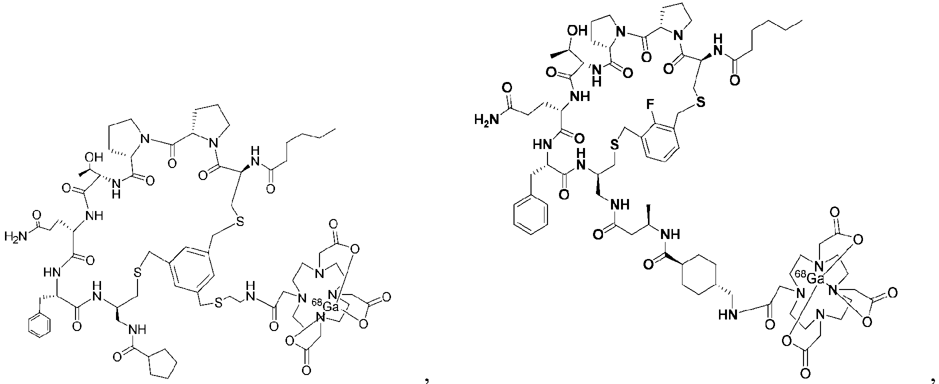 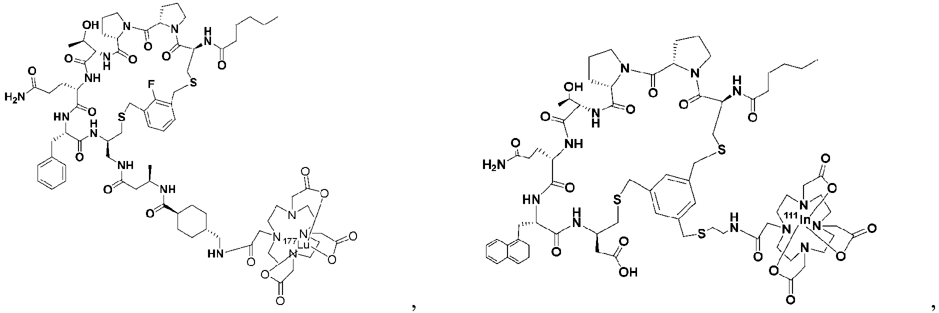 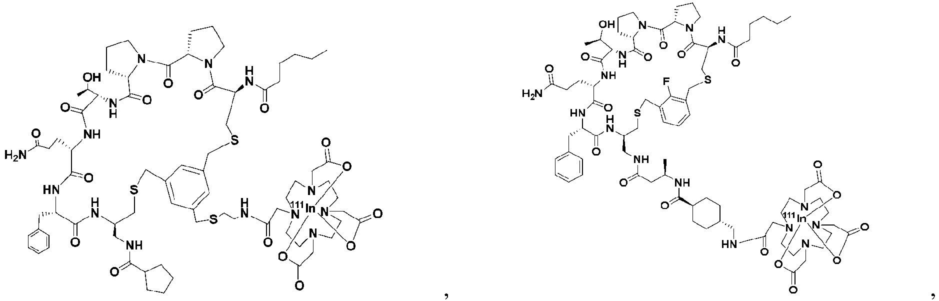 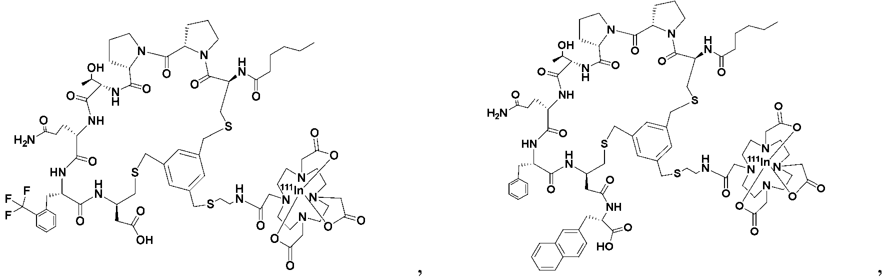 and

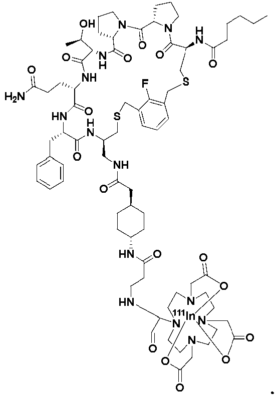

27. A polypeptide compound, a pharmaceutically acceptable salt thereof, a solvate thereof or a solvate of a pharmaceutically acceptable salt thereof, wherein the polypeptide compound is represented by formula VI:
[D-L'-R]·Q VI
D is as defined in any one of claims 18-21;
L' is as defined in any one of claims 18-21;
R is as defined in any one of claims 22-24;
Q is a non-radionuclide; and the non-radionuclide is selected from In, Ga, Y and Lu, optionally In.

28. The polypeptide compound of claim 27, a pharmaceutically acceptable salt thereof, a solvate thereof or a solvate of a pharmaceutically acceptable salt thereof, wherein the polypeptide compound is selected from any of the following compounds:

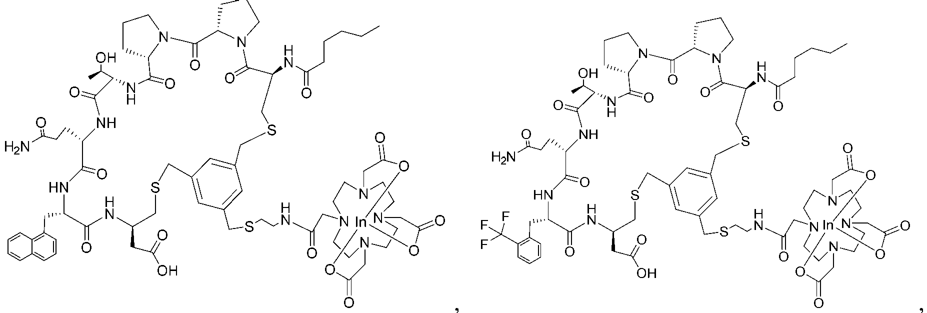

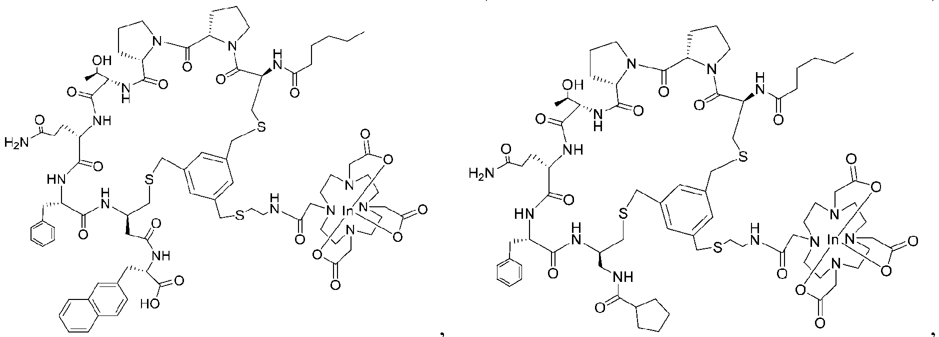

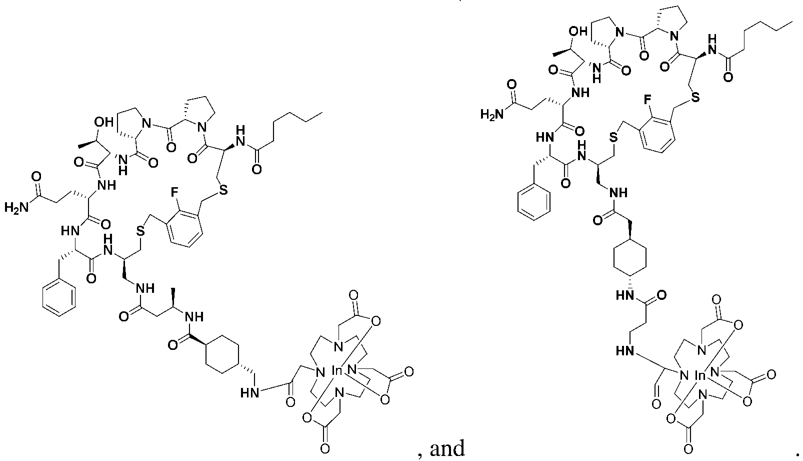

29. A pharmaceutical composition comprising a substance X, and a pharmaceutically acceptable adjuvant; wherein the substance X is a polypeptide compound of any one of claims 1-28, a pharmaceutically acceptable salt thereof, a solvate thereof or a solvate of a pharmaceutically acceptable salt thereof.

30. A kit comprising a substance X, and instructions for use; wherein the substance X is a polypeptide compound of any one of claims 1-28, a pharmaceutically acceptable salt thereof, a solvate thereof or a solvate of a pharmaceutically acceptable salt thereof.

31. Use of a substance X for the manufacture of a medicament for the diagnosis or treatment of a FAP protein-associated disease, wherein the substance X is a polypeptide compound of any one of claims 1-28, a pharmaceutically acceptable salt thereof, a solvate thereof or a solvate of a pharmaceutically acceptable salt thereof, and the FAP protein-associated disease is preferably a tumor.

32. The use of claim 31, wherein the tumor is selected from bronchogenic carcinoma, hidradenocarcinoma, breast cancer, ovarian cancer, prostate cancer, melanoma, oral squamous carcinoma, brain tumor, esophageal cancer, gastric cancer, liver cancer, pancreatic cancer, colorectal cancer, lung cancer, small cell lung cancer, non-small cell lung cancer, renal cancer, skin cancer, glioblastoma, neurilemmoma, meningioma, neuroblastoma, mesothelioma, sarcoma, liposarcoma, chondroioma, osteoma, osteosarcoma, semaginoblastoma, testicular tumor, uterine cancer, head and neck cancer, multiple myeloma, malignant lymphoma, polycythemia vera, leukemia, thyroid cancer, ureter tumor, bladder tumor, gall bladder cancer, cholangiocarcinoma, choriocarcinoma and pediatric tumor.

33. Use of a substance X for the manufacture of a FAP inhibitor, wherein the substance X is a polypeptide compound of any one of claims 1-28, a pharmaceutically acceptable salt thereof, a solvate thereof or a solvate of a pharmaceutically acceptable salt thereof.
